(19) Europäisches Patentamt
European Patent Office
Office européen des brevets

(11) EP 4 106 725 B1

(12) # EUROPEAN PATENT SPECIFICATION

(45) Date of publication and mention of the grant of the patent:
**13.08.2025 Bulletin 2025/33**

(21) Application number: **21718676.6**

(22) Date of filing: **15.02.2021**

(51) International Patent Classification (IPC):
***A61K 9/14*** (2006.01) ***A61K 9/16*** (2006.01)
***A61K 9/20*** (2006.01) ***A61K 36/44*** (2006.01)
***A61K 31/352*** (2006.01) ***A61K 9/19*** (2006.01)
***A61P 9/00*** (2006.01)

(52) Cooperative Patent Classification (CPC):
**A61K 9/2027; A61K 9/0019; A61K 9/146; A61K 9/1635; A61K 9/19; A61K 31/352**

(86) International application number:
**PCT/UA2021/000017**

(87) International publication number:
**WO 2021/167580 (26.08.2021 Gazette 2021/34)**

(54) **WATER-SOLUBLE SOLID DISPERSION OF QUERCETIN, FORMS THEREOF, A METHOD OF OBTAINING THEREOF, USE OF ALKALINE AGENT, AND A KIT**

WASSERLÖSLICHE FESTE DISPERSION VON QUERCETIN, FORMEN DAVON, VERFAHREN ZUR HERSTELLUNG DAVON, VERWENDUNG EINES ALKALIMITTELS UND KIT

DISPERSION DE QUERCÉTINE SOLIDE SOLUBLE DANS L'EAU, SES FORMES, UN PROCÉDÉ POUR L'OBTENIR, UTILISATION D'UN AGENT ALCALIN, ET KIT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.02.2020 UA 202001051**

(43) Date of publication of application:
**28.12.2022 Bulletin 2022/52**

(73) Proprietor: **"Scientific Industrial Centre "Borshchahivskiy Chemical Pharmaceutical Plant" Public Joint-Stock Company**
**Kyiv 03134 (UA)**

(72) Inventors:
- **MAKITRUK, Vasyl**
  **Kyiv 03037 (UA)**
- **SATANOVSKYY, Yan**
  **Kyiv region, 08150 (UA)**

(74) Representative: **KATZAROV S.A.**
**Geneva Business Center**
**12 Avenue des Morgines**
**1213 Petit-Lancy (CH)**

(56) References cited:
**CN-A- 109 771 395**

- **LI BIN ET AL: "Solid dispersion of quercetin in cellulose derivative matrices influences both solubility and stability", CARBOHYDRATE POLYMERS, APPLIED SCIENCE PUBLISHERS , LTD BARKING, GB, vol. 92, no. 2, 3 December 2012 (2012-12-03), pages 2033 - 2040, XP028972821, ISSN: 0144-8617, DOI: 10.1016/J.CARBPOL.2012.11.073**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The invention relates to the field of pharmaceutics and medicine, in particular, it relates to a method of obtaining water-soluble solid dispersions of quercetin with polyvinyl pyrrolidone (PVP) and alkaline agent having high bioavailability. It further relates to water-soluble solid dispersion of quercetin with polyvinyl pyrrolidone (PVP) and alkaline agent, wherein the alkaline agent is present in an amount within the range of (6.3 ± 0.5)*10-2 gE per 1 gE of quercetin, and wherein the alkaline agent does not form insoluble salt with quercetin and/or other components of solid dispersion. It also relates to such water-soluble solid dispersions of quercetin for use as substance for obtaining injectable medicinal products or oral medicinal product for treatment of acute conditions of cardiovascular and cerebrovascular system diseases in subjects in need thereof. The invention also relates to solid dispersion of quercetin, which is obtained using the claimed method, and use thereof.

### BACKGROUND OF THE INVENTION

Bioflavonoid quercetin, 2-(3,4-dihydroxyphenyl)-3,5,7-trihydroxy-4H-chromen-4-one (Formula 1), represents 3,3',4',5,7-pentaoxyflavone and it is the most common aglycon of flavanone-containing glycosides in plants and the most studied bioflavonoid.

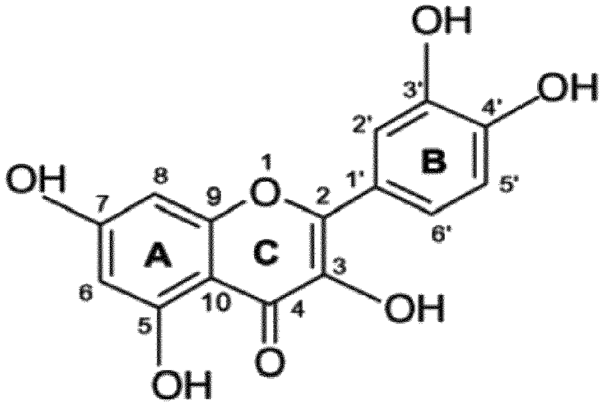

Formula 1

However, quercetin as an active substance with the identified pharmacological properties, has rather restricted application in pharmaceutical production due to its low solubility and, as a consequence, low bioavailability.

A broad spectrum of biochemical and pharmacological properties of quercetin, that have been studied [Monograph. Quercetin. Alternative Medicine Review. - 1998. - V. 3, N2. - P. 140 - 143; Miles S.L., McFarland M., Niles R. Molecular and physiological actions of

quercetin: need for clinical trials to assess its benefits in human disease. Nutr. Rev. - 2014. - Nov. 16; 72 (11) - P. 720 - 734.; J.B. Harborne, T.J. Marbry. (Ed.). The Flavonoids: Advances in Research. London, UK: Chapman and Hall, 1982, p. 1018.; Rice-Evans A., Packer L. Flavonoids in Health and Disease. - 1998.M. Dekker Inc, New York.; Nijveldt R.J., van Nood E., van Hoorn D.E., et al. Flavonoids: a review of probable mechanisms of action and potential applications. Am. J. Clin. Nutr. - 2001. - V 74, No. 4. - P. 418 - 425.; Middleton E.J., Kandaswavi C., Theoharides T.C. The effects of plant flavonoids on mammalian cells: Implications of inflammation, heart disease, and cancer. Pharmacol. Res. - 2000. - V. 52, No. 4. - P. 673 - 751.], is most notable the evidence of its marked antioxidant activity and ability to perform functions of the scavenger of superoxide radicals, singlet oxygen, which results in the processes of inhibition of formation of lipid hydroperoxide radicals [Andersen O.M., Markham K.R. Flavonoids: Chemistry, Biochemistry and Applications. - 2006. - CRC Press Taylor & Francis Group. - 1197 p.; Bors W., Heller W., Michel C., Saran M. Flavonoids as antioxidants: Determination of radical-scavenging efficiencies. Methods Ensymology. - 1990. - V. 186. - P. 343 - 355.; Saija A., Scalese M., Lanza M et al. Flavonoids as antioxidant agents: importance of their interaction with biomembranes. Free Radic. Biol. Med. - 1995. - V.19. - P. 481 - 486.; Van Acker S.A, van der Berg H., Tromp M.N., et al. Structural aspects of antioxidant activity of flavonoids. Free Rad. Biol. Med. - 1996. - V 20. - P. 331 - 342.].

In spite of the fact that a molecule of quercetin contains 5 hydroxylic groups, intermolecular planar interactions of phenolic nuclei, intramolecular and intermolecular hydrogen bonds of phenolic and carbonyl groups of bioflavonoid in crystalline dehydrated state and in the form of dihydrate will result in extremely poor solubility in water (~ 1 mg/100 mL),

which has a substantial impact on the outlooks of obtaining medicinal products of this bioflavonoid with sufficiently high bioavailability [Alvareda E., Denis P.A., Iribarne F., Paulino M. Bond dissociation energies and enthalpies of formation of flavonoids: A G4 and M06-2X investigation. Computat. Theoret. Chem. - 2016. - V 1091. - P. 18 - 23.; Perez-Gonzales A., Rebollar-Zepeda A.M., Galano A. Reactivity Indexes and O-H Bond Dissociation Energies of a Large Series of Polyphenols: Implications for their Free Radical Scavenging Activity. J. Mex. Chem. Soc. - 2012. - V 56, N3. - P. 241 - 249.].

The identified pharmacological properties of a quercetin molecule facilitated the efforts for obtaining medicinal products with improved solubility and bioavailability characteristics, search of methods and substances of solubilizers to provide high solubility of the active moiety in bodily fluids, primarily in development of injectable drugs.

The applicable methods included the use of lipidic and polymeric nanoparticles, micelles, emulsions, liposomes, etc. It is provided that the most promising method for creation of efficient injectable medicinal products of quercetin would consist in formation of its solid dispersions (SDs) with solubilizer matrices, as the structure of the hydrophilic complex, wherein quercetin is in amorphous state, would be sufficiently soluble substance for the future preparation.

Solid dispersions consist of at least two different components, one of which is usually an inert matrix with amphiphilic or hydrophilic properties, and the other is pharmacologically active substance. The solid dispersion may contain further components such as useful excipients and the appropriate active substance. In such systems, solubility and dissolution profile of the active substance can be improved by the active substance being in amorphous state within the solid dispersion and reduction of its particle size to submolecular size.

Water-soluble injectable form, which can be produced from, for example, the corresponding solid dispersion of quercetin in the polymeric matrix is one of the best drug forms for delivery of quercetin. On course, stable maintaining the initial structure of the solid dispersion is possible subject to formation of energetically strong intermolecular hydrogen bonds between the active substance molecule and the solubilizer (polymeric matrix). Suitable polymers include, in particular, polyethylene glycols, polyvinyl pyrrolidones (PVP) with different molecular weights of macromolecules, ß-cyclodextrins, phosphatidylcholines, poly lactic-co-glycolic acids (PLGAs), etc. It should be noted that the essential requirement to the solid dispersion intended for preparation of the injectable drug consists in its storage stability and stability at dissolution in aqueous normal salines in case of long-term use thereof.

It is provided that is polyvinyl pyrrolidone (PVP) is one of the most suitable polymers to be used as a matrix carrier for creation of the medicinal product. It is due to its amphiphilic properties, biocompatibility, lack of substantial toxicity and capability to form water-soluble complexes with hydrophobic preparations.

Examples of solid dispersions of quercetin in polymeric matrices, in particular, in PVP, are known from the prior art documents.

According to US Patent 7,387,805 B2, mixtures of soya saponins were used as solubilizers to obtain solid dispersion of quercetin and flavonoid derivatives with increased bioavailability. However, such approach is not suitable for the use of solid dispersions based on of soya saponins in production of indictable preparations.

The document Kendre PN., Pande V.V., Chavan K.M. Novel formulation strategy to enhance solubility quercetin. Pharmacophore. 2014. - V. 5, (3). - P. 358 - 370, discloses the intention to increase bioavailability of quercetin in gel composition, which consisted in selection of a mixture of solubilizers such as propylene glycol, glycerol and polyethylene glycols - PEG 200, PEG 400, PEG 600 - as hydrophilic solvents, and excipients such as sodium acetate, sodium citrate and urea as hydrotropic agents. With rather complex mixture of solubilizers, the best solubility of quercetin was achieved in gels, wherein its content was only 60 %.

Patent RU 2181051 discloses a method of obtaining an injectable quercetin preparation, which comprises dissolution of the active substance in heated solution of sodium tetraborate and Trilon B, with subsequent addition of polyvinyl pyrrolidone. However, the molecular weight of PVP was not indicated. The solution obtained thereby was subjected to autoclave sterilization in ampoules, which was rather destructive for quercetin. Furthermore, presence of sodium tetraborate solution exhibiting entirely uncontrolled high pH levels also contributed to decomposition of quercetin.

Eurasian Patent EA 021352 B1 discloses a method comprising mixing ethanolic solutions of phosphatidylcholine and quercetin, evaporation of the mixture under vacuum, emulsifying it in aqueous solution, homogenization of the emulsion with addition of lactose as an aqueous solution, stepwise sterile filtration and lyophilization. The resulted preparation was in the form of a liposome with high content of encapsulated quercetin and definite storage stability.

Development of solid dispersions of quercetin with high molecular PVP for obtaining bioavailable forms that would be used as compositions or substances for manufacture of oral drug forms is also known. In particular, the document de Mello Costa A. R., Marquiafável F.S., Mirela Mara de Oliveira M.M. Quercetin-PVP K25 solid dispersion. J. Term. Anal. Calormtre. - 2011. - V. 104. - P. 273 - 278., discloses a method of obtaining solid dispersion of quercetin from PVP - Kollidon K25 (PVP K25, mw 28,000 - 34,000 (Basf)), which was carried out by dissolution of quercetin in solutions containing PVP K25 in the range of 1.3 % to 47.4 % in 0.1M phosphate buffer (pH 7.0) with stirring for 24 hours. Solid dispersion was obtained after evaporation of the solvent. The structure of the obtained samples was studied using the methods of differential scanning calorimetry (DSC), thermogravimetry (TG), X-ray diffraction, UV- and IR-Fourier spectroscopy. Polymorphism of the obtained solid structures and dependency of their solubility on the component ratio have been established. Antioxidant activity of the samples was also studied.

Solid dispersions of quercetin with PVP K25 having the above composition cannot usually be used as pharmaceutical substances for the manufacture of injectable preparations, primarily by reason of high weight-average molecular weight of PVP K25. Solid dispersion produced with the use thereof has high viscosity, which poses a challenge, when used for injections, as well as due to toxico-pharmacological values of the solubilizer matrix. As is known from the documents Ruffer W. Clinical follow-up on the problem of Kollidon storage. Medizinische. - 1955. - V. 15, N9. - P. 539 - 541.; Hosorawa S., Uchino F., Miyazato T., et al. Storage of Polyvinyl Pyrrolidone. Bull. Yamaguchi Med. School. -1966. - V. 13, N4. - P. 281 - 298.; Kojima M., Takahashi K., Honda K. Morphological Study on the Effect of Polyvinyl Pyrrolidone Infusion upon the Reticuloendothelial System. Tohoku J. exp. Med., - 1967. - V. 92, - P. 27 - 54.; Final Report on the Safety Assessment of Polyvinylpyrrolidone (PVP). International Journal of Toxicology. - 1998. - V. 17, (Suppl. 4). - P. 95 - 130., polyvinyl pyrrolidones with molecular weight greater than 11,000, when administered intravenously, are capable to be accumulated in human organs and thereby cause toxic effects.

According to the Ukrainian Patent No. 23996, solid dispersion of quercetin was obtained by performing the following operations: ethanolic solution of PVP (Kollidon K17, PVP K17 (Basf) with a range of weight-average molecular weights of 7,000 to 11,000) and quercetin were boiled for 50-60 min, the solvent was evaporated, the residue was dissolved in water and neutralized with 0.1 M solution of caustic soda to pH 6.5 - 7.0. Aqueous solution of the mixture was sterilized under filtering conditions, vialed and dried by lyophilization. In such a way, solid dispersion of quercetin was obtained, with PVP in the quantitative ratio of 1:50 and 1:25, i.e. with the content of quercetin 2 % and 4 %, respectively.

The cited method has substantial drawbacks due to excessive content of PVP in the solid dispersion at the component ratio of 1:25 and 1:50, with quercetin concentration therein as little as 4 % and 2 %, which would be inadmissible condition exactly for the manufacture of a drug formulation in the form of injectable medicine, when the use of the necessary efficient dose of quercetin leads to excessive intravenous administration of PVP, which would increase toxicity of the resulting infusion solution.

The specified time of stability of aqueous solutions of the samples of solid dispersion with quercetin content in the range of 1 % - 20 % within pH values of 3.4 - 9.0, with quantitative increase of PVP and rising pH levels is also debatable, inasmuch as quercetin disintegrates extremely quickly with increase of basicity of the solution.

The document Porcua E.A., Cossua M., Rassua G., Giunchedia H., et. Al. Aqueous injection of quercetin: An approach for conformation of its direct in vivo cardiovascular effects. Intern. J. Pharmac. 2018. V. 541, P. 224 - 233., discloses obtaining solid dispersion of quercetin within the wide range of weight ratios of quercetin with PVP10 (Sigma-Aldrich) using the methodology of evaporation of aqueous ethanol as a cosolvent. When obtaining the range of solid dispersions of quercetin : PVP 1:3 - 1:20, the solutions of weight-constant aliquots of quercetin in ethanol were added with aqueous solutions having PVP content that corresponded to the weight fraction of PVP in the desirable solid dispersion. The resulting solutions of components were stirred and evaporated under vacuum on a rotary evaporator. Samples of solid dispersions was obtained with further drying the solutions under vacuum or under conditions of freezing and lyophilization.

Physicochemical and pharmacological properties of the range of solid dispersions as obtained was studied only for the samples with 1:12 ratio of quercetin and PVP10, in particular, dissolution consisted in preparation of the solution of solid dispersion in distilled water, the suspension was filtered through a filter with 0.22 μm pores, aliquot of 20 mM of sodium hydroxide was added to the filtrate, and only after 40 minutes quantitative content of quercetin was determined using UV-spectroscopy method by the rate of absorption of quercetin decomposition products.

Drawbacks of said method include incomplete dissolution of the resulting solid dispersion and presence of quercetin in the suspension solution, which is evidence of incomplete complexing involving both components and instability of such complex. Instability of the solid dispersion complex might be attributed to low molecular weight (mw 10,000) of PVP10 used.

It was also indicated in this document that with increasing weight fraction PVP10 in the solid dispersion in Q:PVP ratio from 1:12 to 1:15 and 1:20, aqueous solutions of such solid dispersions were too viscous, and that did not allow to use them for preparation of injectable drugs. However, lowering the content of solubilizer may result in accelerated decomposition of the solid dispersion structure, both in solid and in dissolved states. In case of Q:PVP ratios of 1:3 and 1:5, their solutions were unstable.

While the solid dispersion described in the above mentioned document was intended for its use in preparation of the injectable solution for intravenous administration, the lack of studying stability of such solid dispersion solutions at different pH values remains unclear. It does not allow to predict integrity of the true solution between the time of its preparation and the time of its parenteral administration.

CN109771395A discloses a pharmaceutical composition in form of a solid dispersion containing quercetin, that comprises a drug-loading layer containing 1 to 50 parts by mass of quercetin, 0 to 5 parts by mass of borneol, 0 to 3 parts by mass of glycyrrhetinic acid and 50 to 95 parts by mass of polyvinylpyrrolidone.

In the publication of Li Bin et al: "Solid dispersion of quercetin in cellulose derivative matrices influences both solubility and stability", Carbohydr Polym. 2013 Feb 15;92(2):2033-40 discloses amorphous solid dispersions of quercetin in cellulose derivative matrices, carboxymethylcellulose acetate butyrate (CMCAB), hydroxypropylmethylcellulose acetate succinate (HPMCAS), and cellulose acetate adipate propionate (CAAdP) were prepared with the goal of

identifying an amorphous solid dispersions that effectively increased quercetin aqueous solution concentration. Crystalline quercetin and quercetin/poly(vinylpyrrolidinone) (PVP) amorphous solid dispersions were evaluated for comparison. Powder X-ray diffraction (XRPD) and differential scanning calorimetry (DSC) were used to examine the crystallinity of ASDs, physical mixtures (PM) and quercetin. Amorphous solid dispersions were amorphous up to 50 wt% quercetin. Quercetin stability against crystallization and solution concentrations from these amorphous solid dispersions were significantly higher than those observed for physical mixtures and crystalline quercetin. PVP stabilizes against both quercetin degradation and recrystallization; in contrast, these carboxylated cellulose derivatives inhibit recrystallization but release quercetin slowly. PVP amorphous solid dispersions afforded fast and complete drug release, while amorphous solid dispersions using these three cellulose derivatives provide slow, incomplete, pH-triggered drug release..

The closest prior art to the claimed invention is the method disclosed in the Ukrainian Patent No. 38504, which describes the method of obtaining solid dispersion with 1:9 ratio of weight fractions of quercetin to PVP (Kollidon K17, PVP K17 (Basf)), with a range of weight-average molecular weights of polymer macromolecules, mw 7,000 - 11,000, using the method according to Ukrainian Patent No. 23996, except that the aqueous solution was neutralized with sodium hydroxide solution to pH 6.8 prior to sterilization and lyophilization.

Drawback of this method consists in multiple-step operations for obtaining the solid dispersion of quercetin, that are accompanied by time-consuming boiling of ethanolic solution of components, evaporation of solvent to dryness and dissolution of dry residue in water.

Addition of strong alkaline agent sodium hydroxide to aqueous solution of PVP and quercetin can create local areas with high pH values, which results in decomposition of quercetin molecule and causes significant growth of impurities, which has negative impact on quality of solid dispersion of quercetin with PVP.

Usage of caustic soda solutions as exclusive alkaline agents to adjust pH for obtaining solid solutions of quercetin imposes significant restrictions on technology options for obtaining dispersions, variations of physicochemical and biological properties. Administration of alkaline agent with parallel adjustment of pH value requires much time and additional labor costs.

This method is also restricted due to the exclusive use of 1:9 quantitative ratio of quercetin to PVP, wherein the content of the active component in the solid dispersion is 10.0 %. That allowed to obtain injectable solutions with quercetin content in the concentration range not exceeding 0.05 % in 100 ml of normal saline or 0.1 % in 50 ml.

More concentrated injectable solutions obtained from the solid dispersion of quercetin with PVP at the component ratios of 1:7 and 1:8, or even 1:11 and 1:12, would be more preferable by quercetin content therein, as preparations with high doses of quercetin are not toxic. It is already the case that the studied pharmacotherapeutic activity of quercetin requires application of its preparations in clinical treatment of many diseases in higher doses.

Among the above mentioned methods for obtaining solid dispersions of quercetin with PVP the most successful were those involving low molecular PVPs, especially where PVP K17 (component ratio 1:9) and PVP10 (the most studied ratio 1:12) were used. However, said sources are silent about the analysis of impact of sodium hydroxide as an alkaline agent on stability of structure of the solid dispersion in dissolved states. At the same time, the described examples do not provide any information on the growth of quercetin decomposition products based on changes in pH values of solutions of solid dispersions. Solid dispersion of quercetin, as a substance for manufacture of the medicinal product in the form of injectable solution, has to be controlled for the content of decomposition products as impurities in accordance with compendial quality requirements.

## SUMMARY OF THE INVENTION

The invention is as set out in th appended set of claims.

## BRIEF DESCRIPTION OF THE INVENTION

The task is addressed by means of water-soluble solid dispersion of quercetin (Q) with polyvinyl pyrrolidone (PVP) and alkaline agent, wherein the alkaline agent is present in an amount within the range of $(6.3 \pm 0.5)*10^{-2}$ gE per 1 gE of quercetin, wherein the alkaline agent does not form insoluble salt with quercetin and/or other components of solid dispersion.

In particularly preferable embodiment of the invention, the quercetin to PVP ratio in the water-soluble solid dispersion should be in the range from 1:7 up to and including 1:12, and desirably selected from the ratio group comprising 1:7, 1:9 and 1:12. Furthermore, it is desirable to use polyvinyl pyrrolidone with average molecular weight of polymeric macromolecules between 7,000 and 11,000. The particular example of such polymer may be represented by Kollidon® K 17 from BASF SE, anyway, it is stipulated that polyvinyl pyrrolidones from other manufacturers may also be used.

In particularly preferable embodiment of the invention, in the claimed water-soluble solid dispersion of quercetin according to the invention quercetin selected from quercetin dihydrate, anhydrous quercetin and mixtures thereof is used. If anhydrous quercetin is used it is preferably anhydrous quercetin crystallized from ethanol, more preferably anhydrous

quercetin double-crystallized from 96 % ethanol with a content of the active substance at least 99.7 %.

Preferably, the water-soluble solid dispersion of quercetin contains as an alkaline agent inorganic or organic substance having basic properties and not forming insoluble compounds with the components of the claimed solid dispersion, for example selected from the group consisting of sodium hydroxide, sodium bicarbonate, sodium carbonate, L-arginine or mixtures thereof. The use of individual alkaline agents or combinations thereof can also be possible.

In an embodiment of the present invention it relates to the method for obtaining water-soluble solid dispersion of quercetin with polyvinyl pyrrolidone and alkaline agent, wherein the claimed method provides for performing the following stages of preparation of the mixture of solid dispersion components, said preparation providing for: a) the predetermined amount of PVP is added to alcohol-containing solvent, then the calculated amount of alkaline agent is added, whereafter the predetermined amount of quercetin is added, or b) the predetermined amount of PVP is added to alcohol-containing solvent, then the predetermined amount of quercetin is added, whereafter the calculated amount of alkaline agent is added, or c) the predetermined amount of PVP, predetermined amount of quercetin and the calculated amount of alkaline agent are pooled, whereafter the alcohol-containing solvent is added, upon preparation of the mixture of solid dispersion components, stirring is carried out to complete dissolution of all its components, and water-soluble solid dispersion is obtained, wherein during preparation of the mixture of solid dispersion components, alkaline agent is added in the amount within the range of $(6.3 \pm 0.5)*10^{-2}$ gE of alkaline agent per 1 gE of quercetin, wherein the alkaline agent does not form insoluble salt with quercetin and/or other components of solid dispersion.

In particular embodiment of the method, the weight of the alkaline agent is calculated according to the formula (1):

$$m(BA) = \frac{k * m(Q)}{\mathrm{E}_m(Q)} * \mathrm{E}_m(BA)(1),$$

where:

- m(Q) - weight of quercetin in the solid dispersion in grams;
- $E_m(Q)$ and $E_m(BA)$ - equivalent weights of Q (quercetin) and BA (alkaline agent), respectively, in gE/mol;
- m(BA) - weight of alkaline agent in grams;
- k - ratio factor of the number of gE equivalents of the selected alkaline agent ($*10^{-2}$) to 1 gE of quercetin.

In particularly preferable embodiment of the method, the quercetin to PVP ratio in the water-soluble solid dispersion is from 1:7 up to and including 1:12, and desirably selected from the group comprising 1:7, 1:9 and 1:12. Furthermore, it is desirable to use polyvinyl pyrrolidone with average molecular weight of polymeric macromolecules between 7,000 and 11,000. The particular example of such polymer may be represented by Kollidon® K 17 from BASF SE, Germany, anyway, it is stipulated that polyvinyl pyrrolidones with appropriate average molecular weight of polymeric macromolecules, manufactured by other companies, may also be used.

In particularly preferable embodiment of the method, quercetin selected from quercetin dihydrate, anhydrous quercetin and mixtures thereof is used. If anhydrous quercetin is used it is preferably anhydrous quercetin crystallized from ethanol, preferably anhydrous quercetin double-crystallized from 96 % ethanol, which contains the active substance in the amount of at least 99.7 %.

Preferably, in the method, inorganic or organic substance is used as an alkaline agent, which has alkaline properties and does not form insoluble compounds 3 components of solid dispersion, for example selected from the group consisting of sodium hydroxide, sodium bicarbonate, sodium carbonate, L-arginine or mixtures thereof. The use of individual alkaline agents or combinations thereof can also be possible. Besides, in case where preparation of the mixture of solid dispersion components provides for sequential addition of components, i.e. where the predetermined amount of PVP is added first to alcohol-containing solvent, then the calculated amount of alkaline agent is added, and thereafter the predetermined amount of quercetin is added, or where the predetermined amount of PVP is added to alcohol-containing solvent, then the predetermined amount of quercetin is added, and thereafter the calculated amount of alkaline agent is added, the desired alkaline agent is selected from the group consisting of sodium hydroxide, sodium bicarbonate, sodium carbonate, L-arginine, and mixtures thereof. Furthermore, in case where preparation of the mixture of solid dispersion components provides for simultaneous pooling the predetermined amount of PVP, the predetermined amount of quercetin and the calculated amount of alkaline agent, with addition of the alcohol-containing solvent, the desired alkaline agent is selected from the group consisting of sodium bicarbonate, sodium carbonate, L-arginine, and mixtures thereof.

Preferably, the alcohol-containing solvent is an aqueous ethanol solution, preferably an aqueous ethanol solution with a concentration of 50 - 80 %, more preferably 70 - 72 %.

Preferably, the stage of mixing by codissolution is carried out at a temperature of $40 \pm 10$°C.

Preferably, the stage of obtaining the water-soluble solid dispersion of quercetin is carried out by evaporation of the solution of components under vacuum, preferably under residual pressure of 10 - 12 mbar, or drying by spray drying.

Selection of the desired method for obtaining of water-soluble solid dispersion may be determined by possible subsequent application of the resulting dispersion, for example for parenteral or oral administration.
preferably, it may be useful to carry out all stages of the method under air oxygen or under an inert gas, preferably the method is carried out under argon or under nitrogen, or mixtures thereof.

Preferably, the method further provides for granulation of the obtained water-soluble solid dispersion and its packaging into an airtight container. The airtight container may be represented by a package, which does not interact with the solid dispersion according to the invention, for example glass or plastic vessel protecting the product present therein from environmental impacts such as air and/or solar irradiation. It may be desirable that the water-soluble solid dispersion is contained in the airtight container under an inert gas, for example nitrogen or argon, or mixtures thereof.

Preferably, if it is desirable to produce a sterile dosage form obtainable from the water-soluble solid dispersion, the claimed method further provides for carrying out a stage of dissolution of water-soluble solid dispersion in sufficient quantity of distilled water, filtration sterilization of the solution, filling into vials, lyophilization in covered vials and secure closure of the vials. The vials may be represented by glass vials, for example those (cat. No. 0111075.1063, medical glass, Bratislava, Slovak Republic) closed with rubber stoppers (cat. No. C5919, Aptar Stelmi SAS, Granville, France) and aluminium flip-off caps (cat. No. K-2-20, Chernivtsi Plant of Medical Products, Chernivtsi, Ukraine). It may be desirable that the water-soluble solid dispersion prior to closure of the vials is placed in the atmosphere of purified dry air or inert gas, preferably purified dry inert gas argon or nitrogen, or mixtures thereof. It is desirable that the filtration sterilization should be carried out by filtration through nylon filter with pore diameter of 0.2 μm, such as (DA36MDMM002MCY2, Danmil A/S, Greve, Denmark), preferably with additional filtration through cellulose acetate cartridge filter filled with capsules with pore diameter of 0.45 μm and 0,22 μm, for example (SARTOBRAN P, Sartorius Stedim Biotech GmbH, Göttingen, Germany) under the pressure of 2.2 bar.

Preferably, in case of lyophilization in vials the method of lyophilization includes freezing vials to -40 °C, lyophilization at -40 °C and -20 °C and pressure of 0.12 mbar, drying at +10 °C under residual pressure of 0.12 mbar and at +30 °C and pressure of 0.0023 mbar.

The water-soluble solid dispersion according to the present invention is produced by the claimed method of its obtaining, as described herein.

More preferably, the water-soluble solid dispersion according to the present invention contains quercetin, preferably in amorphous form.

More preferably, the water-soluble solid dispersion according to the present invention after dissolution in the injectable infusion solution has pH value in the range of 6.75 ± 0.15.

More preferably, the water-soluble solid dispersion according to the present invention after dissolution in purified water or infusion solution has osmolality of about 295 to 302 mOsm/kg.

More preferably, the water-soluble solid dispersion according to the present invention after dissolution in water, in particular, with purified water or infusion solution, has total impurities content not exceeding 0.68 % after storage for 24 hours.

In an embodiment of the present invention, the claimed water-soluble solid dispersion of quercetin is used as a substance for production of injectable medicinal products with a dose of the active substance in the range of 14.28 % to 8.33 %.

In another embodiment of the present invention, the claimed water-soluble solid dispersion of quercetin is used as a substance for production of injectable medicinal products for use in the treatment of cardiovascular diseases, in particular, acute conditions of cardiovascular and cerebrovascular system diseases in subjects in need thereof.

The claimed water-soluble solid dispersion of quercetin can be used as a substance for production of an oral medicinal product.

In another embodiment the present invention relates to a lyophilized form of water-soluble solid dispersion of quercetin, which contains solid dispersion of quercetin, as claimed in the present invention, said lyophilized form preferably being intended for preparation of an injection and is sterile. It is desirable that said lyophilized form should contain 50 mg of quercetin per dose.

In another embodiment the present invention relates to the tableted form of water-soluble solid dispersion of quercetin, which contains solid dispersion of quercetin as claimed in the present invention. It is desirable that said tableted form should contain 50 mg quercetin per tablet.

In another embodiment the present invention relates to infusion solution including the solid dispersion of quercetin according to the present invention and a physiologically acceptable solvent. The physiologically acceptable solvent is preferably normal saline, in particular, 0.9 % NaCl solution with pH value in the range of 5.5 to 7.0.

Preferably said infusion solution contains quercetin at the concentration in the range of 1 to 0.5 mg/ml.

In another the present invention relates to an injectable form, which contains a sterile solution of solid quercetin dispersion as claimed in the present invention. Preferably said injectable form has pH value in the range of 5.5 to 7.0. In still more preferable embodiment of the invention, said injectable form contains quercetin at the concentration in the range of 1 to 0.5 mg/ml.

In another embodiment the present invention relates to use of alkaline agent for obtaining the water-soluble solid dispersion of quercetin with polyvinyl pyrrolidone according to the present invention, wherein the alkaline agent is used in the amount within the range of $(6.3 \pm 0.5)*10^{-2}$ gE per 1 gE of quercetin, wherein the alkaline agent does not form insoluble salt with quercetin and/or other components of solid dispersion. Preferably, the alkaline agent is selected from the group consisting of sodium hydroxide, sodium bicarbonate, sodium carbonate, L-arginine, and mixtures thereof.

In another embodiment the present invention relates to a container, which contains sterile water-soluble solid dispersion of quercetin according to the present invention or sterile solution of the solid dispersion of quercetin according to the present invention. The container is intended to provide sterility for the water-soluble solid dispersion of quercetin according to the present invention or solution of the solid dispersion of quercetin according to the present invention to be contained therein.

Preferably, said container contains 50 mg of quercetin per dose if the container contains sterile water-soluble solid dispersion of quercetin according to the present invention. In another more preferable embodiment of the invention said container contains quercetin at the concentration in the range of 1 to 0.5 mg/ml if the container contains sterile solution of the solid dispersion of quercetin according to the present invention.

The container may be represented by any suitable vessel to provide sterility for the water-soluble solid dispersion of quercetin according to the present invention or solution of the solid dispersion of quercetin according to the present invention to be contained therein. In the most preferable embodiment of the invention, said container is a vial.

In another embodiment, the present invention relates to a kit including the container, which contains the sterile water-soluble solid dispersion of quercetin according to the present invention or sterile solution of the solid dispersion of quercetin according to the present invention and, optionally, solvent and instructions for use of the kit.

It is provided that the kit, in particular, the pharmaceutical kit, contains one or more containers that contain the sterile water-soluble solid dispersion of quercetin according to the present invention or sterile solution of the solid dispersion of quercetin according to the present invention. Such kit may optionally include an instruction or information in the form approved by the state authority regulating the production, application and marketing of pharmaceutical drugs. Each component (in case of the presence of more than one component) may be packaged in its individual container, or some components may be combined in the same container, where it is permissible with regard to cross-reactivity and shelf life. The kits may be presented in the form of pharmaceutical dose forms, bulk packages (for example, in multi-dosage containers) or in doses smaller than the standard dose. The kits may also include several dosage units and instructions for use and they may be packaged in the amounts sufficient for storage in pharmacies (*for example,* pharmacies at hospitals or pharmacies with reception areas).

## BRIEF DESCRIPTION OF THE DRAWINGS

The drawings presented in the specification demonstrate certain embodiments of the invention and are not intended to limit the spirit of the invention in any way.

FIG. 1 depicts plots of pH values of aqueous solutions of solid dispersions with Q:PVP ratios of 1:7, 1:9 and 1:12 as a function of the number of gEs of alkaline agents per 1 gE of quercetin.

FIG. 2 depicts plots of ratio factor k as a function of weight fraction of polyvinyl pyrrolidone in the solid dispersion and basic properties of alkaline agents at quercetin to polyvinyl pyrrolidone ratios in the range of 1:7 to 1:12.

FIG. 3 depicts a graph of the rate of release of quercetin from samples of solid dispersions having the following composition: Q:PVP 1:9 NaOH; Q:PVP 1:9 L-arginine; Q:PVP 1:9 $Na_2CO_3$; Q:PVP 1:9 $NaHCO_3$.

FIG. 4 depicts a plot of the rate of release of quercetin from samples of solid dispersions having the following composition: Q:PVP 1:7 L-arginine; Q:PVP1:9 L-arginine; Q:PVP1:12 L-arginine as a function of quantitative content of polyvinyl pyrrolidone therein.

FIG. 5 depicts a graph of disintegration rate of solid dispersion and formation of crystalline precipitate of quercetin. As can be seen from the graph, the solutions of solid dispersion within the range of 1:7 to 1:12 remain stable for at least 24 hours.

FIG. 6 presents the image of Fourier transform infrared spectra of individual components of the claimed solid dispersions, specifically quercetin and polyvinyl pyrrolidone, as well as physical mixture of components and solid dispersions of quercetin in polyvinyl pyrrolidone according to the invention.

FIG. 7 presents X-ray powder diffractograms of quercetin dihydrate and quercetin purified by crystallization from ethanol, as well as two samples of solid dispersions having the following composition: Q:PVP 1:9 k[L-arginine] and Q:PVP 1:9 k[$NaHCO_3$], according to the invention.

FIG. 8 presents differential scanning calorimetry curves for individual components of the solid dispersion of quercetin and polyvinyl pyrrolidone, as well as solid dispersions of Q:PVP 1:7 and Q:PVP 1:9 and physical mixture of quercetin + polyvinyl pyrrolidone with the ratio of 1:9.

FIG. 9 presents a diagram of stability of solutions of solid dispersions obtained from solid dispersions as described in

Examples 4.1 - 4.10 in 0.9 % solution of sodium chloride with pH 5.5.
FIG. 10 presents a diagram of stability of solutions of solid dispersions obtained from solid dispersions as described in Examples 4.1 - 4.10 in 0.9 % solution of sodium chloride with pH 6.2.
FIG. 11 presents a diagram of stability of solutions of solid dispersions obtained from solid dispersions as described in Examples 4.1 - 4.10 in 0.9 % solution of sodium chloride with pH 7.0.
FIG. 12 depicts a plot of pH value of infusion solution as a function of concentration of quercetin in the wide range from 20 to 0.03125 mg/ml in 0.9 % solution of sodium chloride with pH = 5.50; 6.20; 7.00.
FIG. 13 depicts a plot (averaged) of quercetin release from series 041119 preparation as a function of dissolution time, in content percentage.
FIG. 14 depicts a plot (averaged) of quercetin release from series 051119 preparation as a function of dissolution time, in content percentage.
FIG. 15 presents IR spectra of PVPK17, physical mixture of Q + PVP (1:9) + L-arginine and SDs of Q:PVP 1:9 k[L-arginine] obtained using the methods of coevaporation of solvents (Examples 3.7i) and spraying (Examples 8.3)

## DETAILED DESCRIPTION OF THE INVENTION

The claimed water-soluble solid dispersion of quercetin with polyvinyl pyrrolidone (PVP) and alkaline agent, which has high bioavailability for manufacture of injectable and oral medicinal products. The method of obtaining the water-soluble solid dispersion of quercetin, lyophilized and tableted forms of water-soluble solid dispersion of quercetin, and the use of alkaline agent in the method of obtaining the water-soluble solid dispersion of quercetin are also claimed.
In the present invention, the weight ratio of quercetin to PVP in SD is provided as the ratio in the range if values from 1:7 to 1:12, i.e. 1:7, 1:8, 1:9, 1:10, 1:11, 1:12, but it should be understood that the range also comprises ratios tenth and hundredth of integers, for example the ratio from 1:7 to 1:8 comprises weight ratios of 1:7; 1:7.1; 1:7.2; 1:7.3; 1:7.4; 1:7.5; 1:7.6; 1:7.7; 1:7.8; 1:7.9 and 1:8, the ratio from 1:7 to 1:7.1 comprises weight ratios of 1:7; 1:7.01; 1:7.02; 1:7.03; 1:7.04; 1:7.05; 1:7.06; 1:7.07; 1:7.08; 1:7.09 and 1:7.1. In turn, the above mentioned desirable quantitative ratios of Q:PVP in the range of 1:7 to 1:12 provide for the content of the quercetin as an active substance in the range of 14.28 % to 8.33 %, which allows to produce medicinal products with rather high sizes of injectable doses.
Medicinal products of quercetin can be for use in medicinal treatment, in particular, in the treatment of cardiovascular diseases, in particular, acute conditions of cardiovascular and cerebrovascular system diseases.
According to the present invention, crystalline polymorphic forms of quercetin produced by PJSC Scientific Industrial Center (SIC) "Borshchahivskiy Chemical-Pharmaceutical Plant" or from other manufacturer, provided they have appropriate quality, in particular, quercetin dihydrate and anhydrous quercetin obtained by double crystallization of quercetin dihydrate from 94 - 96 % ethanol, having purity of 99.7 % as determined by HPLC, may be used for manufacture of solid dispersions of quercetin in polyvinyl pyrrolidone (SDQ PVP). Anhydrous quercetin substance is free of exogenous pyrogens, which allows to obtain SDQ PVP for manufacture of sterile lyophilized quercetin preparations. The use of quercetin dihydrate for obtaining SDQ PVP allows to use them in manufacture of solid dosage forms for oral administration with high bioavailability of quercetin.
X-ray structural analysis of samples of quercetin dihydrate and anhydrous quercetin, as shown in FIG. 7, confirms polymorphism of their crystalline structures.
According to the present invention, polyvinyl pyrrolidone with weight-average molecular weight of polymeric macromolecules in the range of 7,000 to 11,000 was used as hydrophilic solubilizer matrix for obtaining SDQ PVP, in particular, Kollidon K17 from Basf (PVP K17), which, due to its optimum weight-average molecular weight of macromolecules, is by its toxico-pharmacological values and capability of formation of intermolecular hydrogen bonds with quercetin a preferable solubilizer for production of safe medicinal products for parenteral use. Anyway, it is stipulated that other polyvinyl pyrrolidones with similar weight-average molecular weight of polymeric macromolecules in the range of 6,000 to 14,000 may also be used.
Stability of aqueous solutions of solid dispersions of quercetin in PVP is to a great degree determined by the specific range of pH values of the medium, therefore their production requires using certain correcting reagents (alkaline agents).
Among the five hydroxylic groups present in the structure of quercetin molecule, hydroxylic groups in 4'- and 7-positions of the molecule with acidity constant values $pKa_1$ 6.62 ± 0.04 and $pKa_2$ 9.7 ± 0.3, respectively, turned to be capable of salt formation (ionization) at pH levels close to neutral, which is evidence of weak acidic properties of quercetin (

Зенкевич И.Г., Гущина С.В. Определение констант диссоциации соединений, окисляющихся кислородом воздуха в водных растворах (на примере кверцетина). Журн.

аналит. химии. - 2010. - Т. 65, № 4. - С. 382 - 387.). By changing polarity of these and other hydroxylic groups of quercetin by selection and use of alkaline agents and setting appropriate pH values of solutions of solid dispersion it is possible to achieve their improved stability and reduce the content of disintegration products.

It is known from the documents (I.G. Zenkevich, A.Y Eshchenko, S.V. Makarova, A.G. Vitenberg, Y.G. Dobryakov, C.A. Utsal. Identification of the products of oxidation of quercetin by air oxygen at ambient temperature. Molecules. - 2007. - V. 12. - P. 654 - 672.; R. Sokokova, S. Ramesova, I. Degaano, M. Hromadova, M. Gal, J. Zabka, The oxidation of natural flavonoid quercetin. Chem. Commun.- 2012. - V. 48. - P. 3433 - 3435.; R. Alvarez-Diduk, M.T. Ramirez-Silva, A. Galano, A. Merkoci, Deprotonation mechanism and acidity constants in aqueous solution of flavonols: a combined experimental and theoretical study. J. Phys. Chem. - 2013. - V B 117. - P. 12347 - 12359.) that pH value of the reaction medium should be strictly controlled for obtaining solid dispersions of quercetin and preparation of their solutions. Quercetin is stable in acidic environment, while in pH range between 7 and 11 the amount of impurities increases critically as a result of oxidative degradation of quercetin, which takes place as early as after 15 min of exposure, and at pH values above 11, very fast decomposition of quercetin molecule will begin. Decomposition of quercetin is supposed to be determined by presence of dissolved oxygen in solutions. Such oxidation processes result in formation of substantial amount of products of various chemical nature. Therefore, in preparing solid dispersion of quercetin in PVP as a substance for lyophilized injectable preparation, it is desirable that all process operations for production and storage should be performed in inert atmosphere, for example under nitrogen or other inert gas.

The use of particular alkaline agents taking into account the values of acidity potential (pKa) and the number of their equivalents relative to the number of equivalents of quercetin in the mass of solid dispersion with a certain selected ratio of weight fractions of quercetin : PVP allows to achieve the desirable stability of SDQ PVP, i.e. stability of solid dispersion in both solid and dissolved states. Critical prerequisite consists in maintenance of optimum pH values of SDQ PVP aqueous solutions within narrow limits of 6.6 to 6.9, with concentration of quercetin ranging from 0.5 mg/ml to 2.5 mg/ml and more as a stability factor for injectable forms of the preparation to be produced.

It should also be taken into account that preparing a solution of lyophilized sterile medicinal product with solid dispersion of quercetin in PVP for intravenous administration is carried out in 0.9 % solution of sodium chloride (normal saline), which, according to compendial requirements, may have pH values in the range of 5.7 to 7.0, therefore, the dissolution of solid dispersion in such medium may lead to significant deviations in pH values of the prepared solutions, and such changes in pH may be critical for maintaining physicochemical stability of solutions of solid dispersion and fast formation of quercetin decomposition products.

According to the present invention, series of alkalies of inorganic and organic nature, that were unable to form insoluble salts with quercetin and other components of solid dispersion (SD), were used as alkaline (basic) agents (BAs), in particular, BAs with increasing acidity constant value pKa (increasing basic properties) such as: sodium hydrocarbonate ($NaHCO_3$), sodium carbonate ($Na_2CO_3$), L-arginine and sodium hydroxide (NaOH). Anyway, it is stipulated that other alkaline agents may also be used, provided that they do not form insoluble salts with quercetin and other SD components.

It was surprisingly found that changes in pKa of alkaline agent and in quantitative Q:PVP ratio on SD composition have an impact on the number of equivalents of alkaline agent, which is necessary to obtain solid dispersion with a predetermined pH value of their solutions, which also determines resistance of SD components to decomposition during their production, storage and use. In particular, it was found that amounts of alkaline agent are directly proportional to the number of equivalents of quercetin taken for preparation of the necessary weight of SDQ PVP and depend insignificantly on the acidity value of the particular alkaline agent and weight fraction of PVP in SDQ PVP of certain Q:PVP ratio. Or, in other words, the content of alkaline agent in SD is directly dependent on the number of weight equivalents of quercetin in SD, which is determined by the factor k representing a ratio of the number of equivalents of the selected alkaline agent [gE] ($*10^{-2}$) to 1 [gE] of quercetin.

The established dependencies of the number of gE of alkaline agent on the component ratio in SD and pH values of solutions with a mean concentration of quercetin 2.5 mg/ml are presented in Table 1 and in FIG. 1.

**Table 1**

| Ratios of gE numbers of alkaline agent and quercetin necessary to provide pH values of 5.5 - 8.0 in SDQ PVP solutions with quercetin concentration of 2.5 mg/ml. | | | | | | | | | | | | |
|---|---|---|---|---|---|---|---|---|---|---|---|---|
| Q:PVP | k - average number of gE of alkaline agent to 1 gE of quercetin ($*10^{-2}$) | | | | | | | | | | | |
| SDQ PVP 1:7 | 0.60 | 2.60 | 3.44 | 4.16 | 4.90 | 5.32 | 5.72 | 6.12 | 6.66 | 7.42 | 8.56 | 10.6 |
| SDQ PVP 1:9 | 0.80 | 2.92 | 3.92 | 4.62 | 5.46 | 5.88 | 6.30 | 6.72 | 7.36 | 8.14 | 9.32 | 11.4 |
| SDQ PVP 1:12 | 0.80 | 3.20 | 4.42 | 5.16 | 5.92 | 6.56 | 7.08 | 7.60 | 8.26 | 9.14 | 10.48 | 13.0 |

(continued)

| pH values of SDQ PVP solutions with a mean quercetin concentration of 2.5 mg/ml | | | | | | | | | | | |
|---|---|---|---|---|---|---|---|---|---|---|---|
| pH | 5.5 | 6.0 | 6.2 | 6.4 | 6.6 | 6.7 | 6.8 | 6.9 | 7.0 | 7.2 | 7.5 | 8.0 |

To determine stability and minimize growth of quercetin decomposition products as impurities in the resulting solutions of SDQ PVP samples with different pH values, their stability was checked experimentally, and optimum pH values of those solutions were determined. It was found that aqueous solutions of SDQ PVP of the composition Q:PVP:k [BA] are physically instable and disintegrate shortly after their preparation with release of quercetin as a solid phase at weakly acidic pH values of 5.5 - 6.2; the solutions are physically stable at weakly alkaline pH values within the range of 7.2 - 8.0, but quercetin, which is stable in acidic environment, disintegrates rather quickly being oxidized with air oxygen with formation of a series of oxidation products as impurities within this pH range.

SDQ PVP solutions with pH values within the range of 6.6 - 7.2 proved to be most optimal in terms of stability and quality.

In view of the obtained data, the initial pH range for aqueous solutions of solid dispersions was narrowed to the values 6.75 ± 0.15 that may be considered as optimal for Q:PVP ratios within the range of 1:7 - 1:12.

The obtained experimental data allowed to determine the value of proportionality factor k for the ratio of equivalents of alkaline agent and quercetin in SD to provide for pH range for aqueous solutions of solid dispersions at the level of 6.75 ± 0.15, the factor k being in the range: (6.3 ± 0.5) $*10^{-2}$gE of alkaline agent per 1 gE of quercetin. The results for dependency of the number of gE of the alkaline agent on the component ratio in SD and acidity constants pKa are presented in Table 2.

Table 2

| **Ratios of the number of gE of alkaline agents and quercetin necessary to provide pH values of 6.75 ± 0.15 in solutions in SDQ PVP with quercetin concentration of 2.5 mg/ml.** | | | | | |
|---|---|---|---|---|---|
| Q:PVP ratio in SDQ PVP | pKa | Alkaline agent | Ratio factor k of the number of gE of alkaline agent ($*10^{-2}$) to 1 gE of quercetin | | average pH |
| 1:7 | 6.4 | $NaHCO_3$ | 5.92 | 6.16 ± 0.32 | 6.83 |
| 1:9 | | | 6.08 | | 6.79 |
| 1:12 | | | 6.48 | | 6.72 |
| 1:7 | 10.33 | $Na_2CO_3$ | 5.86 | 6.24 ± 0.46 | 6.82 |
| 1:9 | | | 6.14 | | 6.75 |
| 1:12 | | | 6.70 | | 6.78 |
| 1:7 | 12.50 | L-arginine | 6.30 | 6.45 ± 0.25 | 6.83 |
| 1:9 | | | 6.36 | | 6.76 |
| 1:12 | | | 6.80 | | 6.75 |
| 1:7 | 14.77 | NaOH | 6.28 | 6.40 ± 0.20 | 6.85 |
| 1:9 | | | 6.32 | | 6.78 |
| 1:12 | | | 6.60 | | 6.79 |

In Table 2, alkaline agents are ordered by increasing of values of acidity constants pKa (increase of basicity of alkalies) top down. Taking into account increase of basicity (pKa), alkaline agents were used in obtaining the aqueous solutions of SDQ PVP in wide physiological pH range from 5.5 to 8.0. It was surprisingly found that the factor k was in the range (6.3 ± 0.5) $10^{-2}$ gE of alkaline agent per 1 gE of quercetin and was hardly dependent on the nature of the alkaline agent used.

In scaling-up production of solid dispersions of quercetin in PVP, calculation of the amount of the selected alkaline agent m(BA) can be performed according to the formula (1):

$$m(BA) = \frac{k * m(Q)}{\mathrm{E}_m(Q)} * \mathrm{E}_m(BA) (1)$$,

where:

*m(Q) - weight of quercetin in SD, g;*
*$E_m(Q)$ and $E_m(BA)$ - equivalent weights Q (quercetin) and BA (alkaline agent), respectively, gE/mol;*
*m(BA) - necessary weight of the respective alkaline agent, g;*
*k - ratio factor of the number of equivalents of the selected alkaline agent [gE](*$10^{-2}$) in relation to 1 [gE] of quercetin of certain SD of Q:PVP, necessary for providing optimal pH value of 6.75 ± 0.15 of aqueous solution of SD with quercetin concentration of 2.5 mg/ml;*

For acid-base reaction, which takes place here, equivalent weight of the element or molecule equals to molecular weight *M(X)* divided by the number of $H^+$ ions formed or consumed during the reaction, which is an equivalence factor $f_{eq}$ *(X).* Accordingly, for acid it is the weight giving one mole of $H^+$, while for base it is the weight neutralizing one mole of $H^+$.
Therefore, equivalent weights of quercetin and alkaline agent $E_m(X)$ can be calculated according to the formula (2):

$$E_m(X) = \frac{M(X)}{f_{\text{екв}}(X)} (2)$$

For quercetin $f_{eq}(Q)$ = 5, for $NaHCO_3$, NaOH, L-arginine $f_{eq}(X)$= 1, for $Na_2CO_{3feq}(Na_2CO_3)$= 2, respectively.
For example, for quercetin, upon substitution of values in the formula (2), we get:

$$E_m(Q) = \frac{M(Q)}{f_{\text{екв}}(Q)} = \frac{302,23}{5} = 60,446$$

*M(Q) = 302.23 - molecular weight quercetin, g/mol;*
*$E_m(Q)$ = 60.446 gE/mol*;

For alkaline agents, upon substitution of values in the formula (2), we get:

$E_m$(NaOH) = *40 gE/mol;*
$E_m(Na_2CO_3)$=*53 gE/mol;*
$E_m(NaHCO_3)$= *84 gE/mol;*
$E_m$(L-Arg)=*174.2 gE/mol.*

Additionally, dependency of factor k on the weight fraction of PVP in the solid dispersion and the strength of basicity of alkaline agents was studied (see FIG. 2), and it was found that, within the selected pH range (6.75 ± 0.15), the factor k is increased proportionally to increase of the weight fraction of PVP in the solid dispersion, and increase of the factor k is observed to a greater extent for alkaline agents 3 with more basic properties.
Obviously, increase of the necessary amount of alkaline agent with increasing fraction of PVP in SDQ PVP is determined by weak acidic valued of its aqueous solutions (pH 4 - 5). At the same time, for alkaline agents with insignificant values of basicity constants ($NaHCO_3$ and $Na_2CO_3$), such dependency is almost linear, while for alkaline agents with higher values of basicity constants (L-arginine, NaOH), greater correction was required for production of SDs with fixed pH value, which probably may be attributed to their nonspecific interaction with hydrophilic matrix of PVP.
Strong alkaline properties of sodium hydroxide determine its use exclusively as a solution, which will require selection of its concentration and provides for control of the addition rate.

## Experimental

The following abbreviations are used throughout the description and examples:

| Abbreviation or special term | Explanations |
|---|---|
| SD | solid dispersion |
| SDQ PVP | solid dispersion of quercetin with polyvinyl pyrrolidone |
| Q | quercetin |
| $Q_{dih}$. | quercetin dihydrate |
| PVP | polyvinyl pyrrolidone |
| BA | alkaline agent |
| k | ratio factor of the number of equivalents of the selected alkaline agent [gE](* $10^{-2}$) to 1 [gE] of quercetin |
| Q:PVP·k[BA] | solid dispersion of quercetin with polyvinyl pyrrolidone with a use of alkaline agents |
| pKa | acidity constant value |
| pH | power of hydrogen |
| HPLC | high performance liquid chromatography |
| DSC | differential scanning calorimetry |
| TG | thermogravimetric analysis |
| UV spectroscopy | ultraviolet spectroscopy |
| FTIR | Fourier-transform infrared spectrophotometry |
| eq | equivalent |
| gE | gram-equivalent |
| mgE | milligram-equivalent |
| Min | minute |
| °C | degree Celsius |
| 1N solution | normal solution |

**Materials and methods**

In obtaining the solid dispersion of quercetin in polyvinyl pyrrolidone and medicinal products based thereon, the following reagents were used: Quercetin 99.7 % (PJSC Scientific Industrial Center (SIC) "Borshchahivskiy Chemical-Pharmaceutical Plant", Ukraine); Polyvinyl pyrrolidone PVP (Kollidon® K17, BASF SE, Germany); Sodium hydroxide NaOH, CP («SPOLCHEMIE», Czech Republic); Sodium carbonate, $Na_2CO_3$ CP, Sodium bicarbonate $NaHCO_3$, AR grade. (Scientific-Production Enterprise "Alfarus", Ukraine); L-Arginine Pharma Grade EP, USP, (Ajinomoto, Japan);

Compendial grade croscarmellose («JRS Pharma GmbH & Co. KG», Germany); Microcrystalline cellulose MКЦ-102, compendial grade («JRS Pharma GmbH & Co. KG», Germany); Talc («Imerys Talc Italy S.p.a.», Italy); Corn starch («Tate&Lyle Netherlands B.V», Netherlands); Ethanol 96 % (high-purity solvents, State-Owned Enterprise "UKRSPYRT"", Stadnytsia Village, Ukraine).

**EXAMPLES**

Solid dispersions of quercetin in PVP were obtained with their ratio in the range of 1:7 to 1:12 and with a use of the following compounds as alkaline agents: NaOH, L-arginine, $Na_2CO_3$ and $NaHCO_3$.

Necessary amount of alkaline agent to obtain solid dispersion with the desirable pH value in aqueous solutions depends on the number of equivalents of quercetin in the weight fraction of quercetin in SDQ PVP, weight fraction of PVP and the selected BA, using the following ratio: $(6.3 \pm 0.5) *10^{-2}$gE of alkaline agent per 1 gE of quercetin.

Calculation of the necessary amount of alkaline agent m(BA) was performed according to the formula (1):

$$m(BA) = \frac{k * m(Q)}{E_m(Q)} * E_m(BA)(1)$$

The developed method of obtaining SDQ PVP of the composition Q:PVP k[BA] includes various options for obtaining reaction mixtures with the necessary ratio of components in aqueous-alcoholic solutions having ethanol content in the range of 70 ± 15 %, with stirring and at the process temperature in the range of 20 to 45 °C.

Various options for obtaining SDs include selecting the sequence of addition of components to aqueous-alcoholic solution of PVP with the content thereof from 175 to 200 mg/ml, which was prepared first, or simultaneous charging of components to a reaction vessel, dissolution in common solvent (aqueous ethanol), stirring, evaporation of the solvent, drying, granulation, sifting and storage. Operations for obtaining were carried out under inert atmosphere and without isolation of the reaction from exposure to atmospheric oxygen. To that end, two studies were carried out in parallel, with identical conditions, the amount of the charged components, solvents and methods of charging, specifically under inert atmosphere and in normal conditions. A device for carrying out reactions under inert atmosphere was additionally equipped with h-nozzle for passing inert gas (nitrogen, argon) to displace atmospheric oxygen and create inert atmosphere in the reaction medium, all other conditions being unchanged.

Solid dispersions obtained according to the present invention were studied for content of quercetin decomposition impurities based on conditions of their obtaining and contribution of the stages, where possibility of impurity increment is the highest, in particular, during dissolution and mixing the components (see Table 6), solvent evaporation and drying (see Table 7), as well as at storage of the obtained solid dispersions.

The k ratios of the number of gEs of alkaline agent and quercetin for obtaining aqueous solutions of SDQ PVP with a concentration of 2.5 mg/ml of quercetin within the wide range of pH values (values in the range of 5.5 - 8.0) are presented in Table 1 for obtaining aqueous solutions with optimum pH values (values in the range of 6.75 ± 0.15) are presented in Table 2.

Preparation of SDQ PVP with the specific Q:PVP ratio was carried out using the determined aliquots of components and alkaline agents, with adherence to k factors presented in Tables 1 and 2, respectively.

Depending on the method of obtaining the solid dispersions, duration of the process was determined, the duration of obtaining solid dispersions was 30 to 140 min, preferably 40 to 100 min, wherein the shortest duration of obtaining the end product was in case of simultaneous charging the components.

Operations for evaporation of reaction mixtures were each time carried out in vacuum under residual pressure of 10 - 12 mbar at the heating bath temperature of 40 ± 10 °C.

The resulting amorphous, foamed precipitates of solid dispersions were subjected to further drying in a vacuum drying oven at 60 °C to constant weight, ground in a mortar, sifted through 60 mesh sieve, placed in containers to be closed airtight, and stored for further studies.

Each operation, which provided for quality assessment of SDQ PVP, consisted in stability testing of the obtained intermediate and end products, with determining time span from dissolution of solid dispersion and formation of a clear solution to beginning of its disintegration, which is accompanied by turbidity and sedimentation of quercetin.

**Obtaining solid dispersions of quercetin in polyvinyl pyrrolidone for predetermined Q:PVP k[BA] ratios with varying sequences of addition of components.**

**Method 1. Obtaining solid dispersions of quercetin in polyvinyl pyrrolidone with the following sequence of addition of components: PVP + BA + Q.**

An aliquot of the selected alkaline agent, as calculated by formula (1) is added with stirring to the prepared aqueous-alcoholic (ethanol 70 ± 15 %) solutions of PVP with a concentration in the range of 140 to 240 mg/ml under inert atmosphere or in normal conditions, with adherence to the determined k factor and the ratio of weight fractions of components Q:PVP in the solid dispersion, specifically for 1:7, 1:9 and 1:12, as provided in Table 2. An aliquot of weight fraction of quercetin is added to the prepared solutions. The mixture is stirred for 30 to 40 min at a temperature of 20 - 40 °C to complete dissolution of components, and the solution is filtered.

The resulting solutions is evaporated on a rotary evaporator, with or without prior displacement of atmospheric air with inert gas, in vacuum under residual pressure of 10 - 12 mbar and at heating bath temperature of 40 ± 10 °C until solvent is removed completely. The resulting SD as foamed amorphous glasslike yellow powders are dried in a vacuum drying oven at 60 °C to constant weight, ground in a mortar, sifted through 60 mesh sieve, placed in containers to be closed airtight, and stored for further studies.

**EXAMPLE 1.1.**

**Obtaining a solid dispersion Q:PVP 1:7 k[$NaHCO_3$].**

100 ml of 72 % aqueous ethanol (high-purity solvents, State-Owned Enterprise "UKRSPYRT", Stadnytsia Village, Ukraine) is poured to 250 ml flask, 14.0 g of PVP (Kollidon® K17 from BASF SE, Germany) is added and dissolved with stirring using IKA® RCT basic safety control magnetic stirrer with heating. 0.165 g (1.96 mgE) of $NaHCO_3$ is added to the solution and stirred to complete dissolution of alkaline agent. 2.0 g (33.09 mgE) of quercetin is added to the resulting solution, the mixture of components is stirred at a temperature of 40 °C to obtain clear solution.

The resulting yellow solution is filtered using glass S4 Schott filtering funnel («Simax», Czech Republic), filtrate is evaporated on Büchi R-134 rotary evaporator (BÜCHI Labortechnik AG (Switzerland)) under 10 - 12 mbar vacuum at water bath temperature of 45 °C, until solvent is removed completely with formation of foamed mass of SD.

Glasslike foamed mass (16 g) is dried at 50°C in a vacuum drying oven «SHELLAB 1465» from Sheldon Manufacturing Inc., Oregon, USA, to constant weight, ground in a porcelain mortar, sifted through 60 mesh sieve, placed to a container to be closed airtight, and stored for further studies.

**EXAMPLE 1.1i.**

**Obtaining a solid dispersion Q:PVP 1:7 k[$NaHCO_3$] under inert atmosphere (argon).**

100 ml of 72 % aqueous ethanol is poured to 250 ml flask equipped with h-nozzle for feeding the inert gas, 14.0 g of PVP is added and purged with 5 l/min jet of argon gas for 5 min, then gas flow is reduced to 50-100 ml/min and PVP precipitate is dissolved with stirring in a magnetic stirrer. 0.165 g (1.96 mgE) of $NaHCO_3$ is added to PVP solution and stirred to complete dissolution of alkaline agent. 2.0 g (33.09 mgE) of quercetin is added to the resulting solution and the mixture of components is stirred at a temperature of 40 °C to complete dissolution. After 10 min quercetin precipitate is dissolved substantially completely and the solution is stirred for more 30 min. The resulting yellow solution is filtered through a glass inline filler, filtrate is evaporated under 10 - 12 mbar vacuum on a rotary evaporator prepurged with argon at water bath temperature of 45 °C until solvent is removed completely with formation of solid foamed mass of solid dispersion.

Glasslike foamed mass (16 g) is dried at 50 °C to constant weight in a vacuum drying oven, where slight flow of argon gas is created, then ground in a porcelain mortar, sifted through 60 mesh sieve, placed to a container to be closed airtight, and stored for further studies.

**EXAMPLES 1.2 - 1.16 and 1.2i - 1.16i.**

**Obtaining SDQ PVP with the sequence of addition of components PVP + BA + Q under inert atmosphere and in normal conditions.**

In Examples 1.2 - 1.6 and 1.2i - 1.6i, solid dispersions were obtained with Q:PVP ratio of 1:7 with alkaline agents such as $NaHCO_3$, $Na_2CO_3$, L-arginine base, NaOH (1N solution), and using quercetin in the form of dihydrate (Example 1.5 and 1.5i and 1.6 and 1.6i). Preparation conditions, equipment and reagents were similar to those indicated in Examples 1.1 and 1.1i, and the amounts of components taken are as indicated in Table 3, in lines corresponding to numbers of Examples.

In Examples 1.7 - 1.16 and 1.7i - 1.16i solid dispersions were obtained with Q:PVP ratios of 1:9 and 1:12 with alkaline agents such as $NaHCO_3$, $Na_2CO_3$, L-arginine base, NaOH (1N solution) and using quercetin in the form of dihydrate (Examples 1.11 and 1.11i, and 1.16 and 1.16i) under conditions similar to those indicated in Examples 1.1 and 1.1i except that at constant weight fraction of quercetin in the solid dispersion, the weight fraction of PVP was increased to the respective proportion with increase of its concentration in alcoholic solution; the amount of the respective alkaline agent was calculated according to the value of k factor for selected Q:PVP ratios and the alkaline agent, as shown in Table 2. The components used, their amounts and preparation conditions specified in Table 3, in lines corresponding to numbers of Examples.

As expected, the yield of solid dispersions and their aggregate state are not dependent on conditions of gaseous environment in the reaction vessel; however, providing the inert atmosphere has significant impact on impurity content (see Table 6).

Summary data on the numbers of the used components under successive addition of components PVP + BA + Q, their ratio and the number of obtained dispersions under inert atmosphere and in normal conditions are presented below in Table 3.

Table 3

| Examples of obtaining solid dispersions of quercetin in polyvinyl pyrrolidone with the following sequence of addition of components: PVP + BA + Q | | | | | | |
|---|---|---|---|---|---|---|
| **Example No.** | **Q:PVP** | **Alkaline agent, (mgE)** | **PVP, g** | **Quercetin, g, (mgE)** | **Ethanol, ml, %** | **Yield of solid dispersion, g** |
| 1.1; 1.1i | 1:7 | $NaHCO_3$, 0.165 g (1.96) | 14.0 | 2.0 (33.09) | 100 (72) | 16 |
| 1.2; 1.2i | | $Na_2CO_3$, 0.103 g (1.94) | 14.0 | 2.0 (33.09) | 100 (70) | 16 |
| 1.3; 1.3i | | L-arginine, 0.363 g (2.08) | 14.0 | 2.0 (33.09) | 100 (70) | 16.2 |
| 1.4; 1.4i | | NaOH 2.08 ml (2.08), 1N solution | 14.0 | 2.0 (33.09) | 100 (72) | 16 |
| 1.5; 1.5i | | $NaHCO_3$, 0.165 g (1.96) | 14.0 | 2.24 (33.09) dihydrate | 100 (72) | 16 |
| 1.6; 1.6i | | NaOH 2.08 ml (2.08), 1N solution | 14.0 | 2.24 (33.09) dihydrate | 100 (72) | 16 |
| 1.7; 1.7i | 1:9 | $NaHCO_3$, 0.169 g (2.01) | 18.0 | 2.0 (33.09) | 100 (72) | 20 |
| 1.8; 1.8i | | $Na_2CO_3$, 0.108 g (2.03) | 18.0 | 2.0 (33.09) | 100 (70) | 20 |
| 1.9; 1.9i | | L-arginine, 0.367 g (2.1) | 18.0 | 2.0 (33.09) | 100 (70) | 20.2 |
| 1.10; 1.10i | | NaOH 2.09 ml (2.09), 1N solution | 18.0 | 2.0 (33.09) | 100 (72) | 20 |
| 1.11; 1.11i | | $Na_2CO_3$, 0.108 g (2.03) | 18.0 | 2.24 (33.09) dihydrate | 100 (70) | 20 |
| 1.12; 1.12i | 1:12 | $NaHCO_3$, 0.18 g (2.14) | 24.0 | 2.0 (33.09) | 100 (72) | 26 |
| 1.13; 1.13i | | $Na_2CO_3$, 0.118 g (2.22) | 24.0 | 2.0 (33.09) | 100 (70) | 26 |
| 1.14; 1.14i | | L-arginine, 0.392 g (2.25) | 24.0 | 2.0 (33.09) | 100 (70) | 26.3 |
| 1.15; 1.15i | | NaOH 2.18 ml (2.18), 1N solution | 24.0 | 2.0 (33.09) | 100 (72) | 26 |
| 1.16; 1.16i | | L-arginine, 0.392 g (2.25) | 24.0 | 2.24 (33.09) dihydrate | 100 (70) | 26.3 |

**Method 2. Obtaining solid dispersions of quercetin in polyvinyl pyrrolidone with the following sequence of addition of components: PVP + Q + BA.**

In the method of obtaining solid dispersions, with the sequence of addition of components PVP + Q + BA under inert atmosphere and in normal conditions, aliquots of PVP and quercetin in the desirable ratio of weight fractions Q:PVP, specifically for 1:7; 1:9 and 1:12, are successively dissolved with stirring and heating in 70 ± 15 % aqueous ethanol. In such case, PVP content in aqueous-alcoholic solution was in the range of 140 - 240 mg/ml, depending on the weight fraction of PVP in the solid dispersion. An aliquot of alkaline agent, as calculated by formula (1), is added to the resulting suspension of components with stirring, with adherence to the determined k factor for the selected alkaline agent and the ratio of weight fractions of components Q:PVP in the solid dispersion, in accordance with Table 2.

The resulting solution is evaporated on a rotary evaporator under 10 - 12 mbar vacuum at a heating bath temperature of 40 ± 10 °C until solvent is removed completely. Amorphous, foamed powders of SD obtained under inert

atmosphere or in normal conditions are dried to constant weight in a vacuum drying oven at 50 ± 10 °C, ground in a porcelain mortar, sifted through 60 mesh sieve, placed to a container to be closed airtight, and stored for further studies.

**EXAMPLE 2.1**

**Obtaining a solid dispersion of Q:PVP 1:7 k[$NaHCO_3$].**

90 ml of 70 % aqueous ethanol is poured to 250 ml flask, 14.0 g of PVP is added and dissolved with stirring and heating to a temperature of 40 °C. 2.0 g (33.09 mgE) of quercetin is added to the formed solution of PVP, the mixture is stirred at a temperature of 40 °C for 30 min. 0.165 g (1.96 mgE) of $NaHCO_3$ is added to the resulting suspension of components, the reaction is stirred for 40 min to complete dissolution of components. The resulting yellow clear solution is filtered, the filtrate is evaporated under 10 - 12 mbar vacuum on a rotary evaporator at water bath temperature of 45 °C until solvent is removed completely with formation of yellow glasslike foamed mass of solid dispersion. The glasslike mass (16 g) is dried to constant weight in a vacuum drying oven at 50 °C, ground in a mortar, sifted through 60 mesh sieve, placed to a container to be closed airtight, and stored for further studies.

**EXAMPLE 2.1i**

**Obtaining a solid dispersion of Q:PVP 1:7 k[$NaHCO_3$] under inert atmosphere (argon).**

90 ml of 70 % aqueous ethanol is poured to 250 ml flask equipped with h-nozzle for feeding the inert gas, 14.0 g of PVP is added and purged with 5l/min jet of argon gas for 5 min, then gas flow is reduced to 50 - 100 ml/min, PVP precipitate is dissolved with stirring and heating in a magnetic stirrer at a temperature of 40 °C. 2.0 g (33.09 mgE) quercetin is added to the resulting PVP solution, the mixture is stirred at a temperature of 40 °C for 30 min. 0.165 g (1.96 mgE) of $NaHCO_3$ is added to the suspension of components and the mixture is stirred for 40 min to complete dissolution of components. The resulting yellow solution is filtered through a glass inline filler, filtrate is evaporated under 10 - 12 mbar vacuum on a rotary evaporator prepurged with inert argon gas at water bath temperature of 45 °C until solvent is removed completely with formation of solid dispersion as yellow glasslike foamed mass.

The glasslike foamed mass (16 g) is dried to constant weight in a vacuum drying oven at 50 °C, wherein a weak (1 - 5 ml/min) flow of argon gas is created, ground in a porcelain or glass mortar, sifted through 60 mesh sieve, placed to a container to be closed airtight, and stored for further studies.

**EXAMPLES 2.2 - 2.16 and 2.2i - 2.16i.**

**Obtaining SDQ PVP with the following sequence of addition of components: PVP+ Q + BA.**

In Examples 2.2 - 2.6 and 2.2i - 2.6i solid dispersions are obtained with Q:PVP ratios of 1:7 and with alkaline agents such as $NaHCO_3$, $Na_2CO_3$, L-arginine base, NaOH (1N solution), and using quercetin in the form of dihydrate (Example 2.5 and 2.5i and 2.6 and 2.6i). Preparation conditions were similar to those indicated in Examples 2.1 and 2.1i, and the amounts of components taken were as indicated in Table 4, in lines corresponding to numbers of Examples.

In Examples 2.7 - 2.16 and 2.7i - 2.16i solid dispersions are obtained with Q:PVP ratios of 1:9 and 1:12 and with alkaline agents such as $NaHCO_3$, $Na_2CO_3$, L-arginine base, NaOH (1N solution) and using quercetin in the form of dihydrate (Examples 2.11 and 2.11i and 2.16 and 2.16i) under conditions similar to those indicated in Examples 2.1 and 2.1i, except that at constant weight fraction of quercetin in the solid dispersion, the weight fraction of PVP was increased proportionally to the increase in its concentration in alcoholic solution; the amount of the respective alkaline agent was calculated according to the value of k factor for selected ratios of Q:PVP and alkaline agent, as shown in Table 2. The components used, their amounts and conditions are presented in Table 4, in lines corresponding to numbers of Examples.

Summary data on the numbers of the used components with the sequence of addition of components PVP + Q + BA, their ratio and the number of obtained dispersions are presented below in Table 4.

Table 4

| Examples of obtaining solid dispersions of quercetin in polyvinyl pyrrolidone with the following sequence of addition of components: PVP + Q + BA | | | | | | |
|---|---|---|---|---|---|---|
| **Example No.** | **Q:PVP** | **Alkaline agent, g, (mgE)** | **PVP, g** | **Quercetin, g, (mgE)** | **Ethanol, ml (%)** | **Yield of solid dispersion, g** |
| 2.1; 2.1i | 1:7 | $NaHCO_3$, 0.165 g (1.96) | 14.0 | 2.0 (33.09) | 90 (70) | 16 g |
| 2.2; 2.2i | | $Na_2CO_3$, 0.103 g (1.94) | 14.0 | 2.0 (33.09) | 90 (72) | 16 g |
| 2.3; 2.3i | | L-arginine, 0.363 g, (2.08) | 14.0 | 2.0 (33.09) | 100 (70) | 6.2 g |
| 2.4; 2.4i | | NaOH 2.08 ml (2.08), 1N solution | 14.0 | 2.0 (33.09) | 100 (70) | 6 g |
| 2.5; 2.5i | | NaOH 2.08 ml (2.08), 1N solution | 14.0 | 2.24 (33.09) dihydrate | 90 (70) | 6 g |
| 2.6; 2.6i | | $NaHCO_3$, 0.165 g (1.96) | 14.0 | 2.24 (33.09) dihydrate | 90 (70) | 16 g |
| 2.7; 2.7i | 1:9 | $NaHCO_3$, 0.169 g (2.01) | 18.0 | 2.0 (33.09) | 90 (70) | 20 g |
| 2.8; 2.8i | | $Na_2CO_3$, 0.108 g (2.03) | 18.0 | 2.0 (33.09) | 90 (72) | 20 g |
| 2.9; 2.9i | | L-arginine, 0.367 g (2.1) | 18.0 | 2.0 (33.09) | 100 (70) | 20.2 g |
| 2.10; 2.10i | | NaOH 2.09 ml (2.09), 1N solution | 18.0 | 2.0 (33.09) | 100 (70) | 20 g |
| 2.11; 2.11i | | $Na_2CO_3$, 0.108 g (2.03) | 18.0 | 2.24 (33.09) dihydrate | 100 (72) | 20 g |
| 2.12; 2.12i | 1:12 | $NaHCO_3$, 0.18 g (2.14) | 24.0 | 2.0 (33.09) | 100 (70) | 26 g |
| 2.13; 2.13i | | $Na_2CO_3$, 0.118 g (2.22) | 24.0 | 2.0 (33.09) | 100 (70) | 26.3 g |
| 2.14; 2.14i | | L-arginine, 0.392 g (2.25) | 24.0 | 2.0 (33.09) | 100 (70) | 26 g |
| 2.15; 2.15i | | NaOH 2.18 ml (2.18), 1N solution | 24.0 | 2.0 (33.09) | 100 (72) | 26 g |
| 2.16; 2.16i | | L-arginine, 0.392 g (2.25) | 24.0 | 2.24 (33.09) dihydrate | 100 (70) | 26.3 g |

**Method 3. Obtaining solid dispersions of quercetin in polyvinyl pyrrolidone with codissolution of all components PVP, Q and BA.**

Aliquots of quercetin and PVP with the selected ratio of weight fractions Q:PVP in the range of 1:7 to 1:12 and the amount of the selected alkaline agent, which was calculated using the formula (1), with adherence to k factor in the range of $(6.3 \pm 0.5) * 10^{-2}$ gE of alkaline agent per 1 gE of quercetin, depending on the selected alkaline agent and the ratio of weight fractions of components Q:PVP in SDQ PVP (in accordance with Table 2) are charged to a reaction vessel with atmospheric air, or inert atmosphere is created therein. The predetermined amount of 70 $\pm$ 15 % ethanol is added in the vessel to provide PVP content in the aqueous-alcoholic solution in the range of 140 - 240 mg/ml and dissolution is carried out with stirring and heating at a temperature of 40 $\pm$ 10 °C for 40 min

The resulting solutions is filtered and evaporated to dryness on a rotary evaporator under 10 - 12 mbar vacuum at a heating bath temperature 50 $\pm$ 10 °C. The resulting glasslike, amorphous, foamed precipitates of yellow solid dispersion

are dried to constant weight in a vacuum drying oven at 50 ± 10 °C, ground in a glass or porcelain mortar and sifted through 60 mesh sieve, placed to a container to be closed airtight, and stored for further studies.

**EXAMPLE 3.1**

**Obtaining a solid dispersion Q:PVP 1:7 k[$NaHCO_3$].**

14.0 g of PVP, 2.0 g (33.09 mgE) of quercetin and 0.165 g (1.96 mgE) of $NaHCO_3$ are charged into 250 ml flask, 90 ml of 70 % aqueous ethanol is added and dissolved with stirring and heating at a temperature of 40 °C. Then, when after 10 minutes the components of the solid dispersion are dissolved almost completely, the reaction is stirred for another 30 min. The resulting yellow solution is filtered, the filtrate is evaporated under 10 - 12 mbar vacuum on a rotary evaporator at a heating bath temperature of 40 ± 10 °C until solvent is removed completely with formation of glasslike foamed mass of yellow solid dispersion. The glasslike yellow mass is dried to constant weight (≈ 16 g) in a vacuum drying oven at 50 °C, ground in a porcelain mortar, sifted through 60 mesh sieve, placed to a container to be closed airtight, and stored for further studies.

**EXAMPLE 3.1i.**

**Obtaining a solid dispersion Q:PVP 1:7 k[$NaHCO_3$] under inert atmosphere (argon).**

14.0 g of PVP, 2.0 g (33.09 mgE) of quercetin and 0.165 g (1.96 mgE) of $NaHCO_3$ are charged into 250 ml flask equipped with h-nozzle for feeding the inert gas, 90 ml of 70 % aqueous ethanol is added and purged with 5 l/min jet of argon gas for 5 min. Then mixture in the vessel is dissolved with stirring and heating at a temperature of 45 °C, with passing argon gas flow at a rate of 50 - 100 ml/min. After 10 minutes, the components of solid dispersion are dissolved almost completely and the reaction is stirred for another 30 min The resulting yellow clear solution is filtered through a glass inline filler, filtrate is evaporated until solvent is removed completely under 10 - 12 mbar vacuum on a rotary evaporator prepurged with argon at water bath temperature of 45 °C, with formation of glasslike foamed mass of yellow solid dispersion. The glasslike mass is dried at 50 °C to constant weight (≈ 16 g) in a vacuum drying oven, wherein a weak (1 - 5 ml/min) flow of argon gas is created, ground in a porcelain mortar, sifted through 60 mesh sieve, placed to a container to be closed airtight, and stored for further studies.

**EXAMPLES 3.2 - 3.12 and 3.2i - 3.12i.**

**Obtaining SDQ PVP with codissolution of all components of SD: mixture of PVP, Q and BA.**

In Examples 3.2 - 3.4 and 3.2i - 3.4i, solid dispersions are obtained with Q:PVP ratio of 1:7 and alkaline agents such as $NaHCO_3$, $Na_2CO_3$ and L-arginine base, and using quercetin in the form of dihydrate (Example 3.4 and 3.4i). The conditions for obtaining solid dispersions were similar to those indicated in Examples 3.1 and 3.1i, and the amounts of components taken are as indicated in Table 5, in lines corresponding to numbers of Examples.

In Examples 3.5 - 3.12 and 3.5i - 3.12i solid dispersions are obtained with Q:PVP ratios of 1:9 and 1:12 and alkaline agents such as $NaHCO_3$, $Na_2CO_3$, L-arginine base, and using quercetin in the form of dihydrate (Examples 3.8 and 3.12 and 3.8i and 3.12i) under conditions similar to those indicated in Examples 3.1 and 3.1i, except that at constant weight fraction of quercetin in the solid dispersion, the weight fraction of PVP was increased to the respective proportion with increase of its concentration in alcoholic solution; the amount of the respective alkaline agent was calculated according to the value of k factor for selected Q:PVP ratios and the alkaline agent, as shown in Table 2. The components used, their amounts, conditions of obtaining and yields of solid dispersions are presented in Table 5, in lines corresponding to numbers of Examples.

Summary data on the numbers of the used components with codissolution of all components PVP, Q and BA, their ratio and the weight of the resulting dispersions are presented below in Table 5.

**Table** 5.

| Examples of obtaining solid dispersions of quercetin in polyvinyl pyrrolidone with codissolution of the mixture of all components PVP, Q and BA. | | | | | | |
|---|---|---|---|---|---|---|
| **Example No.** | **Q:PVP** | **Alkaline agent, g, (mgE)** | **PVP, g** | **Quercetin, g, (mgE)** | **Ethanol, ml, (%)** | **Yield of solid dispersion, g** |
| 3.1; 3.1i | 1:7 | $NaHCO_3$, 0.165 g (1.96) | 14.0 | 2.0 g (33.09) | 90 (70) | 16 g |
| 3.2; 3.2i | | $Na_2CO_3$, 0.103 g (1.94) | 14.0 | 2.0 g (33.09) | 90 (70) | 16 g |
| 3.3; 3.3i | | L-arginine, 0.363 g (2.08) | 14.0 | 2.0 g (33.09) | 100 (72) | 16.2 g |
| 3.4; 3.4i | | $NaHCO_3$, 0.165 g (1.96) | 14.0 | 2.24 g (33.09) dihydrate | 90 (70) | 16 g |
| 3.5; 3.5i | 1:9 | $NaHCO_3$, 0.169 g (2.01) | 18.0 | 2.0 g (33.09) | 100 (72) | 20 g |
| 3.6; 3.6i | | $Na_2CO_3$, 0.103 g (1.94) | 18.0 | 2.0 g (33.09) | 90 (72) | 19.8 g |
| 3.7; 3.7i | | L-arginine, 0.367 g (2.1) | 18.0 | 2.0 g (33.09) | 100 (72) | 20.2 g |
| 3.8; 3.8i | | $Na_2CO_3$, 0.108 g (2.03) | 18.0 | 2.24 g (33.09) dihydrate | 90 (72) | 20.3 g |
| 3.9; 3.9i | 1:12 | $NaHCO_3$, 0.18 g (2.14) | 24.0 | 2.0 g (33.09) | 100 (72) | 26 g |
| 3.10; 3.10i | | $Na_2CO_3$, 0.118 g (2.22) | 24.0 | 2.0 g (33.09) | 100 (72) | 26 g |
| 3.11; 3.11i | | L-arginine, 0.392 g (2.25) | 24.0 | 2.0 g (33.09) | 100 (72) | 26.1 g |
| 3.12; 3.12i | | L-arginine, 0.392 g (2.25) | 24.0 | 2.24 g (33.09) dihydrate | 100 (72) | 26.2 g |

**Studying the effect of atmospheric oxygen on impurities content in preparation of solid dispersions using Methods 1 - 3.**

To study the effect of atmospheric oxygen on impurities content in solid dispersions, samples of solid dispersions obtained under normal conditions of possible contact of reaction mixture with atmospheric oxygen as described in Examples 1.1 - 1.16, 2.1 - 2.16 and 3.1 - 3.12, as well as samples of solid dispersions obtained under argon atmosphere as described in Example 1.1i - 1.16i, 2.1i - 2.16i and 3.1i - 3.12i were analyzed.

Determination of the total content of impurities, without their identification, was performed using HPLC method, wherein aqueous-alcoholic solutions after codissolution operations (Table 6) and precipitates of solid dispersion after coevaporation operations (Table 7), that were obtained both under inert atmosphere and in the presence of atmospheric oxygen, were used for analysis.

Chromatographic study of SDQ PVP samples by HPLC method was carried out using a liquid chromatograph Agilent 1260 Infinity VL (Agilent Technologies, Germany) under the following conditions: chromatographic column Kromasil®100-5-C18 HPLC, 150 x 4.6 mm (Nouryon, Separation Products, Sweden); particle size: 5 μm; column temperature: 25°C; detection wavelength: 254 nm; injection volume: 10 μL; mobile phase rate: 1 ml/min; mobile phase: 0.1 % phosphoric acid in water - mobile phase A, methanol - mobile phase B; gradient elution: 20 % of mobile phase B (1 min), 20 - 80 % of mobile phase B (16 min), 80 - 20 % of mobile phase B (3 min), 20 % of mobile phase B (6 min). The results of the study are presented below in Tables 6 and 7.

Table 6.

| Content of quercetin decomposition impurities in aqueous-alcoholic solutions in preparation of solid dispersion Q:PVP k[BA] after codissolution operation. | | | | | | | | | | |
|---|---|---|---|---|---|---|---|---|---|---|
| Q:PVP | Alkaline agent (BA) | PVP+BA+Q Method 1 | | | PVP+Q+BA Method 2 | | | Codissolution of mixture of Q, PVP and BA Method 3 | | |
| | | Example No. | Total impurities, % | | Example No. | Total impurities, % | | Example No. | Total impurities, % | |
| | | | Air | Argon | | Air | Argon | | Air | Argon |
| 1:7 | $NaHCO_3$ | 1.1; 1.1i | 0.13 | 0.08 | 2.1; 2.1i | 0.17 | 0.11 | 3.1; 3.1i | 0.14 | 0.09 |
| 1:7 | $Na_2CO_3$ | 1.2; 1.2i | 0.15 | 0.07 | 2.2; 2.2i | 0.19 | 0.14 | 3.2; 3.2i | 0.17 | 0.12 |
| 1:7 | L-arginine | 1.3; 1.3i | 0.09 | 0.04 | 2.3; 2.3i | 0.16 | 0.10 | 3.3; 3.3i | 0.08 | 0.05 |
| 1:7 | NaOH | 1.4; 1.4i | 0.21 | 0.11 | 2.4; 2.4i | 0.21 | 0.13 | - | - | - |
| $Q_{dih.}$:PVP 1:7 | $NaHCO_3$ | 1.5; 1.5i | 0.13 | 0.07 | 2.5; 2.5i | 0.17 | 0.11 | 3.4; 3.4i | 0.13 | 0.09 |
| $Q_{dih.}$:PVP 1:7 | NaOH | 1.6; 1.6i | 0.22 | 0.11 | 2.6; 2.6i | 0.22 | 0.14 | - | - | - |
| 1:9 | $NaHCO_3$ | 1.7; 1.7i | 0.11 | 0.06 | 2.7; 2.7i | 0.12 | 0.06 | 3.5;3.5i | 0.13 | 0.08 |
| 1:9 | $Na_2CO_3$ | 1.8; 1.8i | 0.13 | 0.08 | 2.8; 2.8i | 0.21 | 0.12 | 3.6; 3.6i | 0.15 | 0.09 |
| 1:9 | L-arginine | 1.9; 1.9i | 0.06 | 0.02 | 2.9; 2.9i | 0.12 | 0.06 | 3.7; 3.7i | 0.08 | 0.06 |
| 1:9 | NaOH | 1.10; 1.10i | 0.23 | 0.09 | 2.10; 2.10i | 0.27 | 0.16 | - | - | - |
| $Q_{dih.}$:PVP 1:9 | $Na_2CO_3$ | 1.11; 1.11i | 0.14 | 0.08 | 2.11; 2.11i | 0.18 | 0.13 | 3.8; 3.8i | 0.17 | 0.12 |
| 1:12 | $NaHCO_3$ | 1.12; 1.12i | 0.13 | 0.08 | 2.12; 2.12i | 0.14 | 0.07 | 3.9; 3.9i | 0.12 | 0.07 |
| 1:12 | $Na_2CO_3$ | 1.13; 1.13i | 0.21 | 0.12 | 2.13; 2.13i | 0.23 | 0.12 | 3.10; 3.10i | 0.20 | 0.12 |
| 1:12 | L-arginine | 1.14; 1.14i | 0.10 | 0.06 | 2.14; 2.14i | 0.11 | 0.05 | 3.11; 3.11i | 0.08 | 0.06 |
| 1:12 | NaOH | 1.15; 1.15i | 0.22 | 0.12 | 2.15; 2.15i | 0.21 | 0.14 | - | - | - |
| $Q_{dih.}$:PVP 1:12 | L-arginine | 1.16; 1.16i | 0.10 | 0.07 | 2.16; 2.16i | 0.16 | 0.10 | 3.12; 3.12i | 0.08 | 0.06 |

Table 7.

| Content of quercetin decomposition impurities in samples of solid dispersions Q:PVP k[BA] after coevaporation operations. | | | | | | | | | | |
|---|---|---|---|---|---|---|---|---|---|---|
| Q:PVP | Alkaline agent (BA) | PVP+BA+Q Method 1 | | | PVP+Q+BA Method 2 | | | Codissolution of mixture of PVP, Q and BA Method 3 | | |
| | | Example No. | Total impurities, % | | Example No. | Total impurities, % | | Example No. | Total impurities, % | |
| | | | Air | Argon | | Air | Argon | | Air | Argon |
| 1:7 | $NaHCO_3$ | 1.1; 1.1i | 0.27 | 0.24 | 2.1; 2.1i | 0.32 | 0.21 | 3.1; 3.1i | 0.25 | 0.18 |
| 1:7 | $Na_2CO_3$ | 1.2; 1.2i | 0.31 | 0.27 | 2.2; 2.2i | 0.41 | 0.24 | 3.2; 3.2i | 0.34 | 0.22 |
| 1:7 | L-arginine | 1.3; 1.3i | 0.24 | 0.19 | 2.3; 2.3i | 0.28 | 0.20 | 3.3; 3.3i | 0.22 | 0.14 |
| 1:7 | NaOH | 1.4; 1.4i | 0.45 | 0.38 | 2.4; 2.4i | 0.47 | 0.32 | - | - | - |

(continued)

| Content of quercetin decomposition impurities in samples of solid dispersions Q:PVP k[BA] after coevaporation operations. | | | | | | | | | | |
|---|---|---|---|---|---|---|---|---|---|---|
| Q:PVP | Alkaline agent (BA) | PVP+BA+Q Method 1 | | | PVP+Q+BA Method 2 | | | Codissolution of mixture of PVP, Q and BA Method 3 | | |
| | | Example No. | Total impurities, % | | Example No. | Total impurities, % | | Example No. | Total impurities, % | |
| | | | Air | Argon | | Air | Argon | | Air | Argon |
| $Q_{dih.}$:PVP 1:7 | $NaHCO_3$ | 1.5; 1.5i | 0.26 | 0.23 | 2.5; 2.5i | 0.30 | 0.20 | 3.4; 3.4i | 0.26 | 0.18 |
| $Q_{dih.}$:PVP 1:7 | NaOH | 1.6; 1.6i | 0.43 | 0.37 | 2.6; 2.6i | 0.45 | 0.31 | - | - | - |
| 1:9 | $NaHCO_3$ | 1.7; 1.7i | 0.25 | 0.17 | 2.7; 2.7i | 0.26 | 0.19 | 3.5;3.5i | 0.23 | 0.18 |
| 1:9 | $Na_2CO_3$ | 1.8; 1.8i | 0.30 | 0.23 | 2.8; 2.8i | 0.61 | 0.32 | 3.6; 3.6i | 0.26 | 0.19 |
| 1:9 | L-arginine | 1.9; 1.9i | 0.23 | 0.16 | 2.9; 2.9i | 0.25 | 0.11 | 3.7; 3.7i | 0.22 | 0.15 |
| 1:9 | NaOH | 1.10; 1.10i | 0.33 | 0.21 | 2.10; 2.10i | 0.67 | 0.35 | - | - | - |
| $Q_{dih.}$:PVP 1:9 | $Na_2CO_3$ | 1.11; 1.11i | 0.32 | 0.27 | 2.11; 2.11i | 0.42 | 0.27 | 3.8; 3.8i | 0.43 | 0.27 |
| 1:12 | $NaHCO_3$ | 1.12; 1.12i | 0.34 | 0.22 | 2.12; 2.12i | 0.26 | 0.18 | 3.9; 3.9i | 0.12 | 0.07 |
| 1:12 | $Na_2CO_3$ | 1.13; 1.13i | 0.42 | 0.25 | 2.13; 2.13i | 0.53 | 0.32 | 3.10; 3.10i | 0.20 | 0.12 |
| 1:12 | L-arginine | 1.14; 1.14i | 0.23 | 0.12 | 2.14; 2.14i | 0.18 | 0.14 | 3.11; 3.11i | 0.08 | 0.06 |
| 1:12 | NaOH | 1.15; 1.15i | 0.57 | 0.35 | 2.15; 2.15i | 0.64 | 0.41 | - | - | - |
| $Q_{dih.}$:PVP 1:12 | L-arginine | 1.16; 1.16i | 0.23 | 0.19 | 2.16; 2.16i | 0.26 | 0.20 | 3.12; 3.12i | 0.11 | 0.08 |

**Study of** pH levels of aqueous solutions of solid dispersions prepared by Methods 1 - 3, according to the present invention.

To study pH levels of aqueous solutions of solid dispersions, both samples of solid dispersions obtained under normal conditions of possible contact of reaction mixture with atmospheric oxygen as described in Examples 1.1 - 1.16, 2.1 - 2.16 and 3.1 - 3.12 and samples of solid dispersions obtained under argon inert gas as described in Example 1.1i - 1.16i, 2.1i - 2.16i and 3.1i - 3.12i were used, all other conditions being unchanged.

Measuring pH values of aqueous solutions of solid dispersions with quercetin concentration of 2.5 mg/ml was carried out using 692 pH/Ionmeter Metrohm (Metrohm AG Company, Switzerland). Prior to measuring, pH-meter was precalibrated using standard buffered salines. Temperature of aqueous solutions for pH measuring was maintained in the range of 25 ± 1 °C using a thermostated bath. Measuring pH values of solutions of each sample was carried out in triplicate, arithmetic mean values of the measuring are shown in Table 8.

**Example of measuring pH value of aqueous solution of SDQ PVP obtained as described in Example 1.1 - Q:PVP 1:7 with quercetin concentration of 2.5 mg/ml.**

An aliquot 500 mg of solid dispersion obtained in Example 1.1, which contains 62.5 mg of quercetin is placed to 25 mm amber glass measuring flask. 20 ml of purified water is added in the flask, then stirred in a thermostated bath at 25 °C to complete dissolution of precipitate and the volume of liquid in the flask is made up to the mark with purified water.

pH value of the solution is measured using 692 pH/Ionmeter / Metrohm with constant stirring. pH measurements of 1.1 Q:PVP 1:7 sample solution are carried out for three parallel aliquots, the results of mean values for three consecutive

measurements are presented in Table 8.

**Example of measuring pH value of aqueous solution of SDQ PVP obtained as described in Example 1.2 - 1.16, 2.1 - 2.16 and 3.1 - 3.12, and Examples 1.1i - 1.16i, 2.1i - 2.16i and 3.1i - 3.12i.**

Similarly to the above study of solid dispersion obtained as described in Example 1.1 (pH measurement of aqueous solution of SD with quercetin concentration of 2.5 mg/ml), pH measurements of aqueous solutions of all samples of SD obtained as described in Examples 1.1 - 1.16, 2.1 - 2.16 and 3.1 - 3.12, and samples of solid dispersions obtained under inert argon gas as described in Example 1.1i - 1.16i, 2.1i - 2.16i and 3.1i - 3.12i, are carried out, whereby aliquots of solid dispersions with Q:PVP ratios of 1:7; 1:9; 1:12, that contain 62.5 mg of quercetin are placed to 25 ml measuring flasks, dissolved with stirring and thermostating in 20 ml of purified water, made up to the mark and pH measurements are carried out.

The results of mean values for three consecutive measurements pH of aqueous solutions samples SDQ PVP with quercetin concentration of 2.5 mg/ml are presented in Table 8.

**Table 8.**

| pH values of aqueous solutions of solid dispersions samples. | | | | | | | | | | |
|---|---|---|---|---|---|---|---|---|---|---|
| Q:PVP | Alkaline agent (BA) | PVP+BA+Q Method 1 | | | PVP+Q+BA Method 2 | | | Codissolution of PVP, Q and BA Method 3 | | |
| | | Example No. | pH | | Example No. | pH | | Example No. | pH | |
| | | | Air | Argon | | Air | Argon | | Air | Argon |
| 1:7 | $NaHCO_3$ | 1.1; 1.1i | 6.84 | 6.84 | 2.1; 2.1i | 6.83 | 6.83 | 3.1; 3.1i | 6.83 | 6.83 |
| 1:7 | $Na_2CO_3$ | 1.2; 1.2i | 6.80 | 6.80 | 2.2; 2.2i | 6.82 | 6.82 | 3.2; 3.2i | 6.84 | 6.84 |
| 1:7 | L-arginine | 1.3; 1.3i | 6.83 | 6.83 | 2.3; 2.3i | 6.86 | 6.86 | 3.3; 3.3i | 6.78 | 6.78 |
| 1:7 | NaOH | 1.4; 1.4i | 6.88 | 6.88 | 2.4; 2.4i | 6.82 | 6.82 | - | - | - |
| $Q_{dih.}$:PVP 1:7 | $NaHCO_3$ | 1.5; 1.5i | 6.83 | 6.82 | 2.5; 2.5i | 6.80 | 6.81 | 3.4; 3.4i | 6.81 | 6.80 |
| $Q_{dih.}$:PVP 1:7 | NaOH | 1.6; 1.6i | 6.84 | 6.85 | 2.6; 2.6i | 6.77 | 6.78 | - | - | - |
| 1:9 | $NaHCO_3$ | 1.7; 1.7i | 6.83 | 6.83 | 2.7; 2.7i | 6.75 | 6.75 | 3.5;3.5i | 6.82 | 6.82 |
| 1:9 | $Na_2CO_3$ | 1.8; 1.8i | 6.70 | 6.70 | 2.8; 2.8i | 6.82 | 6.82 | 3.6; 3.6i | 6.79 | 6.79 |
| 1:9 | L-arginine | 1.9; 1.9i | 6.77 | 6.77 | 2.9; 2.9i | 6.78 | 6.78 | 3.7; 3.7i | 6.84 | 6.84 |
| 1:9 | NaOH | 1.10; 1.10i | 6.77 | 6.77 | 2.10; 2.10i | 6.83 | 6.83 | - | - | - |
| $Q_{dih.}$:PVP 1:9 | $Na_2CO_3$ | 1.11; 1.11i | 6.79 | 6.78 | 2.11; 2.11i | 6.82 | 6.83 | 3.8; 3.8i | 6.78 | 6.79 |
| 1:12 | $NaHCO_3$ | 1.12; 1.12i | 6.72 | 6.72 | 2.12; 2.12i | 6.71 | 6.71 | 3.9; 3.9i | 6.85 | 6.85 |
| 1:12 | $Na_2CO_3$ | 1.13; 1.13i | 6.80 | 6.80 | 2.13; 2.13i | 6.76 | 6.76 | 3.10; 3.10i | 6.78 | 6.78 |
| 1:12 | L-arginine | 1.14; 1.14i | 6.75 | 6.75 | 2.14; 2.14i | 6.73 | 6.73 | 3.11; 3.11i | 6.74 | 6.74 |
| 1:12 | NaOH | 1.15; 1.15i | 6.80 | 6.80 | 2.15; 2.15i | 6.78 | 6.78 | - | - | - |
| $Q_{dih.}$:PVP 1:12 | L-arginine | 1.16; 1.16i | 6.78 | 6.78 | 2.16; 2.16i | 6.83 | 6.82 | 3.12; 3.12i | 6.78 | 6.78 |

To sum up the above studies as to the effect of the conditions of obtaining a solid dispersion on its characteristics, the following conclusions can be made:

- solid dispersions obtained with a use of quercetin dihydrate and anhydrous quercetin were substantially identical by their characteristics and impurities content;
- total impurities content in SDQ PVP obtained by the method of codissolution with subsequent coevaporation did not exceed 0.67 %. The impurities content was slightly increased with increase in basicity of alkaline agents;
- when sodium hydrocarbonate ($NaHCO_3$) was used, total impurities content in SDQ PVP obtained by the method of codissolution with subsequent coevaporation did not exceed 0.34 %;
- the use of L-arginine surprisingly provided substantial decrease in the amount of impurities (at maximum 0.28 %); such result could be attributed to formation of intermolecular complex of basic amino acid with weak acidic phenolic groups of quercetin;
- preparation of SDQ PVP within the range of Q:PVP ratios from 1:7 to 1:12 with a use of alkaline agents in quantitative proportion indicated in Table 2 and according to the calculation by Formula (1) allowed to obtain solid dispersions with pH values of aqueous solutions, that were within the desired predetermined range of 6.75 $\pm$ 0.15.
- change in weight fractions of quercetin to PVP in SDQ PVP has little effect on impurities content in the resulting solid dispersions;
- preparation of solid dispersions according to the invention under inert atmosphere significantly decreases content of impurities of oxidative degradation of quercetin;
- in the methods of obtaining solid dispersions by separate dissolution of components, significant change in the amount of impurities is not observed; somewhat greater impurities content in the product obtained as described in Method 2 can be attributed to relatively longer duration of the process;
- long-term effect of high temperature on the process of preparation of solid dispersions according to the invention results in the increase of impurities content. Decrease in the total amount of impurities occurs in the following order: Method 3 < Method 1 < Method 2;

Method 3, which provides for codissolution and coevaporation of mixture components proved to be the most efficient in terms of process simplicity, shortened time of performance and the amount of impurities in the product.

**Study of physicochemical characteristics of solid dispersions having the following composition: Q:PVP k [BA].**

**Solubility study.**

Essential characteristics for creation of medicinal products in injectable form include completeness and rate of dissolution of the preparation with achievement of the effective therapeutic concentration of the active pharmaceutical ingredient, and stability of infusion solutions for sufficient time. Safety of such preparation in use is thereby ensured. In this connection, studies were carried out to investigate dissolution rate of the obtained solid dispersions with achievement of the therapeutic concentration of quercetin 0.5 mg/ml in the infusion solution, and their stability within a certain time range.

Studies of dissolution rate were according to requirements of State Pharmacopoeia of Ukraine, clause 2.9.3. of "Dissolution" section for solid dosage forms with a use of ERWEKA DT 700 dissolution tester equipped with a basket from ERWEKA GmbH. Samples of solid dispersions in the amount containing 250 mg of quercetin was placed in a cylindrical basket mounted on a shaft of the device. A vessel for dissolution was filled with 500 ml of degassed purified water. The basket with samples was stirred at 100 rpm. At set intervals of time (1, 2, 3, 4, 5, 10, 15, 20 min), 1 ml aliquote samples of the solution were taken through Mdi SYRINGE Filter SY25TG (PTFE+Prefilter), placed to a 100 ml measuring flask, immediately made up to the mark with purified water. The content of quercetin in the solution was determined by absorbance at 370 nm using LAMBDA™ 25 spectrophotometer from PerkinElmer, Inc. upon precalibration of quercetin solutions.

Studying dissolution kinetics is presented on the graphs of dissolution profiles (FIG. 3 and FIG. 4) plotted based on results of mean values of three sequential measurements.

Analysis of the results allows to conclude that the effect of basic properties of alkaline agents included in SDQ PVP at the same ratio of components Q:PVP on change in dissolution rate of those solid dispersions is insignificant (FIG. 3); furthermore, the rate of quercetin release from SDQ PVP during preparation of solutions with active ingredient concentration of 0.5 mg/ml only to a small degree depends on the choice of alkaline agent and on the weight fraction of hydrophilic solubilizer matrix (FIG. 3 and FIG. 4). Complete dissolution of solid dispersion with 90 % release of the active ingredient occurs as soon as after 5 min, and 100 % release of the active ingredient occurs in about 6 - 10 min from the beginning of dissolution.

**Study of physical stability of solutions of solid dispersions.**

The prepared solutions of solid dispersions with Q:PVP ratios of 1:7; 1:9; 1:12 in amber glass vials with quercetin

concentration of 2.5 mg/ml in purified water were stored in dark at room temperature 22 °C. pH values of solutions were within the set ranges 6.75 ± 0.15. During predetermined periods of storage, appearance of opalescence, turbidity and precipitate was observed.

Data on physical stability of quercetin solutions obtained from respective solid dispersions are presented in Table 9.

**Table 9.**

| Stability study of quercetin solutions of the obtained solid dispersions in different periods of time. | | | | | | | |
|---|---|---|---|---|---|---|---|
| SDQ PVP Q:PVP composition | Alkaline agent | Example | pH of solution | 1 day | 2 days | 3 days | 4 days |
| 1:7 | L-arginine | 1.3i | 6.83 | - | opalescence | **+** | **+** |
| 1:7 | $NaHCO_3$ | 2.1i | 6.83 | - | opalescence | **+** | **+** |
| 1:7 | NaOH | 2.4i | 6.82 | - | opalescence | **+** | **+** |
| 1:7 | $Na_2CO_3$ | 3.2i | 6.84 | - | opalescence | + | + |
| 1:9 | $Na_2CO_3$ | 1.8i | 6.7 | - | opalescence | + | + |
| 1:9 | NaOH | 1.10i | 6.77 | - | turbidity | + | + |
| $Q_{dih}$. 1:9 | $Na_2CO_3$ | 1.11i | 6.78 | - | opalescence | + | + |
| 1:9 | L-arginine | 2.9i | 6.78 | - | opalescence | + | + |
| 1:9 | $NaHCO_3$ | 3.5i | 6.82 | - | turbidity | + | + |
| 1:12 | $Na_2CO_3$ | 1.13i | 6.8 | - | - | - | opalescence |
| 1:12 | NaOH | 1.15i | 6.8 | - | - | - | opalescence |
| $Q_{dih}$. 1:12 | L-arginine | 2.16i | 6.82 | - | - | - | opalescence |
| 1:12 | $NaHCO_3$ | 3.9i | 6.85 | - | - | - | opalescence |
| 1:12 | L-arginine | 3.11i | 6.74 | - | - | - | opalescence |
| «-» - no precipitate; «+» - precipitate formed | | | | | | | |

Analysis of the results allows to conclude that solutions of solid dispersions having the composition Q:PVP 1:12, i.e. with maximum content of polyvinyl pyrrolidone, appeared to be most physically stable: the approximate time, during which the solution remains in clear state without opalescence and precipitate was 72 - 84 hours. They were followed by solutions of solid dispersions having the composition Q:PVP 1:9, that were stable for 24 - 30 hours, and solutions of solid dispersions having the composition Q:PVP 1:7 were less stable and maintained stability for 24 hours (Table 9 and FIG. 5).

Thus, the studied samples of solutions of solid dispersions remained stable, without opalescence, turbidity precipitation during 24 hours, which is acceptable for manufacture of infusion preparations.

**Studying the effect of degree of basic properties of alkaline agents on chemical stability of solid dispersions at storage.**

Samples of solid dispersions containing 250 mg of quercetin, having compositions Q:PVP 1:9 k[$NaHCO_3$] and Q:PVP 1:9 k[L-arginine], are selected by their storage time: immediately in obtaining, after 1 hour, after 6 hours and after 24 hours, placed to a container with 500 ml of purified water and dissolved at a temperature of 25 ± 0.5 °C with stirring at 100 rpm. 1 ml of each solution is taken, filtered it through a filter having pore diameter of 0.45 μm and dissolved in 100 ml of purified water, and content of quercetin is assessed by UV-spectrophotometry at wavelength $\lambda$ = 370 nm. The results of studies are presented in Table 10.

Table 10.

| Impurities content in solutions of solid dispersions depending on their storage time. | | | | | | |
|---|---|---|---|---|---|---|
| Q:PVP | Alkaline agent | Example | ∑ basic impurities | | | |
| | | | Storage time (hours) | | | |
| | | | 0 | 1 | 6 | 24 |
| 1:7 | $NaHCO_3$ | 2.1i | 0.21 | 0.27 | 0.33 | 0.41 |
| 1:9 | | 3.5i | 0.18 | 0.21 | 0.26 | 0.39 |
| 1:12 | | 3.9i | 0.07 | 0.15 | 0.22 | 0.34 |
| 1:7 | $Na_2CO_3$ | 3.2i | 0.22 | 0.31 | 0.43 | 0.58 |
| 1:9 | | 1.8i | 0.23 | 0.33 | 0.45 | 0.57 |
| 1:9 | | 1.11i | 0.27 | 0.35 | 0.47 | 0.61 |
| 1:12 | | 2.13i | 0.32 | 0.42 | 0.49 | 0.58 |
| 1:7 | L-arginine | 1.3i | 0.19 | 0.24 | 0.33 | 0.39 |
| 1:7 | | 2.3i | 0.11 | 0.15 | 0.25 | 0.35 |
| 1:9 | | 2.9i | 0.20 | 0.26 | 0.33 | 0.42 |
| 1:12 | | 3.11i | 0.06 | 0.14 | 0.22 | 0.33 |
| 1:7 | NaOH | 2.4i | 0.32 | 0.41 | 0.51 | 0.63 |
| 1:9 | | 1.10i | 0.35 | 0.43 | 0.59 | 0.65 |
| 1:12 | | 1.15i | 0.35 | 0.47 | 0.57 | 0.68 |

The obtained data are indicative of rather rapid increase in the amount of impurities in samples of solutions of solid dispersions with different alkaline agents (total amount of impurities increased about twofold in 24 hours). It was found that the respective correlation between increase in amounts of impurities and the degree of basic properties of alkaline agent was not observed. Significant difference in basicity of sodium bicarbonate (pKa 6.4) and L-arginine (pKa 12.5) was not a factor, which influenced chemical stability of the obtained solid dispersions during their storage. Somewhat higher stability of SD solutions with L-arginine as alkaline agent can be attributed to the stabilizing factor of formation of intermolecular hydrogen bonds between guanidine group of L-arginine molecule and molecule of quercetin.

**Analysis of IR spectra with Fourier transformation (FTIR) of quercetin, polyvinyl pyrrolidone, their physical mixture and solid dispersions according to the invention with different ratios of weight fractions Q:PVP.**

Fourier-transform infrared spectrophotometry (FTIR) was performed with a use of FTIR spectrophotometer Agilent Cary 630 from Agilent Technologies, Inc.

FTIR spectra of pure quercetin, polymer polyvinyl pyrrolidone K17 PF, physical mixture Q+PVP 1:9 and solid dispersions of quercetin in polyvinyl pyrrolidone with ratios of weight fractions 1:7, 1:9 and 1:12 were received using a disk method with KBr and they are presented in FIG. 6.

All determined objects of FTIR study were selected in order to establish interaction of intermolecular hydrogen bonds between quercetin molecule and polyvinyl pyrrolidone.

It is known that IR spectrum of anhydrous quercetin demonstrates wide intensive peaks of stretching and bending vibrations of hydroxylic groups at 3284 and 3381 $cm^{-1}$, that are capable of forming internal and intermolecular hydrogen bonds with phenolic -OH groups and aromatic ketonic fragment. Moreover, stretching vibrations peak >C=O of aryl ketonic group is at 1664 $cm^{-1}$. Characteristic absorbance peaks of quercetin molecule, that are related to stretching vibrations C=C of aromatic ring will develop at 1616, 1560 and 1511 $cm^{-1}$ (see Catauro, M., Papale, F., Bollino, F., et al. Silica/quercetin sol-gel hybrids as antioxidant dental implant materials. Sci. Technol. Adv. Mater. 2015. - V 16, 035001; Borghetti, G.S., Lula, I.S., Sinisterra, R.D., Bassani, VL., Quercetin/β-Cyclodextrin solid complexes prepared in aqueous solution followed by spray-drying or by physical mixture. AAPS Pharm. Sci. Tech. 2009.- V. 10, - P. 235 - 242.).

Stretching and bending vibrations C-H in aromatic nuclei of quercetin molecule determine the absorbance band at 1315 $cm^{-1}$, as well as peaks at 930, 818, 689 and 596 $cm^{-1}$.

It is found that polyvinyl pyrrolidone K17 PF develops characteristic peaks at 3431 $cm^{-1}$ and at 2957 $cm^{-1}$, caused

by stretching and bending vibrations of N-H and $C_{sp3}$-H, respectively. Characteristic peaks at 1658 and 1655 $cm^{-1}$ are determined by stretching and bending vibrations of amide carbonyl group and carbonyl group of PVP monomers, respectively. The peak at 1291 $cm^{-1}$ is characteristic of stretching vibrations of C-N bonds in polyvinyl pyrrolidone. (de Mello Costa A. R., Marquiafável F.S., Mirela Mara de Oliveira M.M. Quercetin-PVP K25 solid dispersion. J. Term. Anal. Calorimetre. - 2011. - V 104. - P. 273 - 278.; Bryaskova, R., Pencheva, D., Nikolov, S., Kantardjiev, T. Synthesis and comparative study on the antimicrobial activity of hybrid materials based on silver nanoparticles (AgNps) stabilized by polyvinylpyrrolidone (PVP). J. Chem. Biol. - 2011. - V 4, - P. 185 - 191; Zhu, J., Yang, Z., Chen, X., et. al. Preparation and physicochemical characterization of solid dispersion of quercetin and polyvinylpyrrolidone. J. Chin. Pharm. Sci.- 2007.- V. 16, P. 51 - 57).

FTIR spectra of SDQ PVP Q:PVP 1:7, Q:PVP 1:9 and Q:PVP 1:12 were compared to spectra of quercetin, PVP and their physical mixture of composition Q+PVP 1:9. In all cases spectra of SD were similar to the spectrum of polymer (peaks at 3435, 2958, 1659, 1495 $cm^{-1}$) (FIG. 6). Almost all peaks of quercetin were shifted and decreased in intensity or disappeared completely (intensive peaks at 1664, 1616, 1511 $cm^{-1}$), which is evidence of formation of hydrogen bond between quercetin and polyvinyl pyrrolidone.

FTIR spectra of physical mixture of composition Q+PVP 1:9 appeared to be only a sum of their individual absorbance spectra, which is evidence of absence of chemical interaction, i.e. absence of formation of intermolecular hydrogen bonds between quercetin and PVP.

**X-ray phase analysis of samples of quercetin and solid dispersions according to the invention**

X-ray powder diffraction study of samples of quercetin and SDQ PVP was performed using Siemens D500 X-ray powder diffraction apparatus (Siemens AG, Germany) (filtered CuK$\alpha$ radiation, Bragg-Brentano geometry). Phase identification was performed using the results of search in Cambridge Structural Database (CCDC) and PDF-1 index included in the diffractometer software.

X-ray powder diffractograms of samples of quercetin dihydrate (FIG. 7(A)) and quercetin double crystallized from ethanol (FIG. 7(B)) with presence of sharp, clear and intensive peaks are indicative of high crystallinity of samples.

In case of diffractograms of samples in FIG. 7(A), the structure of quercetin dihydrate determined by X-ray image data of the captured phase characteristics with parameters (cf. gr. P-1, a=13.060, b=16.564, c=3.725(7), $\alpha$=92.05(4), $\beta$=94.39(3), $\gamma$=120.55(3) is confirmed by CCDC=FERBEX (01) data, which is evidence of homogeneity of its structure. At the same time, X-ray image of sample 1 shows impurity lines of an unidentified phase, while samples 2 and 3 are pure (FIG. 7(A)).

On X-ray images of samples 4 and 6 of quercetin double crystallized from ethanol, phases present therein could not be identified, and none of the known phases of quercetin (dihydrate, monohydrate, anhydrous) at the location of the lines corresponds to the observed diffraction pattern. Sample 5 also contains unidentified lines, but dihydrate lines are observed therein as impurities (FIG. 7(B)). Such samples should be characterized as substances with polymorphous structure.

X-ray phase analysis of two samples of solid dispersions according to the invention of composition Q:PVP 1:9 k [L-arginine] (Sample 7) and Q:PVP 1:9 k[$NaHCO_3$] (Sample 8) showed absence of crystalline structure of quercetin in the solid dispersion, which is characteristic of solid solutions, thereby confirming amorphous state of quercetin in high molecular polyvinyl pyrrolidone (FIG. 7(C)).

**Differential scanning calorimetry (DSC)**

DSC studies of individual components of solid dispersions such as quercetin, polyvinyl pyrrolidone, their physical mixture and solid dispersions according to the invention, with different ratios of weight fractions Q:PVP were carried out using STA 449F1 NETZSCH device, which allowed to perform all study operations in automatic mode, from room temperature to 1500 °C under inert atmosphere. Accuracy of temperature measuring is 1.5 °C. Sensitivity of DSC signal recording is < 1 $\mu$W. Enthalpy measuring range covers from 1 to 30000 J/g, and thermal capacity range is from 10 to 5000 J/kg·K, respectively. In such case, accuracy of enthalpy and thermal capacity determination at the level of $\pm$ 3 % is provided.

Recording of DSC reflexes of the samples was carried out under inert atmosphere (Ar), flow rate 20 ml/min, heating rate of samples 10 K/min, temperature range 30 - 700 °C. DSC thermograms of pure quercetin, pure PVP, physical mixture and solid dispersions are presented in FIG. 8.

DSC curve of pure quercetin shows a narrow endothermic melting peak of quercetin at 324.1 °C, phase transition enthalpy is - 193.2 J/g. In case of physical mixture Q:PVP 1:9 (curve 5) this endothermic peak expands and shifts to the lower temperature region - 302.2 °C, phase transition enthalpy is only a third as much - 61.47 J/g. Complete disappearance of the endothermic peak, which corresponds to phase transition of quercetin, is observed in solid dispersions. This fact confirms that certain interaction appears between the hydrophobic active ingredient (quercetin) and hydrophilic carrier

matrix, and quercetin is in amorphous form in the solid dispersion. Further confirmation of interaction between the components of solid dispersion can be seen, when compared characteristics of DSC curves, from pure polyvinyl pyrrolidone to physical mixture quercetin + polyvinyl pyrrolidone and solid dispersions with the ratios of weight fractions of quercetin : polyvinyl pyrrolidone 1:7 and quercetin : polyvinyl pyrrolidone 1:9. With reduction of weight fraction of polyvinyl pyrrolidone in the solid dispersion disintegration enthalpy of polyvinyl pyrrolidone is decreased from -531.8 J/g for pure polyvinyl pyrrolidone to -301 J/g for solid dispersion of quercetin : polyvinyl pyrrolidone 1:9, further to -280.4 J/g for solid dispersion of quercetin : polyvinyl pyrrolidone 1:7, reaching its minimum of -268 J/g for physical mixture of quercetin + polyvinyl pyrrolidone 1:9.

**4. Method of obtaining water-soluble solid dispersions in sterile lyophilized form for preparation of injections.**

In accordance with Examples 1.1i - 1.16i, 2.1i - 2.16i and 3.1i - 3.12i substances of solid dispersions were obtained, that were used for manufacture of injectable drugs with observance of the conditions and sequence of operations for manufacture of sterile injectable dosage forms as indicated below.

Dissolution, sterilization of solutions, dispensing in sterile amber glass vials and final lyophilization drying under vacuum were performed in a box with laminar flow of sterile air. Dispensing solutions of solid dispersions according to the invention in vials was carried out taking into account the content per 1 dose of 50 mg quercetin.

Filtration sterilization of the resulting solutions was carried out in two stages: prefiltration was performed through nylon filter with pore diameter of 0.2 μm on «Технофильтр» (Tekhnofiltr) plate from "Scientific-Production Enterprise "Tekhnofiltr" LLC; final filtration was performed through cellulose acetate filter with pore diameter of 0.45 μm and 0.2 μm in sterile capsules (Pall/Sartodran, Sartorius) under excessive pressure of 2.2 bar.

Vials were filled with 4 ml of the solution of solid dispersion according to the invention, closed with rubber stoppers and placed in EPSILON 2-65 DSCHRIST freeze-drying unit from Martin Christ Gefriertrocknungsanlagen GmbH, Germany.

All operations for freeze drying samples were completed with vacuum relief in the freeze-drying unit by supplying purified dry nitrogen, capping and sealing the vials with aluminium flip-off caps. Vials transferred for storage and assay performance. The sequence of operations for lyophilization is presented in Table 11.

**Table 11.**

| Operations | Process parameters |
|---|---|
| I phase of freezing | 2 hrs, -40 °C |
| II phase of lyophilization | 30 min, -40 °C, 0.12 mbar |
| III phase of lyophilization | 2 hrs, -20 °C, 0.12 mbar |
| IV phase of lyophilization | 17 hrs, +10 °C, 0.12 mbar |
| V phase of drying | 2 hrs, +30 °C, 0.0023 mbar |

Alternatively, injectable forms of solid of the invention were obtained as described in the above described Methods 1, 2 or 3, wherein after vacuum evaporation of reaction mixtures, the resulting solid foamed mass, without discharging it out of the vessel for evaporation, was dissolved in distilled water, then the resulting solution of solid dispersion was processed with observance of the set sequence of operations for preparing sterile lyophilized injectable dosage forms, as specified above.

**EXAMPLE 4.1**

**Obtaining finished dosage form of sterile lyophilized preparation** of **solid dispersion with a use of the substance obtained as described in Method 3 (Example 3.1i).**

Substance of the solid dispersion of composition Q:PVP 1:7k[$NaHCO_3$], obtained as described in Method 3 (PVP+Q+BA, codissolution), Example 3.1i, in the amount of 13 g is dissolved in 50 ml of distilled water, volume of the solution is made up to 130 ml with distilled water (pH value of the solution is 6.78) and subjected to sterile filtration, the filtrate is dispensed in sterile 4 ml vials and, after their lyophilization, capped with rubber stoppers and rolled over. 32 vials of lyophilized preparation of solid dispersion according to the invention were obtained.

Samples obtained as described in Example 4.1 in the amount of 30 vials were used for further analyses of solubility values, pH values of solutions, their stability at storage and osmolality of solutions.

**EXAMPLE 4.2**

**Obtaining finished dosage form of sterile lyophilized preparation** of **solid dispersion with a use of the substance obtained as described in Method 1 (Example 1.2i).**

Substance of the solid dispersion of composition Q:PVP 1:7k[$Na_2CO_3$] obtained as described in Method 1 (PVP+BA+Q, sequentially), Example 1.2i, in the amount of 12 g is dissolved in 50 ml of distilled water, volume of the solution is made up to 120 ml with distilled water (pH value of the solution is 6.77) and subjected to sterile filtration, the filtrate is dispensed in sterile vials and, after their lyophilization, capped with rubber stoppers and sealed with aluminium flip-off caps. 30 vials of lyophilized preparation of solid dispersion according to the invention were obtained.

Samples obtained as described in Example 4.2 in the amount of 25 vials were used for further analyses of solubility values, pH values of solutions, their stability at storage and osmolality of solutions.

**EXAMPLE 4.3**

**Obtaining finished dosage form of sterile lyophilized preparation** of **solid dispersion with a use of the substance obtained as described in Method 3 (Example 3.3i).**

Substance of the solid dispersion of composition Q:PVP 1:7k[L-arginine], obtained as described in Method 3 (PVP+Q+BA, codissolution, Example 3.3i), in the amount of 13 g is dissolved in 50 ml of distilled water, volume of the solution is made up to 130 ml with distilled water (pH value of the solution is 6.72), subjected to sterile filtration, the filtrate is dispensed in sterile 4 ml vials and, after their lyophilization, capped with rubber stoppers and rolled over. 32 vials of lyophilized preparation of solid dispersion according to the invention were obtained.

Samples obtained as described in Example 4.3 in the amount of 30 vials were used for further analyses of solubility values, pH values of solutions, their stability at storage and osmolality of solutions.

**EXAMPLE 4.4**

**Obtaining finished dosage form of sterile lyophilized preparation** of **solid dispersion with a use of the substance obtained as described in Method 2 (Example 2.7i).**

Substance of the solid dispersion of composition Q:PVP 1:9k[$NaHCO_3$] obtained as described in Method 2 (PVP+Q+BA, sequentially), Example 2.6i, in the amount of 16.25 g is dissolved in 50 ml of distilled water, volume of the solution is made up to 130 ml with distilled water (pH value of the solution is 6.67), subjected to sterile filtration, the filtrate is dispensed in sterile 4 ml vials and, after their lyophilization, capped with rubber stoppers and rolled over. 32 vials of lyophilized preparation of solid dispersion according to the invention were obtained.

Samples obtained as described in Example 4.4 in the amount of 12 vials were used for further analyses of solubility values, pH values of solutions, their stability at storage and osmolality of solutions.

**EXAMPLE 4.5**

**Obtaining finished dosage form of sterile lyophilized preparation** of **solid dispersion as described in Method 3 without isolation of substance of solid dispersion.**

Substance of the solid dispersion of composition Q:PVP 1:9k[$Na_2CO_3$] is obtained as described in Method 3 (Q+PVP+BA, codissolution), Example 3.6i, except that after vacuum coevaporation of solvents the resulting solid foamed mass is dissolved without isolation in distilled water to obtain solution of solid dispersion with quercetin concentration of 12.5 mg/ml (pH 6.77). The solution is subjected to sterile filtration, dispensed in 4 ml y sterile vials and, after their lyophilization, capped with rubber stoppers and sealed with aluminium caps. 39 vials of lyophilized preparation of solid dispersion according to the invention were obtained.

Samples obtained as described in Example 4.5 in the amount of 15 vials were used for further analyses of solubility values, pH values of solutions, their stability at storage and osmolality of solutions.

**EXAMPLE 4.6**

**Obtaining finished dosage form of sterile lyophilized preparation of solid dispersion as described in Method 3 without isolation of substance of solid dispersion.**

Substance of the solid dispersion of composition Q:PVP 1:9k[L-arginine] is obtained as described in Method 3 (Q+PVP+BA, codissolution), Example 3.7i, except that after vacuum coevaporation of solvents, the resulting solid foamed mass is dissolved without isolation in distilled water to obtain solution of solid dispersion with quercetin concentration of 12.5 mg/ml (pH 6.75). The solution is subjected to sterile filtration, dispensed in 4 ml y sterile vials and, after their lyophilization, capped with rubber stoppers and sealed with aluminium flip-off caps. 39 vials of lyophilized preparation of solid dispersion according to the invention were obtained.

Samples obtained as described in Example 4.6 in the amount of 15 vials were used for further analyses of solubility values, pH values of solutions, their stability at storage and osmolality of solutions.

**EXAMPLE 4.7**

**Obtaining finished dosage form of sterile lyophilized preparation of solid dispersion with a use of the substance obtained as described in Method 1 (Example 1.12i).**

Substance of the solid dispersion of composition Q:PVP 1:12k[$NaHCO_3$] produced as described in Method 1 (PVP+BA+Q, sequentially), Example 1.12i, in the amount of 19.5 g is dissolved in 50 ml of distilled water, volume of the solution is made up to 120 ml with distilled water (pH value of the solution is 6.75), subjected to sterile filtration, the filtrate is dispensed in sterile 4 ml vials and, after their lyophilization, capped with rubber stoppers and sealed with aluminium flip-off caps. 29 vials of lyophilized preparation of solid dispersion according to the invention were obtained.

Samples obtained as described in Example 4.7 in the amount of 15 vials were used for further analyses of solubility values, pH values of solutions, their stability at storage and osmolality of solutions.

**EXAMPLE 4.8**

**Obtaining finished dosage form of sterile lyophilized preparation of solid dispersion with a use of the substance obtained as described in Method 3 (Example 3.10i).**

Substance of the solid dispersion of composition Q:PVP 1:12k[$Na_2CO_3$] obtained as described in Method 3 (PVP+Q+BA, codissolution), Example 3.10i, in the amount of 19.5 g is dissolved in 50 ml of distilled water, volume of the solution is made up to 120 ml with distilled water (pH value of the solution is 6.76), subjected to sterile filtration, the filtrate is dispensed in sterile 4 ml vials and, after their lyophilization, capped with rubber stoppers and sealed with aluminium flip-off caps. 30 vials of lyophilized preparation of solid dispersion according to the invention were obtained.

Samples obtained as described in Example 4.8 in the amount of 20 vials were used for further analyses of solubility values, pH values of solutions, their stability at storage and osmolality of solutions.

**EXAMPLE 4.9**

**Obtaining finished dosage form of sterile lyophilized preparation of solid dispersion as described in Method 3 without isolation of substance of solid dispersion.**

Substance of the solid dispersion of composition Q:PVP 1:12k[L-arginine] is obtained as described in Method 3 (Q+PVP+BA, codissolution), Example 3.11i, except that after vacuum coevaporation of solvents the resulting glasslike amorphous mass is dissolved without isolation in distilled water to obtain solution of solid dispersion with quercetin concentration of 12.5 mg/ml (pH 6.69). The solution is subjected to sterile filtration, dispensed in 4 ml y sterile vials and, after their lyophilization, capped with rubber stoppers and sealed with aluminium flip-off caps. 39 vials of lyophilized preparation of solid dispersion according to the invention were obtained.

Samples obtained as described in Example 4.9 in the amount of 15 vials were used for further analyses of solubility values, pH values of solutions, their stability at storage and osmolality of solutions.

**EXAMPLE 4.10**

**Obtaining finished dosage form of sterile lyophilized preparation of solid dispersion with a use of the substance obtained as described in Method 2 (Example 2.15i).**

Substance of the solid dispersion of composition Q:PVP 1:12 k[NaOH] obtained as described in Method 2

(PVP+Q+BA, sequentially), Example 2.15i, in the amount of 19.5 g is dissolved in 50 ml of distilled water, volume of the solution is made up to 120 ml with distilled water (pH value of the solution is 6.78), subjected to sterile filtration, the filtrate is dispensed in sterile 4 ml vials and, after their lyophilization, capped with rubber stoppers and sealed with aluminium flip-off caps. 29 vials of lyophilized preparation of solid dispersion according to the invention were obtained.

Samples obtained as described in Example 4.10, in the amount of 15 vials were used for further analyses of solubility values, pH values of solutions, their stability at storage and osmolality of solutions.

**5. Determination of the values of solubility, pH and stability of solutions of solid dispersions of sterile lyophilized forms with quercetin content of 0.5 mg/ml.**

Determination of solubility of samples of lyophilized sterile forms of solid dispersions obtained as described in Example 4.1 - 4.10 was carried out similarly to the method described above in section «*Study of physicochemical characteristics of solid dispersions having the following composition Q:PVP k[BA], Solubility study».* Characteristics of solubility of lyophilized sterile forms of solid dispersions as described in Examples 4.1 - 4.10 were substantially similar to respective solid dispersions as described in Examples 1.1i - 1.16i, 2.1i - 2.16i and 3.1i - 3.12i.

To study characteristics of physical stability of solutions of sterile forms of solid dispersions based on pH level of 0.9 % infusion solution of sodium chloride, samples of solid dispersions of sterile lyophilized forms obtained as described in Example 4.1 - 4.10 were dissolved in parallel in previously prepared 0.9 % solutions of sodium chloride 3 with pH values of 5.5, 6.2 and 7.0. The resulting solutions with quercetin concentration of 0.5 mg/ml were left standing at room temperature in dark place and checked after specified time for changes in physical condition such as appearance of opalescence, turbidity or sedimentation of disperse phase particles.

Besides, similar solutions of solid dispersions obtained as described in Example 4.1 - 4.10 in previously prepared 0.9 % infusion solutions of sodium chloride 3 with pH values of 5.5, 6.2 and 7.0 were prepared in parallel to determine their acidity.

**Preparation of 0.9 % infusion solution of sodium chloride with pH 5.50; 6.20; 7.00**

For preparation of solution, 1000 ml measuring flask, precision class A, sodium chloride NaCl classified as CP (manufacturer Akzo Nobel Salt A/S, Denmark), 0.1 M solutions of NaOH and HCl, 692 pH/Ionmeter / Metrohm pH-meter and Metrohm 702 SM Titrino autotitrator were used. Before starting, pH-meter is calibrated using standard buffered salines (manufacturer Lab Logistics Group GmbH) with the following pH values: 4.00; 7.00; 10.00 at a temperature of 25 °C, instrumental error ± 0.02.

9.045 g (9.0 g equivalent to 100 % substance) of sodium chloride NaCl classified as CP (manufacturer «Akzo Nobel Salt A/S, Denmark») is placed to 1000 ml class A measuring flask, about 600 ml of water for injection is added and solution is stirred to complete dissolution of precipitate at room temperature, with avoiding intense agitation. After dissolution, the volume of solution in the flask is made up to the mark with water for injection and stirred. Acidity of the resulting solution is determined with a precalibrated pH-meter, pH = 5.87.

300 ml samples of the prepares solution are placed in three 500 ml glass vessels and pH values of solutions are adjusted to 5.50, 6.20 and 7.00, respectively, with 0.1 M of NaOH and HCl solutions using Metrohm 702 SM Titrino autotitrator with constant stirring in a magnetic stirrer.

Vessels with solutions are closed with glass stoppers and stored before use.

The results of determination of physical stability characteristics of solutions of lyophilized SDQ PVP with quercetin content of 0.5 mg/ml and their pH values are presented in FIG. 9 - 11 and in Table 12.

**Table 12**

| pH values of solutions of sterile lyophilized forms of solid dispersions obtained with a use of normal 0.9 % solutions of NaCl with pH values of 5.5; 6.2; 7.0 | | | | |
|---|---|---|---|---|
| Example No. | Q:PVP k[BA] | pH 5.5 | pH 6.2 | pH 7.0 |
| | | pH of solutions samples | | |
| 4.1 | 1:7 $NaHCO_3$ | 6.69 | 6.72 | 6.74 |
| 4.2 | 1:7 $Na_2CO_3$ | 6.73 | 6.74 | 6.75 |
| 4.3 | 1:7 L-arginine | 6.68 | 6.71 | 6.72 |
| 4.4 | 1:9 $NaHCO_3$ | 6.68 | 6.69 | 6.73 |
| 4.5 | 1:9 $Na_2CO_3$ | 6.68 | 6.69 | 6.73 |

(continued)

| pH values of solutions of sterile lyophilized forms of solid dispersions obtained with a use of normal 0.9 % solutions of NaCl with pH values of 5.5; 6.2; 7.0 | | | | |
|---|---|---|---|---|
| Example No. | Q:PVP k[BA] | pH 5.5 | pH 6.2 | pH 7.0 |
| | | pH of solutions samples | | |
| 4.6 | 1:9 L-arginine | 6.67 | 6.69 | 6.71 |
| 4.7 | 1:12 $NaHCO_3$ | 6.68 | 6.70 | 6.71 |
| 4.8 | 1:12 $Na_2CO_3$ | 6.67 | 6.68 | 6.70 |
| 4.9 | 1:12 L-arginine | 6.67 | 6.69 | 6.69 |
| 4.10 | 1:12 NaOH | 6.73 | 6.75 | 6.77 |

After pH measuring, it was found that lyophilized sterile forms of solid dispersions having the composition Q:PVP k[BA] according to the invention had buffering properties maintaining pH level of injectable solutions in the range of 6.67 to 6.77, irrespectively of pH values of the used 0.9 % infusion solution of sodium chloride, which significantly improved stability and reliability of such systems.

**6. Studying buffer capacity of solutions of solid dispersions in 0.9 % infusion solutions of sodium chloride 3 with pH values of 5.5, 6.2 and 7.0.**

To study buffer capacity of solutions of solid dispersions obtained according to the invention in 0.9 % solutions of sodium chloride 3 with pH values of 5.5, 6.2 and 7.0 on the samples as described in Examples 4.3 and 4.4, pH values of SD solutions were determined in the wide range of concentrations of the active substance (quercetin).

All pH measurements are performed using 692 pH/Ionmeter / Metrohm pH-meter. Before starting, pH-meter is calibrated at a temperature of 25 °C using standard buffered salines (manufacturer Lab Logistics Group GmbH) with the following pH values: 4.00; 7.00; 10.00; instrumental error ± 0.02.

**Determination methodology.**

Three 10 ml measuring flasks are sequentially charged with samples of solid dispersion as described in Example 4.3 with quercetin content of 200 mg, and 8 ml of infusion solution with pH values of 5.50; 6.20 and 7.00 are added in each flask. Precipitates in flasks are dissolved with stirring and the volume is made up to the mark with infusion solution having appropriate pH value, stirred, and pH value of the sample with quercetin content of 20 mg/ml is determined. The data are entered in the table. Then, using the method of sampling and dissolution in the precise amount of 0.9 % sodium chloride solution with appropriate pH value, concentration of quercetin in solution is decreased, and the samples with quercetin content of 10; 5; 2.5; 2.0; 1.0; 0.5; 0.25; 0.125; 0.0625; 0.03125 mg/ml, respectively, are obtained. pH values of samples are measured. Likewise solutions for the sample as described in Example 4.4 are prepared. Numbers of samples, concentration of quercetin in solution ($[C]_Q$) and the determined pH values of SD solutions in respective infusion solutions of sodium chloride are presented in Table 13.

**Table 13.**

| pH values of solutions samples SD Examples 4.3 and 4.4 in the range of quercetin concentrations 0.03 - 20 mg/ml in 0.9 % sodium chloride solutions with pH = 5.50; 6.20 and 7.00 | | | | | | | |
|---|---|---|---|---|---|---|---|
| Sample No. | $[C]_Q$ (mg/ml) | Example 4.3 Q:PVP 1:7 k[L-arginine] | | | Example 4.4 Q:PVP 1:9 k[$NaHCO_3$] | | |
| | | 0.9 % NaCl pH = 5.50 | 0.9 % NaCl pH = 6.20 | 0.9 % NaCl pH =7.00 | 0.9 % NaCl pH = 5.50 | 0.9 % NaCl pH = 6.20 | 0.9 % NaCl pH = 7.00 |
| 1 | 20.0 | 6.69 | 6.70 | 6.70 | 6.70 | 6.70 | 6.70 |
| 2 | 10.0 | 6.70 | 6.70 | 6.70 | 6.70 | 6.69 | 6.69 |
| 3 | 5.0 | 6.70 | 6.70 | 6.70 | 6.69 | 6.69 | 6.69 |
| 4 | 2.5 | 6.70 | 6.69 | 6.70 | 6.69 | 6.69 | 6.69 |
| 5 | 2.0 | 6.70 | 6.70 | 6.70 | 6.68 | 6.69 | 6.69 |

(continued)

| pH values of solutions samples SD Examples 4.3 and 4.4 in the range of quercetin concentrations 0.03 - 20 mg/ml in 0.9 % sodium chloride solutions with pH = 5.50; 6.20 and 7.00 | | | | | | | |
|---|---|---|---|---|---|---|---|
| Sample No. | $[C]_Q$ (mg/ml) | **Example 4.3 Q:PVP 1:7 k[L-arginine]** | | | **Example 4.4 Q:PVP 1:9 k[$NaHCO_3$]** | | |
| | | 0.9 % NaCl pH = 5.50 | 0.9 % NaCl pH = 6.20 | 0.9 % NaCl pH =7.00 | 0.9 % NaCl pH = 5.50 | 0.9 % NaCl pH = 6.20 | 0.9 % NaCl pH = 7.00 |
| 6 | 1.0 | 6.69 | 6.70 | 6.70 | 6.68 | 6.68 | 6.69 |
| 7 | 0.5 | 6.68 | 6.68 | 6.69 | 6.67 | 6.67 | 6.70 |
| 8 | 0.25 | 6.66 | 6.67 | 6.71 | 6.64 | 6.66 | 6.71 |
| 9 | 0.125 | 6.62 | 6.64 | 6.71 | 6.57 | 6.64 | 6.69 |
| 10 | 0.0625 | 6.57 | 6.59 | 6.72 | - | - | 6.72 |
| 11 | 0.03125 | 6.44 | 6.53 | 6.74 | - | - | - |
| «-» - no data | | | | | | | |

Graphic plotting of pH value as a function of concentration of quercetin in normal salines with pH = 5.50; 6.20; and 7.00 is presented in FIG. 12.

As can be seen from Table 13 and FIG. 12, buffer capacity of sterile solutions of solid dispersions according to the invention in infusion solutions with pH = 5.50; 6.20; 7.00 is capable of maintaining pH values of solutions in the wide range of concentrations of quercetin in solution, 20 to 0.5 mg/ml. There is no effect of acidity of infusion salines with pH values of 5.50; 6.20; 7.00 within the range of quercetin concentrations from 20 to 1 mg/ml on pH values of the resulting solutions of solid dispersions. At concentration of quercetin in solutions within the range of 1 to 0.5 mg/ml insignificant changes in pH values are observed. At concentration of quercetin in the prepared solution below 0.5 mg/ml, there are significant decrease in buffer strength of solution of solid dispersion and shift of pH value of the solution towards pH value of 0.9 % solution of sodium chloride, which was used for dissolution of solid dispersion.

Thus, preferable concentration of quercetin in the injection solution should be within the range of 0.5 to 1 mg/ml, which corresponds to the solution of medicinal dosage form in 50 - 100 ml of injectable infusion solution.

**7. Determination of osmolality of sterile lyophilized forms of solid dispersions obtained as described in Examples 4.1 - 4.10.**

Selected aliquots of the studied lyophilized sterile forms of solid dispersions obtained as described in Examples 4.1 - 4.10, with the content of 50 mg quercetin therein, specifically, 400 mg of solid dispersion of Q:PVP 1:7, 500 mg of solid dispersion of Q:PVP 1:9 and 650 mg of solid dispersion of Q:PVP 1:12, are dissolved in 100 ml of 0.9 % solution of sodium chloride and osmolality values are measured in mOsm/kg. Determination of osmolality of solutions is performed using Gonotec - Osmomat 030 cryoscopic osmometer (Gonotec GmbH, Germany). The determined osmolality values of solutions are presented in Table 14.

Table 14.

| Osmolality of solutions of lyophilized sterile forms of solid dispersions | | |
|---|---|---|
| Example No. | Q:PVP·k[BA] | Osmolality, mOsm/kg |
| 4.1 | 1:7 $NaHCO_3$ | 296 |
| 4.2 | 1:7 $Na_2CO_3$ | 298 |
| 4.3 | 1:7 L-arginine | 302 |
| 4.4 | 1:9 $NaHCO_3$ | 300 |
| 4.5 | 1:9 $Na_2CO_3$ | 301 |
| 4.6 | 1:9 L-arginine | 297 |
| 4.7 | 1:12 $NaHCO_3$ | 295 |
| 4.8 | 1:12 $Na_2CO_3$ | 298 |

(continued)

| Osmolality of solutions of lyophilized sterile forms of solid dispersions | | |
|---|---|---|
| Example No. | Q:PVP·k[BA] | Osmolality, mOsm/kg |
| 4.9 | 1:12 L-arginine | 302 |
| 4.10 | 1:12 NaOH | 298 |

The results of studies suggest that osmolality values of solutions of lyophilized sterile forms of solid dispersions having different compositions and ratios of polyvinyl pyrrolidone, quercetin and basicity of alkaline agents, that were obtained using various methods of addition of components, are within the range of normal osmolality of blood serum (L. De Lemos M, Monfared Sh, Denyssevych T., et. al. Evaluation of osmolality and pH of various concentrations of methotrexate, cytarabine, and thiotepa prepared in normal saline, sterile water for injection, and lactated Ringer's solution for intrathecal administration. J. Oncol. Pharm. Practice. - 2009. - V. 15. - P. 45 - 52.), which allows to use them as preparations for parenteral administration.

**8. Preparation of substances of solid dispersion of Q:PVP·k[BA] for manufacture of tableted forms of medicinal products.**

**EXAMPLE 8.1.**

**Preparation of substance of solid dispersion of Q:PVP·1:7 k[L-arginine] as described in Method 3 - codissolution of all components**

800 ml of 75 % ethanol is poured in 4 l round-bottomed flask, then 461.33 g of PVP (445.32 g equivalent to 100 % substance), 73.26 g of quercetin dihydrate (63.62 g of 100 % substance) and 12.11 g L-arginine base (11.916 g equivalent to 100 % substance) are charged and mixed with shaking. A flask with the mixture of components is attached to a rotary evaporator, a conduit for inert gas (argon) is connected thereto and inert atmosphere is created within the working volume of the apparatus by successive, three-times creation of 10 - 12 mbar vacuum within the working volume of the evaporator using a pump and filling that volume with inert gas. The content of the flask is dissolved under inert atmosphere with rotation (80 rpm) and heating in water bath at a temperature of 50 °C and pressure of 850 mbar, with passing a weak flow of inert gas (~10 ml/min) through the working volume of the apparatus. After 40 minutes, precipitate in the flask is completely dissolved, after dissolution the solution is stirred for more 30 minutes, the inert gas flow is stopped, vacuum is created in the rotary evaporator and solvent is removed to dryness under the following conditions: water bath temperature 50 - 60 °C, residual pressure 10 - 12 mbar, flask rotation speed 80 rpm. After certain time, rotation speed is lowered to 50 rpm. In the course of coevaporation vacuum is deepened in such a rate that continuous boiling of the solution is maintained, with avoidance of intensive foaming and spilling foamed precipitate of solid dispersion outside a flask. Evaporation is continued until solvent is removed completely with formation of foamed glasslike yellow mass. Then the precipitate of solid dispersion is discharged on trays of 316L grade stainless steel, placed in a vacuum oven and dried under inert atmosphere to constant weight at 50 °C and residual pressure of 40 mbar.

512 g of solid dispersion of Q:PVP 1:7 k[L-arginine] as amorphous glasslike yellow precipitate is obtained. Weight loss on drying is 2.74 %.

The precipitate is packaged in a double polyethylene bag. The external polyethylene bag is black, the internal polyethylene bag may be of any color. The bags are subjected to heat sealing. They are stored in a dark dry place at a temperature not exceeding 25 °C.

**EXAMPLE 8.2.**

**Preparation of substance of solid dispersion of Q:PVP·1:7 k[$NaHCO_3$] as described in Method 3 - codissolution of mixture of all components**

800 ml of 75 % ethanol is poured in 4 l round-bottomed flask, then 475.89 g of PVP (459.38 g equivalent to 100 % substance), 75.59 g of quercetin dihydrate (65.62 g equivalent to 100 % substance) are charged, 5.41 g of $NaHCO_3$ (5.4 g equivalent to 100 % substance) is added and stirred with shaking. All further operations for obtaining solid dispersion of Q:PVP·1:7 k[$NaHCO_3$] are performed similarly to those presented above in Example 8.1.

523 g of solid dispersion of Q:PVP 1:7 k[$NaHCO_3$] as amorphous glasslike yellow precipitate is obtained. Weight loss on drying is 3.15 %.

The precipitate is packaged in a double polyethylene bag. The external polyethylene bag is black, the internal polyethylene bag may be of any color. The bags are subjected to heat sealing. They are stored in a dark dry place at a temperature not exceeding 25 °C.

**EXAMPLE 8.3.**

**Preparation of substance of solid dispersion of Q:PVP 1:9 k[L-arginine] using Büchi B-191 Mini Spray Dryer, which corresponds to the modified Method** 3 - **codissolution of all components**

463.33 g PVP (450.0 g equivalent to 100 % substance), 57.8 g of quercetin dihydrate (50.0 g equivalent to 100 % substance) and 9.319 g of L-arginine (9.165 g equivalent to 100 % substance) are charged in 4 l round-bottomed flask and stirred with shaking. Then 600 ml of 72 % ethanol is added in the flask with mixture of components, the flask is attached to a rotary evaporator and inert atmosphere is created within the working volume of the apparatus by suction of air out of the working volume of the evaporator using a vacuum pump to the residual pressure of 10 - 12 mbar, and filling it with inert gas (argon). The content of the flask is dissolved under inert atmosphere with rotation (80 rpm) and heating the flask in water bath at a temperature of 50 °C and pressure of 850 mbar, with passing a weak flow (~10 ml/min) of inert gas (argon) through the working volume of the apparatus. After 50 minutes, precipitate in the flask is dissolved completely, the solution after dissolution of precipitate is stirred for more 30 minutes, then the inert gas flow is stopped, vacuum is created in the rotary evaporator, and 3/4 of solvent volume (~ 450 ml) is removed under the following conditions: water bath temperature 60 ± 10 °C, residual pressure 10 - 12 mbar, flask rotation speed 80 rpm. After certain time, rotation speed is lowered to 50 rpm. In the course of coevaporation vacuum is deepened in such a rate that continuous boiling of the solution is maintained, with avoidance of intensive foaming. Then ml of purified water is added to viscous residue in the flask 600, dissolved with stirring and evaporated again ~ 450 ml of solvent under the above described conditions. Flask is detached from the rotary evaporator and weighed. The solution weight equals to 0.844 kg. Weight concentration of the dry residue solution of solid dispersion in the flask is adjusted to 25 % with purified water. The total weight of SDQ PVP solution is 2.036 kg. The solution is placed in a plastic vessel and passed for drying in a spray drier.

Drying 2.08 l of aqueous solution of the studied composition of solid dispersion of Q:PVP·1:9 k[L-arginine] with dry matter concentration of 25 % is carried out in Büchi B-191 Mini Spray Dryer (BÜCHI Labortechnik AG (Switzerland)) at the following parameters: temperature of the working gas (air) at the spraying chamber inlet is 190 °C, temperature at the spraying chamber outlet is 108 °C, flow rate of the working gas is 35 $m^3$/h, feeding speed of 25 % solution for drying is 0.3 kg/h.

0.484 kg (95 %) of fine dry yellow powder of solid dispersion of Q:PVP 1:9 k[L-arginine] was obtained. Particle size, bulk density $\rho_b$ and tapped density $\rho_t$ of SDQ PVP powder was determined according to the guidelines of the State Pharmacopoeia of Ukraine (SPU), 2$^{nd}$ edition, clauses 2.9.31 and 2.9.34. Powder particle size in 90 % is 8 - 12 μm, bulk density $\rho_b$ = 0.24 g/cm$^3$, tapped density $\rho_t$ = 0.262 g/cm$^3$. Weight loss on drying is 3.08 %.

The results of HPLC analysis of the obtained substance of solid dispersion showed that it was no different by impurities content from the products obtained by the conventional method of solvent coevaporation on a rotary evaporator, being at the level of 0.3 to 0.4 %.

To compare the internal structure, quantitative and qualitative composition of solid dispersion of Q:PVP 1:9 k[L-arginine] obtained using the spray drying method, Example 8.3, to solid dispersion obtained using coevaporation method on a rotary evaporator, Example 3.7i, which was similar in composition to the former, Fourier-transform infrared spectrophotometry (FTIR) was used by means of Agilent Cary 630 FTIR spectrophotometer from Agilent Technologies, Inc.

FTIR spectra of solid dispersion of Q:PVP 1:9 k[L-arginine] obtained using the spraying method, Example 8.3, and those of solid dispersion obtained by coevaporation method, Example 3.7i, as well as their physical mixture and polyvinyl pyrrolidone PVP K17, were received using the disk method with KBr and presented in FIG. 15.

Comparison of IR spectra of solid dispersion of Q:PVP 1:9 k[L-arginine] obtained using the coevaporation and spraying methods with a use of Agilent Cary 630 spectrophotometer software demonstrated their substantial identity - similarity of spectra is 93 %, as shown in FIG. 15.

In terms of solubility of solid dispersion as described in Example 8.3, physical and chemical stability of its solutions and pH value it is no different from solid dispersions previously obtained by codissolution method with subsequent coevaporation. The quality of SD substance powder obtained by spraying method is quite sufficient for manufacture of medicinal products in tableted form. pH of aqueous solution with quercetin concentration of [C] = 2.5 mg/ml is 6.72.

**9. Manufacture of tableted dosage forms of quercetin with a use of substances of solid dispersions Q:PVP k [BA]**

Substances of solid dispersions Q:PVP 1:7 k[L-arginine] and Q:PVP 1:7 k[$NaHCO_3$] obtained in Examples 8.1 and 8.2 were used for manufacture of film coated tableted dosage forms of SD containing 50 mg of quercetin in 1 tablet.

**Description of the process procedure for tablet manufacture**

The following components were used to manufacture tablets: 1 - substances of solid dispersions selected from Q:PVP 1:7k[L-arginine] and Q:PVP 1:7k[$NaHCO_3$]; 2 - sodium

croscarmellose; 3 - microcrystalline cellulose МКЦ-102, for example from VIVAPUR®102 or HEWETEN®102; 4 - corn starch; 5 - talc; 6 - film coating Aquarius Preferred HSP BPP314069 Yellow from Ashland Specialty Ingredients G.P., USA.

Components 1, 2, 3 and 4 presifted through the 0.5 mm sieve were mixed for 30 min in LM-40 laboratory mixer (L. B. Bohle Maschinen und Verfahren GmbH, Germany) at a rotation speed of 20 rpm.

Component 5 was added to the resulting mixture, and mixing the mass components was continued for preparation of tablets for 5 min.

To obtain tablets, direct tableting of tablet mass on XP 1 tableting press (Korsch AG, Germany) was carried out at the main compacting force of 22 kN.

The prepared 18 % aqueous suspension of component 5, film coating Aquarius Preferred HSP BPP314069 Yellow, was applied to tablets in HT/M 003 coating machine (IMA S.p.A., Italy). Compositions of components in the obtained tablets are presented in Table 15.

**Table 15.**

| Composition of components of tableted forms of solid dispersions | | | | | |
|---|---|---|---|---|---|
| No. | **Component** | **Series 041119** | | **Series 051119** | |
| | | Weight per tablet, mg | Content in one tablet, % | Weight per tablet, mg | Content in one tablet, % |
| 1 | Q:PVP 1:7 k[L-arginine] | 409.08 | 49.59 | - | - |
| | Q:PVP 1:7 k[$NaHCO_3$] | - | - | 404.11 | 48.98 |
| 2 | Croscarmellose sodium | 32 | 3.88 | 32 | 3.88 |
| 3 | МКЦ-102 | 246.92 | 29.93 | 251.89 | 30.53 |
| 4 | Corn starch | 100 | 12.12 | 100 | 12.12 |
| 5 | Talc | 12 | 1.45 | 12 | 1.45 |
| 6 | Film coating Aquarius Preferred HSP BPP314069 Yellow | 25 | 3.03 | 25 | 3.03 |
| **Total:** | | **825** | **100** | **825** | **100** |
| «-» - component is absent | | | | | |

**10. Studying dissolution rate of tableted dosage forms of quercetin of Series 041119 and 051119.**

Studying dissolution rate of tableted dosage forms of quercetin of Series 041119 and 051119 obtained on the basis of SDQ PVP substances Q:PVP 1:7k[L-arginine] and Q:PVP 1:7k[$NaHCO_3$] was carried out according to the requirements of the State Pharmacopoeia of Ukraine, clause 2.9.3. of "Dissolution" for solid dosage forms with a use of ERWEKA DT 700 Type I dissolution device (with a basket) from ERWEKA GmbH. 825 mg tablet of the preparation, which contains 50 mg of quercetin was placed in a cylindrical basket mounted on a shaft of the device. A vessel for dissolution is filled with 500 ml of degassed

phosphate-buffered saline (pH 6.8) as dissolution medium. Temperature of the medium is 37 ± 0.5 °C. The basket with a tablet sample is stirred at a rotation speed of 100 rpm. After set intervals of time (10, 15, 20, 30, 45, 60 and 90 min), 10 ml aliquote samples of the studied solution are collected through the mdi SYRINGE Filter SY25TG (PTFE+Prefilter) from ADVANCED MICRODEVICES PVT. LTD for preparation of the solution to be tested. The collected aliquote of the studied solution is substituted with 10.0 ml of appropriate dissolution medium.

The content of quercetin in solution is determined by spectrophotometric method (European Pharmacopoeia / State Pharmacopoeia of Ukraine - EP/SPU, 2.2.25). Absorbancy of solutions is measured by UV/VIS Spectrometer "LAMBDA™ 25" from PerkinElmer, Inc. at a wavelength of 374 nm in a quartz measuring cell with layer thickness of 10 mm, using the appropriate dissolution medium as compensation solution.

**Preparation of phosphate-buffered saline, pH 6.8:**

250.0 ml of 0.2 M potassium dihydrogen phosphate P solution is mixed in 1000 ml measuring flask with 112.0 ml of 0.1 M sodium hydroxide solution, the solution volume is made up to the mark with water P and stirred (European Pharmacopoeia, 5.17.1).

**Preparation of comparison solutions:**

To study dissolution kinetics of Series 041119 preparation, a working standard sample of Series 040517 quercetin (quercetin content 95.05 %) was used; for Series 051119 preparation, a working standard sample of Series 060919 quercetin (quercetin content 93.02 %) produced by PJSC Scientific Industrial Center (SIC) "Borshchahivskiy Chemical-Pharmaceutical Plant" was used.

Working standard samples of quercetin are placed in 100 ml measuring flasks, 50 ml of 70 % alcohol is added in each flask, the solution volume is made up the mark with 70 % alcohol and stirred. 5 ml of the resulting solution is placed in 200 ml measuring flask, the solution volume is made up the mark with dissolution medium and stirred.

Absorbancy of comparison solutions is measured with UV/VIS Spectrometer "LAMBDA™ 25" at a wavelength of 374 nm in a quartz measuring cell with layer thickness of 10 mm, using the appropriate dissolution medium as compensation solution.

| Standard sample of quercetin S. 040517 (95.05 %) | | | Standard sample of quercetin S. 60919 (93.02 %) | | |
|---|---|---|---|---|---|
| $m_0$ = 39.95 mg | P 95.05 % | λ 374 | $m_0$ = 40.09 mg | P 93.02 % | λ 374 |
| | | $A_0$ = 0.564 | | | $A_0$ = 0.548 |

**Preparation of the test solution:**

10 ml samples of test solutions are filtered immediately after sampling using Microlab Scientific PTFE Syringe Filter from Microlab Scientific Co., Ltd, China, with pore diameter of 0.45 μm and diameter of 25 mm, or similar teflon membrane filter (discarding the first 3 ml of filtrate). 2.0 ml of the obtained filtrate is placed in 20 ml measuring flask, the solution volume is made up to the mark with dissolution medium and stirred.

**Analysis method:**

Determination is carried out using the spectrophotometric method (European Pharmacopoeia / State Pharmacopoeia of Ukraine - EP/SPU, 2.2.25). Absorbancy of solutions is measured with UV/VIS Spectrometer Lambda 25 at a wavelength of 374 nm in a quartz measuring cell with layer thickness of 10 mm, using the appropriate dissolution medium as compensation solution.

The amount of quercetin (X) that transfers to solution from the tablet, as percentage of the nominal content, is calculated by the formula:

$$X_{10} = \frac{A_{10} * m_0 * 500 * 20 * 2{,}5 * 100 * P}{A_0 * 2 * 100 * 100 * 100 * 50} = \frac{A_{10} * m_0 * P * 0{,}025}{A_0},$$

$$X_{15} = \frac{\left(A_{15} + \frac{10}{500} * A_{10}\right) * m_0 * P * 0{,}025}{A_0},$$

$$X_n = \frac{\left(A_n + \frac{10}{500} * (A_{10} + ... + A_{n-1})\right) * m_0 * P * 0{,}025}{A_0},$$

where:

$A_n$ - absorbancy of the 4n-th test solution;
$A_0$ - absorbancy of comparison solution;
$m_0$ - aliquot weight of the standard sample of quercetin, in milligrams;
*P* - content of quercetin in the standard sample of quercetin, in percent;
*50* - nominal content of quercetin in the preparation, in milligrams.

The results of six consecutive absorbancy measurements of the test solution for S. 041119, that were received with a use of the substance SDQ:PVP 1:7 k[L-arginine], are presented in Table 16.

**Table 16.**

| Dynamics of quercetin release from Series 041119 preparation based on the time of dissolution | | | | | | | |
|---|---|---|---|---|---|---|---|
| Time, min | Test, % release | | | | | | Averaged concentration |
| | No. 1 | No. 2 | No. 3 | No. 4 | No. 5 | No. 6 | |
| 0 | 0 | 0 | 0 | 0 | 0 | 0 | 0 |
| 10 | 25.75 | 28.45 | 27.27 | 26.76 | 26.59 | 23.90 | 26.45 |
| 15 | 35.86 | 41.13 | 40.10 | 40.76 | 39.41 | 35.66 | 38.82 |
| 20 | 46.16 | 53.90 | 52.51 | 53.01 | 51.30 | 48.48 | 50.89 |
| 30 | 67.60 | 77.16 | 75.41 | 76.60 | 74.85 | 70.80 | 73.74 |
| 45 | 92.47 | 95.83 | 94.38 | 94.41 | 95.49 | 95.23 | 94.64 |
| 60 | 95.26 | 94.97 | 95.85 | 96.90 | 96.99 | 96.73 | 96.12 |
| 90 | 96.22 | 96.42 | 96.98 | 96.87 | 96.63 | 97.05 | 96.69 |

The results of six consecutive absorbancy measurements for tableted dosage form of Series 051119, that were received with a use of the substance of solid dispersion of quercetin Q:PVP 1:7 k[$NaHCO_3$], are presented in Tables 17.

**Table 17.**

| Dynamics of quercetin release from Series 051119 preparation based on the time of dissolution | | | | | | | |
|---|---|---|---|---|---|---|---|
| Time, min | Test, % release | | | | | | Averaged concentration |
| | No. 1 | No. 2 | No. 3 | No. 4 | No. 5 | No. 6 | |
| 0 | 0 | 0 | 0 | 0 | 0 | 0 | 0 |
| 10 | 21.44 | 21.61 | 23.48 | 24.84 | 23.65 | 21.44 | 22.74 |
| 15 | 35.99 | 34.8 | 35.01 | 38.78 | 37.9 | 33.94 | 36.07 |
| 20 | 48.61 | 46.88 | 48.12 | 51.11 | 49.71 | 44.99 | 48.24 |
| 30 | 70.48 | 70.09 | 69.98 | 72.69 | 72.79 | 69.69 | 70.95 |
| 45 | 93.62 | 91.01 | 93.62 | 95.71 | 93.77 | 94.52 | 93.71 |
| 60 | 95.60 | 94.80 | 95.26 | 96.36 | 94.89 | 96.00 | 95.49 |
| 90 | 96.04 | 95.07 | 94.85 | 96.30 | 94.98 | 95.78 | 95.50 |

The results of studies of dissolution kinetics of two series of tableted forms of MPs, S. 041119 (substance Q:PVP

1:7 k[L-arginine]) and S. 051119 (substance Q:PVP 1:7 k[$NaHCO_3$]) containing 50 mg of quercetin as the active substance, are presented in the graphs of dissolution profiles (FIG. 13 and FIG. 14) plotted based on the results of mean values of six sequential measurements.

As can be seen from the graphs of dissolution kinetics, as early as after 50 minutes quercetin concentration reaches 95 - 96 % of complete release relative of its content in the tablet. Lack of 100 % release can be attributed to partial insignificant adsorption of quercetin on auxiliary components of the tablet content.

The developed method of obtaining water-soluble solid dispersions of quercetin has prospects to be used in industrial production of medicinal products based on SDQ PVP according to the invention for use in the treatment of cardiovascular diseases, in particular, acute conditions of cardiovascular and cerebrovascular system diseases.

The developed method of obtaining water-soluble solid dispersions of quercetin includes the use of various weight quantities of quercetin and PVP in the composition of SDQ PVP at their ratios from 1:7 to 1:12, which allows to produce preparations with wide ranges of doses of the active substance in the finished dosage form.

In the method of obtaining water-soluble solid dispersions of quercetin, various alkaline agents are used, that act as stabilizers for the obtained SDQ PVP in solid and dissolved states, as well as means of adjustment of pH values of solutions of solid dispersions, which is also a key factor of quality of finished medicinal products.

Solid dispersions according to the invention will find application in production of not only sterile injectable drugs but also solid dosage forms as means of oral administration.

## Claims

1. Water-soluble solid dispersion of quercetin with polyvinyl pyrrolidone (PVP) and alkaline agent, **characterized in that** the alkaline agent is present in an amount within the range of $(6.3 \pm 0.5)*10^{-2}$ gE per 1 gE of quercetin, wherein the alkaline agent does not form insoluble salt with quercetin and/or other components of solid dispersion.

2. Method of obtaining water-soluble solid dispersion of quercetin with polyvinyl pyrrolidone (PVP) and alkaline agent, comprising the following stages:

    i) preparation of a mixture of solid dispersion components:

        a) the predetermined amount of PVP is added to alcohol-containing solvent, then the calculated amount of alkaline agent is added, whereafter predetermined amount of quercetin is added, or
        b) the predetermined amount of PVP is added to alcohol-containing solvent, then the predetermined amount of quercetin is added, whereafter the calculated amount of alkaline agent is added, or
        c) the predetermined amount of PVP, predetermined amount of quercetin and the calculated amount of alkaline agent are pooled, whereafter the alcohol-containing solvent is added,

    ii) stirring the resulting mixture of components to complete dissolution of components, and
    iii) obtaining water-soluble solid dispersion,

    wherein
    the alkaline agent is added in the amount within the range of $(6.3 \pm 0.5)*10^{-2}$ gE of alkaline agent per 1 gE of quercetin, wherein the alkaline agent does not form insoluble salt with quercetin and/or other components of solid dispersion.

3. The method according to claim 2, wherein the weight of the alkaline agent is calculated according to Formula (1):

$$m(BA) = \frac{k * m(Q)}{\mathrm{E}_m(Q)} * \mathrm{E}_m(BA) (1),$$

    wherein:

    m(Q) - weight of quercetin in the solid dispersion in grams;
    $E_m(Q)$ and $E_m(BA)$ - equivalent weights of Q (quercetin) and BA (alkaline agent), respectively, in gE/mol;
    m(BA) - weight of alkaline agent in grams;
    k - ratio factor of the number of gE of the selected alkaline agent ($* 10^{-2}$) to 1 gE quercetin.

4. Water-soluble solid dispersion according to claim 1 or the method according to claim 2 or 3, wherein the ratio of the

amounts of quercetin to PVP is from 1:7 up to and including 1: 12, preferably the ratio of the amounts of quercetin to PVP is selected from the group including 1:7, 1:9 and 1: 12.

**5.** Water-soluble solid dispersion according to claim 1 or 4 or the method according to any one of claims 2 to 4, wherein PVP has average molecular weight of polymeric macromolecules between 7,000 and 11,000, preferably PVP is PVP K 17.

**6.** Water-soluble solid dispersion according to claim 1, 4 or 5 or the method according to any one of claims 2 to 5, wherein quercetin is selected from quercetin dihydrate, anhydrous quercetin and mixtures thereof, preferably quercetin is anhydrous quercetin crystallized from ethanol, preferably quercetin is anhydrous quercetin double-crystallized from 96 % ethanol, with a content of the active substance of at least 99.7 %, more preferably quercetin is quercetin dihydrate.

**7.** Water-soluble solid dispersion of quercetin produced by the method according to any one of claims 2 to 6.

**8.** Water-soluble solid dispersion according to any one of claims 1 or 4 to 7, wherein quercetin is in amorphous form.

**9.** Water-soluble solid dispersion of quercetin according to any one of claims 1 or 4 to 8, for use as substance for obtaining injectable medicinal products or oral medicinal product for treatment of acute conditions of cardiovascular and cerebrovascular system diseases in subjects in need thereof, preferably with a dose of the active substance in the range of 14.28 % to 8.33 %.

**10.** Lyophilized form of water-soluble solid dispersion of quercetin, **characterized in that** it comprises a solid dispersion of quercetin according to any one of claims 1 or 4 to 8 or a water-soluble solid dispersion of quercetin for use according to claim 9.

**11.** Tableted form water-soluble solid dispersion of quercetin, **characterized in that** it comprises a solid dispersion of quercetin according to any one of claims 1 or 4 to 8.

**12.** Lyophilized form according to claim 10 or Tableted form according to claim 11, comprising 50 mg quercetin.

**13.** Infusion solution comprising solid dispersion of quercetin according to any one of claims 1 or 4 to 8 or the solid dispersion of quercetin according to claim 9 and physiologically acceptable solvent.

**14.** Injectable form comprising a sterile solution of the solid dispersion of quercetin according to any one of claims 1 or 4 to 8 or the solid dispersion of quercetin according to claim 9.

**15.** Infusion solution according to claim 13 or injectable form according to claim 14, wherein pH value is in the range of 5.5 to 7.0 and the concentration of quercetin is in the range of 1 to 0.5 mg/ml.

**16.** Use of alkaline agent for obtaining a water-soluble solid dispersion of quercetin with polyvinyl pyrrolidone (PVP), wherein the alkaline agent is used in an amount within the range of $(6.3 \pm 0.5)*10^{-2}$ gE per 1 gE of quercetin, wherein the alkaline agent does not form insoluble salt with quercetin and/or other components of solid dispersion.

**17.** Water-soluble solid dispersion according to claim 1 or 4 to 6, the method according to any one of claims 2 to 6 or the use according to claim 16, wherein the alkaline agent is selected from the group consisting of sodium hydroxide, sodium bicarbonate, sodium carbonate, L-arginine, and mixtures thereof.

**18.** A container, comprising sterile water-soluble solid dispersion of quercetin according to any one of claims 1, 4 to 8 or 17, the water-soluble solid dispersion of quercetin according to claim 9, or the infusion solution or the injectable form according to any one of claims 13 to15.

**19.** A kit, comprising one or more containers according to claim 18 and, optionally, solvent and/or instructions for use thereof.

## Patentansprüche

1. Wasserlösliche feste Dispersion von Quercetin mit Polyvinylpyrrolidon (PVP) und alkalischem Mittel, **dadurch gekennzeichnet, dass** das alkalische Mittel liegt in einer Menge im Bereich von (6,3 ± 0,5)*$10^{-2}$ gE pro 1 gE Quercetin, wobei das alkalische Mittel kein unlösliches Salz mit Quercetin und/oder anderen Bestandteilen der festen Dispersion bildet.

2. Verfahren zur Gewinnung einer wasserlöslichen festen Dispersion von Quercetin mit Polyvinylpyrrolidon (PVP) und alkalischem Mittel, bestehend aus folgenden Stufen:

   i) Herstellung eines Gemischs aus festen Dispersionskomponenten:

      a) die vorbestimmte Menge PVP dem alkoholhaltigen Lösungsmittel zugesetzt wird, dann wird die berechnete Menge des alkalischen Mittels zugegeben, wonach die vorbestimmte Menge Quercetin zugegeben wird, oder
      b) die vorbestimmte Menge PVP dem alkoholhaltigen Lösungsmittel zugesetzt wird, dann wird die vorbestimmte Menge Quercetin zugegeben, wonach die berechnete Menge des alkalischen Mittels zugegeben wird, oder
      c) die vorgegebene Menge an PVP, die vorgegebene Menge Quercetin und die berechnete Menge an alkalischem Mittel werden gepoolt, wonach das alkoholhaltige Lösungsmittel zugegeben wird,

   ii) Rühren des resultierenden Mischungs von Komponenten bis zur vollständigen Auflösung der Komponenten und
   iii) Gewinnung einer wasserlöslichen Feststoffdispersion,

   worin
   das alkalische Mittel wird in einer Menge innerhalb des Bereichs von (6,3 ± 0,5)*$10^{-2}$ gE alkalischem Mittel pro 1 gE Quercetin zugegeben, wobei das alkalische Mittel kein unlösliches Salz mit Quercetin und/oder anderen Komponenten der festen Dispersion bildet.

3. Verfahren nach Anspruch 2, wobei das Gewicht des alkalischen Mittels berechnet wird nach Formel (1):

$$m(BA) = \frac{k * m(Q)}{E_m(Q)} * E_m(BA) (1),$$

   worin:

   m(Q) - Gewicht von Quercetin in der festen Dispersion in Gramm;
   Em(Q) und Em(BA) - äquivalente Gewichte von Q (Quercetin) bzw. BA (alkalisches Mittel) in gE/mol;
   m(BA) - Gewicht des alkalischen Mittels in Gramm;
   k - Verhältnisfaktor der Anzahl gE des ausgewählten alkalischen Mittels (* $10^{-2}$) zu 1 gE Quercetin.

4. Wasserlösliche feste Dispersion nach Anspruch 1 oder das Verfahren nach Anspruch 2 oder 3, wobei das Verhältnis der Mengen an Quercetin zu PVP von 1:7 bis einschließlich 1:12 beträgt, vorzugsweise das Verhältnis der Mengen an Quercetin zu PVP aus der Gruppe von 1:7, 1:9 und 1:12 ausgewählt wird.

5. Wasserlösliche feste Dispersion nach Anspruch 1 oder 4 oder Verfahren nach einem der Ansprüche 2 bis 4, wobei PVP ein durchschnittliches Molekulargewicht von polymeren Makromolekülen zwischen 7.000 und 11.000 aufweist, vorzugsweise PVP ist PVP K 17.

6. Wasserlösliche feste Dispersion nach Anspruch 1, 4 oder 5 oder Verfahren nach einem der Ansprüche 2 bis 5, wobei Quercetin ausgewählt ist aus Quercetin-Dihydrat, wasserfreiem Quercetin und Mischungen daraus, vorzugsweise Quercetin ist wasserfreies Quercetin, das aus Ethanol kristallisiert ist, vorzugsweise Quercetin ist wasserfreies Quercetin, das aus 96 % Ethanol doppelt kristallisiert ist, mit einem Gehalt des Wirkstoffs von mindestens 99,7 %, Bevorzugter ist Quercetin-Dihydrat.

7. Wasserlösliche feste Dispersion von Quercetin, hergestellt nach dem Verfahren nach einem der Ansprüche 2 bis 6.

**8.** Wasserlösliche feste Dispersion nach einem der Ansprüche 1 oder 4 bis 7, wobei Quercetin in amorpher Form vorliegt.

**9.** Wasserlösliche feste Dispersion von Quercetin nach einem der Ansprüche 1 oder 4 bis 8 zur Verwendung als Substanz zur Herstellung von injizierbaren Arzneimitteln oder als orales Arzneimittel zur Behandlung akuter Erkrankungen des Herz-Kreislauf- und zerebrovaskulären Systems bei Patienten, die dies benötigen, vorzugsweise mit einer Dosis des Wirkstoffs im Bereich von 14,28 % bis 8,33 %.

**10.** Lyophilisierte Form einer wasserlöslichen festen Dispersion von Quercetin, **dadurch gekennzeichnet, dass** sie eine feste Dispersion von Quercetin nach einem der Ansprüche 1 oder 4 bis 8 oder eine wasserlösliche feste Dispersion von Quercetin zur Verwendung nach Anspruch 9 umfasst.

**11.** Tablettierte Form einer wasserlöslichen festen Dispersion von Quercetin, **dadurch gekennzeichnet, dass** sie eine feste Dispersion von Quercetin nach einem der Ansprüche 1 oder 4 bis 8 umfasst.

**12.** Lyophilisierte Form nach Anspruch 10 oder Tablettierte Form nach Anspruch 11, bestehend aus 50 mg Quercetin.

**13.** Infusionslösung, umfassend aus einer festen Dispersion von Quercetin nach einem der Ansprüche 1 oder 4 bis 8 oder einer festen Dispersion von Quercetin nach Anspruch 9 und einem physiologisch akzeptablen Lösungsmittel.

**14.** Injizierbare Form, umfassend eine sterile Lösung aus der festen Dispersion von Quercetin nach einem der Ansprüche 1 oder 4 bis 8 oder die feste Dispersion von Quercetin nach Anspruch 9.

**15.** Infusionslösung nach Anspruch 13 oder injizierbare Form nach Anspruch 14, wobei der pH-Wert im Bereich von 5,5 bis 7,0 und die Konzentration von Quercetin im Bereich von 1 bis 0,5 mg/ml liegt.

**16.** Verwendung eines alkalischen Mittels zur Gewinnung einer wasserlöslichen festen Dispersion von Quercetin mit Polyvinylpyrrolidon (PVP), wobei das alkalische Mittel in einer Menge im Bereich von $(6{,}3 \pm 0{,}5)*10^{-2}$ gE pro 1 gE Quercetin verwendet wird, wobei das alkalische Mittel kein unlösliches Salz mit Quercetin und/oder anderen Bestandteilen der festen Dispersion bildet.

**17.** Wasserlösliche feste Dispersion nach Anspruch 1 oder 4 bis 6, das Verfahren nach einem der Ansprüche 2 bis 6 oder die Verwendung nach Anspruch 16, wobei das alkalische Mittel aus der Gruppe ausgewählt wird, die aus Natriumhydroxid, Natriumbicarbonat, Natriumcarbonat, L-Arginin und Mischungen davon besteht.

**18.** Behälter, umfassend eine sterile wasserlösliche feste Dispersion von Quercetin nach einem der Ansprüche 1, 4 bis 8 oder 17, die wasserlösliche feste Dispersion von Quercetin nach Anspruch 9 oder die Infusionslösung oder die injizierbare Form nach einem der Ansprüche 13 bis 15.

**19.** Ein Bausatz, einschließend ein oder mehrere Behältnisse nach Anspruch 18 und gegebenenfalls Lösungsmittel und/oder eine Gebrauchsanweisung dafür.

## Revendications

**1.** Dispersion solide hydrosoluble de quercétine avec de la polyvinylpyrrolidone (PVP) et un agent alcalin, **caractérisé en ce que** l'agent alcalin est présent en quantité comprise dans la gamme de $(6{,}3 \pm 0{,}5)*10^{-2}$ gE pour 1 gE de quercétine, où l'agent alcalin ne forme pas de sel insoluble avec la quercétine et/ou d'autres composants de la dispersion solide.

**2.** Procédé d'obtention d'une dispersion solide hydrosoluble de quercétine avec de la polyvinylpyrrolidone (PVP) et un agent alcalin, comprenant les étapes suivantes :

i) préparation d'un mélange de composants de dispersion solide :

a) la quantité prédéterminée de PVP est ajoutée à un solvant contenant de l'alcool, puis la quantité calculée d'agent alcalin est ajoutée, après quoi une quantité prédéterminée de quercétine est ajoutée, ou
b) la quantité prédéterminée de PVP est ajoutée à un solvant contenant de l'alcool, puis la quantité

prédéterminée de quercétine est ajoutée, après quoi la quantité calculée d'agent alcalin est ajoutée, ou
c) la quantité prédéterminée de PVP, la quantité prédéterminée de quercétine et la quantité calculée d'agent alcalin sont regroupées, après quoi le solvant contenant de l'alcool est ajouté,

ii) agitation du mélange de composants obtenu jusqu'à la dissolution complète des composants, et
iii) obtention d'une dispersion solide hydrosoluble,

où
l'agent alcalin est ajouté en quantité dans la gamme de $(6,3 \pm 0,5)*10^{-2}$ gE d'agent alcalin pour 1 gE de quercétine, où l'agent alcalin ne forme pas de sel insoluble avec la quercétine et/ou d'autres composants de dispersion solide.

**3.** Procédé selon la revendication 2, où le poids de l'agent alcalin est calculé selon la formule (1) :

$$m(BA) = \frac{k*m(Q)}{\mathrm{E}_m(Q)} * \mathrm{E}_m(BA)(1),$$

où:

m(Q) - poids de la quercétine dans la dispersion solide en grammes ;
Em(Q) et Em(BA) - poids équivalents de Q (quercétine) et BA (agent alcalin), respectivement, en gE/mol ;
m(BA) - poids de l'agent alcalin en grammes ;
k - facteur de rapport entre le nombre de gE de l'agent alcalin choisi $(*10^{-2})$ et 1 gE de quercétine.

**4.** Dispersion solide hydrosoluble selon la revendication 1 ou le procédé selon la revendication 2 ou 3, où le rapport des quantités de quercétine à la PVP est de 1:7 à 1:12 inclus, de préférence le rapport des quantités de quercétine à la PVP est choisi dans le groupe comprenant 1:7, 1:9 et 1:12.

**5.** Dispersion solide hydrosoluble selon la revendication 1 ou 4 ou le procédé selon l'une quelconque des revendications 2 à 4, dans lequel la PVP a un poids moléculaire moyen des macromolécules polymériques compris entre 7 000 et 11 000, de préférence la PVP est PVP K 17.

**6.** Dispersion solide hydrosoluble selon la revendication 1, 4 ou 5 ou le procédé selon l'une quelconque des revendications 2 à 5, où la quercétine est choisie parmi la quercétine dihydratée, de quercétine anhydre et des mélanges de celles-ci, de préférence la quercétine est de la quercétine anhydre cristallisée à partir d'éthanol, de préférence la quercétine est une quercétine anhydre doublement cristallisée à partir de d'éthanol à 96 %, avec une teneur en substance active d'au moins 99,7 %, plus préférenciellement, la quercétine est la quercétine dihydratée.

**7.** Dispersion solide hydrosoluble de quercétine produite par le procédé selon l'une quelconque des revendications 2 à 6.

**8.** Dispersion solide hydrosoluble selon l'une quelconque des revendications 1 ou 4 à 7, dans laquelle la quercétine est sous forme amorphe.

**9.** Dispersion solide hydrosoluble de quercétine selon l'une quelconque des revendications 1 ou 4 à 8, destinée à être utilisée comme substance pour l'obtention de médicaments injectables ou comme médicament oral pour le traitement d'affections aiguës de maladies des systèmes cardiovasculaires et cérébrovasculaires chez des sujets qui en ont besoin, de préférence avec une dose de la substance active dans la gamme de 14,28 % à 8,33 %.

**10.** Forme lyophilisée de dispersion solide hydrosoluble de quercétine, **caractérisée en ce qu'**elle comprend une dispersion solide de quercétine selon l'une quelconque des revendications 1 ou 4 à 8 ou une dispersion solide hydrosoluble quercétine destinée à être utilisée selon la revendication 9.

**11.** Dispersion solide hydrosoluble de quercétine sous forme de comprimé, **caractérisée en ce qu'**elle comprend une dispersion solide de quercétine selon l'une quelconque des revendications 1 ou 4 à 8.

**12.** Forme lyophilisée selon la revendication 10 ou forme en comprimés selon la revendication 11, comprenant 50 mg de quercétine.

**13.** Solution de perfusion comprenant une dispersion solide de quercétine selon l'une quelconque des revendications 1 ou 4 à 8 ou la dispersion solide de quercétine selon la revendication 9 et un solvant physiologiquement acceptable.

**14.** Forme injectable comprenant une solution stérile de la dispersion solide de quercétine selon l'une quelconque des revendications 1 ou 4 à 8 ou la dispersion solide de quercétine selon la revendication 9.

**15.** Solution de perfusion selon la revendication 13 ou forme injectable selon la revendication 14, dans laquelle la valeur du pH est dans la gamme de 5,5 à 7,0 et la concentration de quercétine est dans la gamme de 1 à 0,5 mg/ml.

**16.** Utilisation d'un agent alcalin pour obtenir une dispersion solide hydrosoluble de quercétine avec de la polyvinylpyrrolidone (PVP), dans laquelle l'agent alcalin est utilisé en une quantité dans la gamme de $(6,3 \pm 0,5)* 10^{-2}$ gE pour 1 gE de quercétine, où l'agent alcalin ne forme pas de sel insoluble avec la quercétine et/ou d'autres composants de la dispersion solide.

**17.** Dispersion solide hydrosoluble selon la revendication 1 ou 4 à 6, le procédé selon l'une quelconque des revendications 2 à 6 ou l'utilisation selon la revendication 16, où l'agent alcalin est choisi dans le groupe constitué par l'hydroxyde de sodium, le bicarbonate de sodium, le carbonate de sodium, la L-arginine et leurs mélanges.

**18.** Récipient comprenant une dispersion solide hydrosoluble stérile de quercétine selon l'une quelconque des revendications 1, 4 à 8 ou 17, la dispersion solide hydrosoluble de quercétine selon la revendication 9, ou la solution de perfusion ou la forme injectable selon l'une quelconque des revendications 13 à 15.

**19.** Un kit, comprenant un ou plusieurs récipients selon la revendication 18 et, éventuellement, solvant et/ou mode d'emploi de celui-ci.

**pH value of aqueous solution of solid dispersion as a function of gE of alkaline agents per 1 gE of quercetin.**

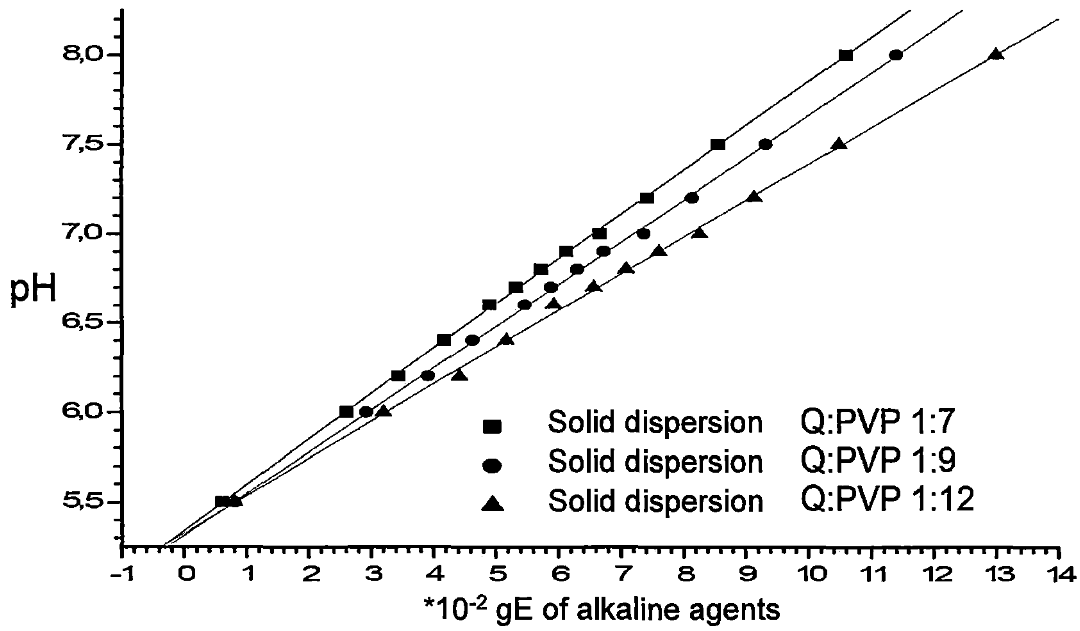


FIG. 1

**k ratio factor as a function of weight fraction polyvinyl pyrrolidone in the solid dispersion**

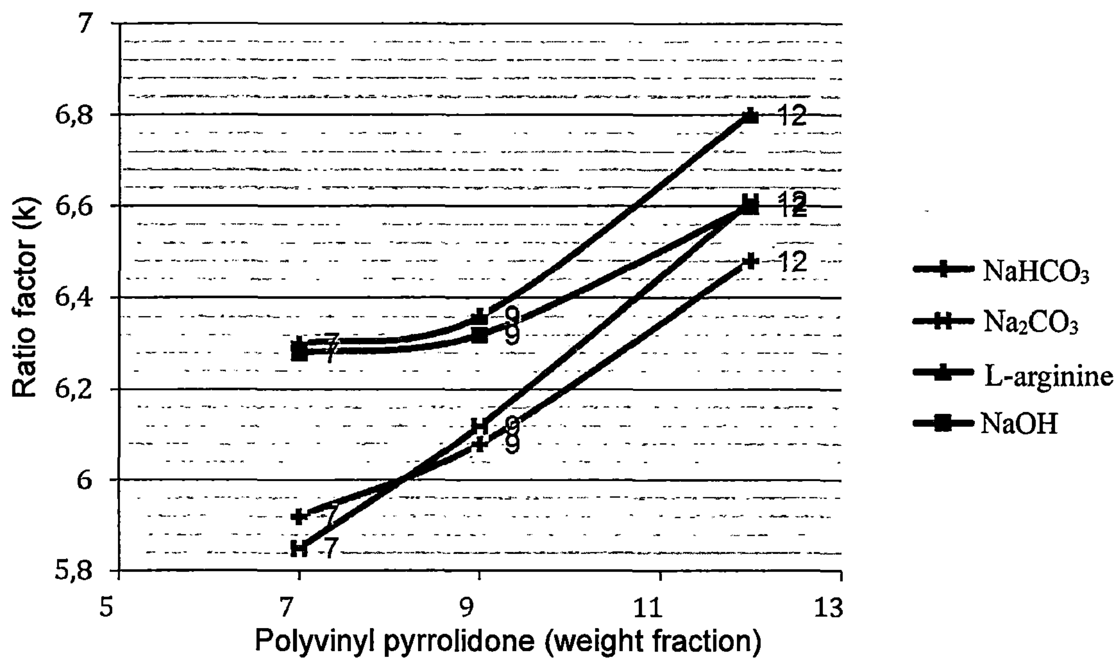


FIG. 2

**Rate of release of quercetin from samples of solid dispersions**

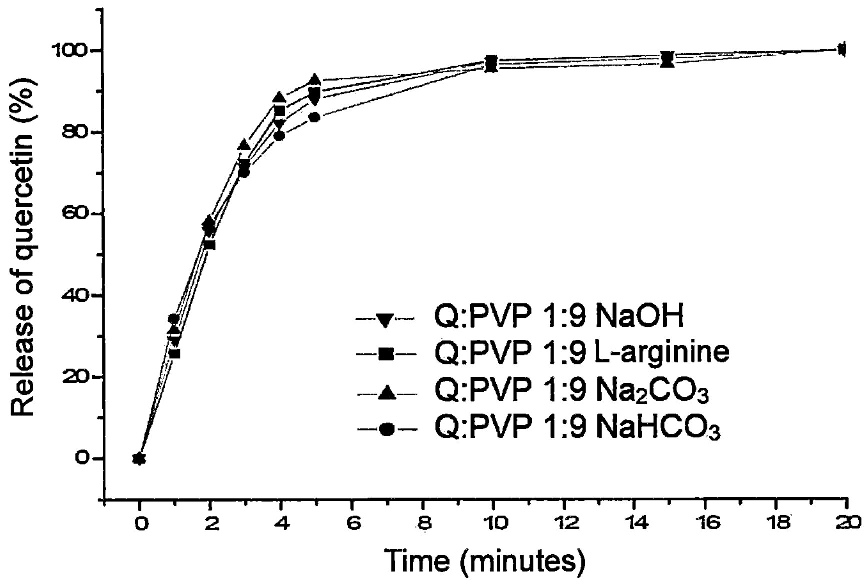


FIG. 3

**Rate of release of quercetin from samples of solid dispersions as a function of quantitative content of polyvinyl pyrrolidone therein**

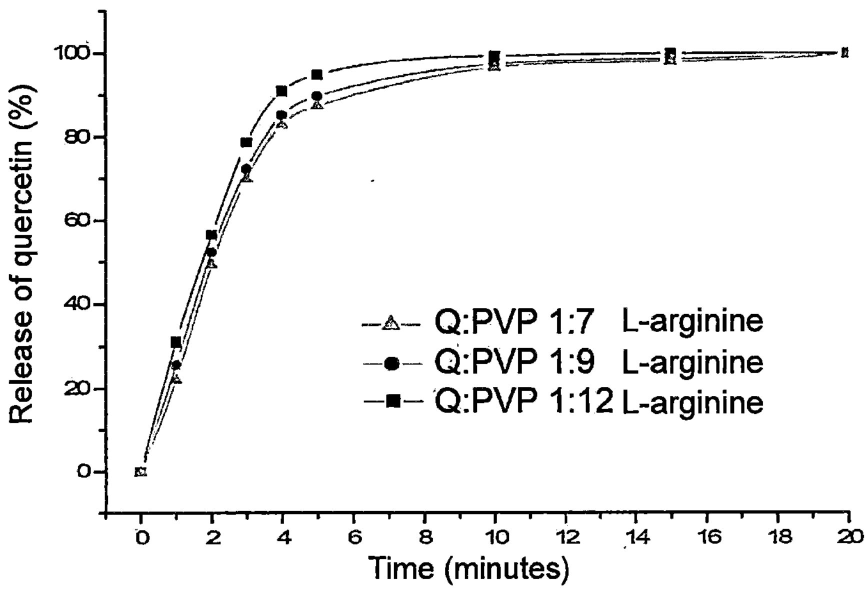


FIG. 4

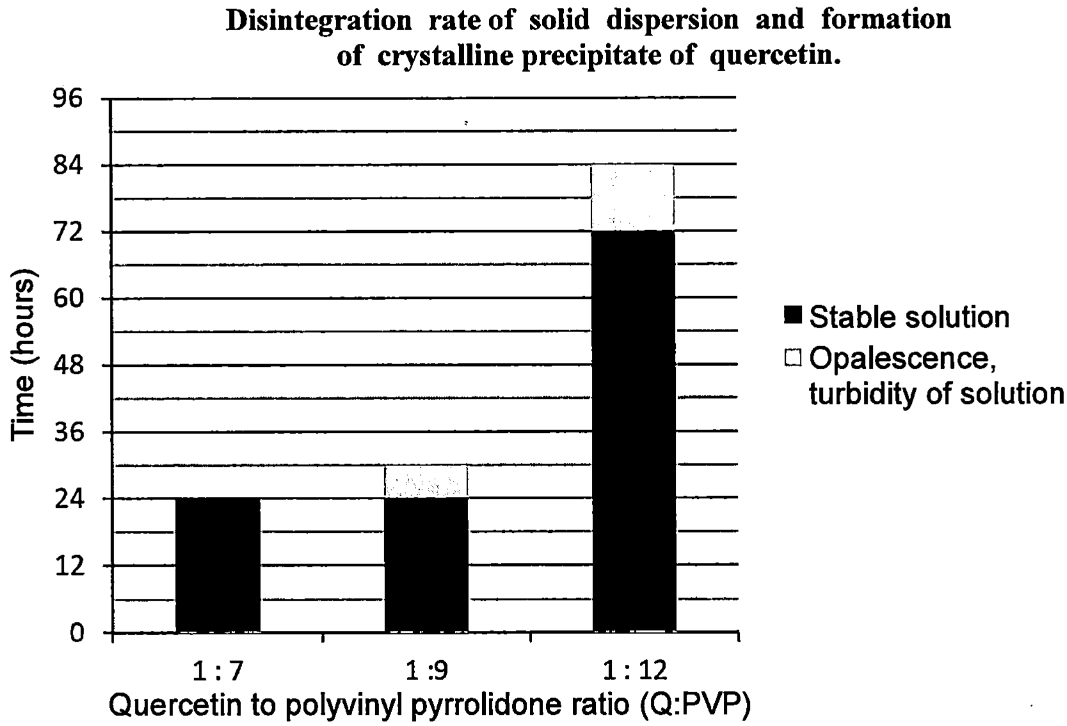
Disintegration rate of solid dispersion and formation of crystalline precipitate of quercetin.
96
84
72
60
48
36
24
12
0
Time (hours)
1 : 7
1 :9
1 : 12
Quercetin to polyvinyl pyrrolidone ratio (Q:PVP)
Stable solution
Opalescence, turbidity of solution

FIG. 5

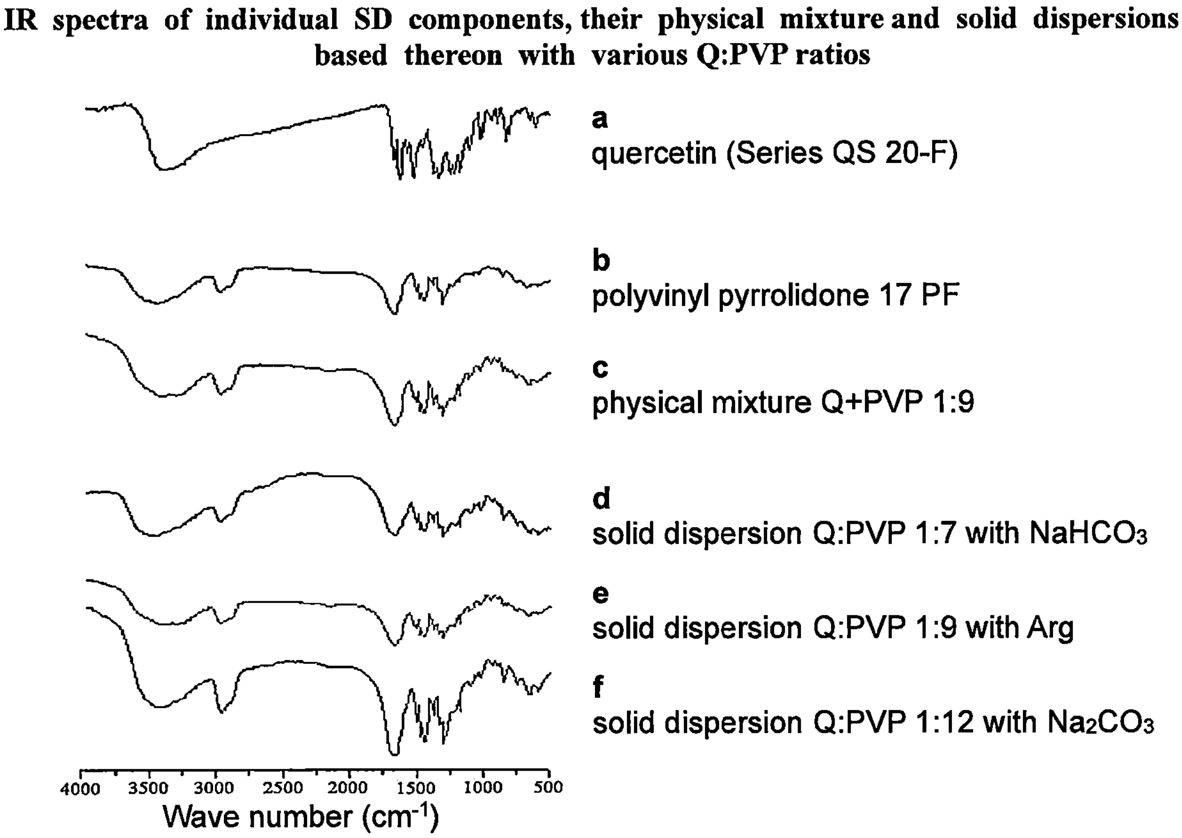
IR spectra of individual SD components, their physical mixture and solid dispersions based thereon with various Q:PVP ratios
a
quercetin (Series QS 20-F)
b
polyvinyl pyrrolidone 17 PF
c
physical mixture Q+PVP 1:9
d
solid dispersion Q:PVP 1:7 with NaHCO3
e
solid dispersion Q:PVP 1:9 with Arg
f
solid dispersion Q:PVP 1:12 with Na2CO3
4000
3500
3000
2500
2000
1500
1000
500
Wave number (cm-1)

FIG. 6

X-ray phase analysis of samples of quercetin and of solid dispersions

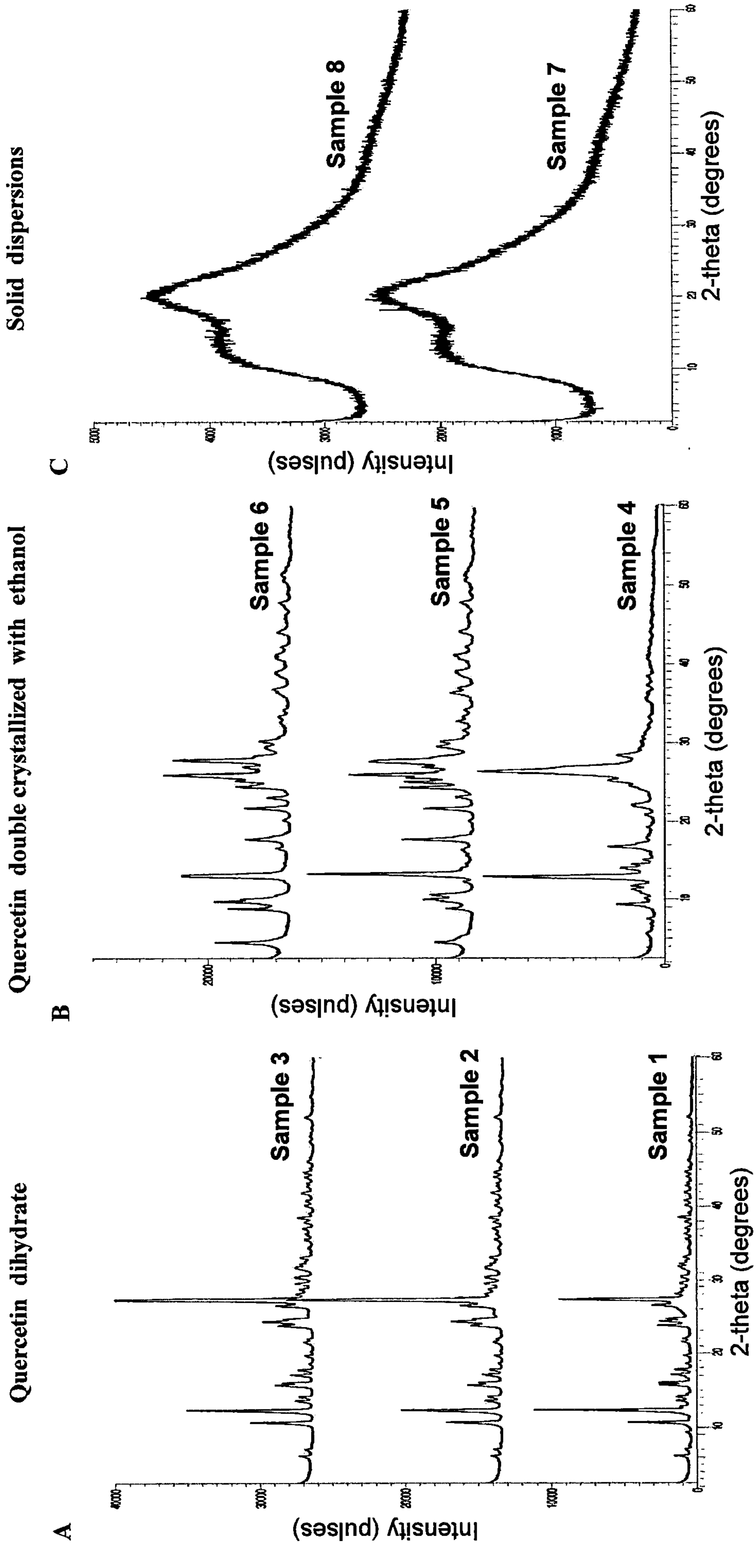


FIG. 7

**DSC curves**

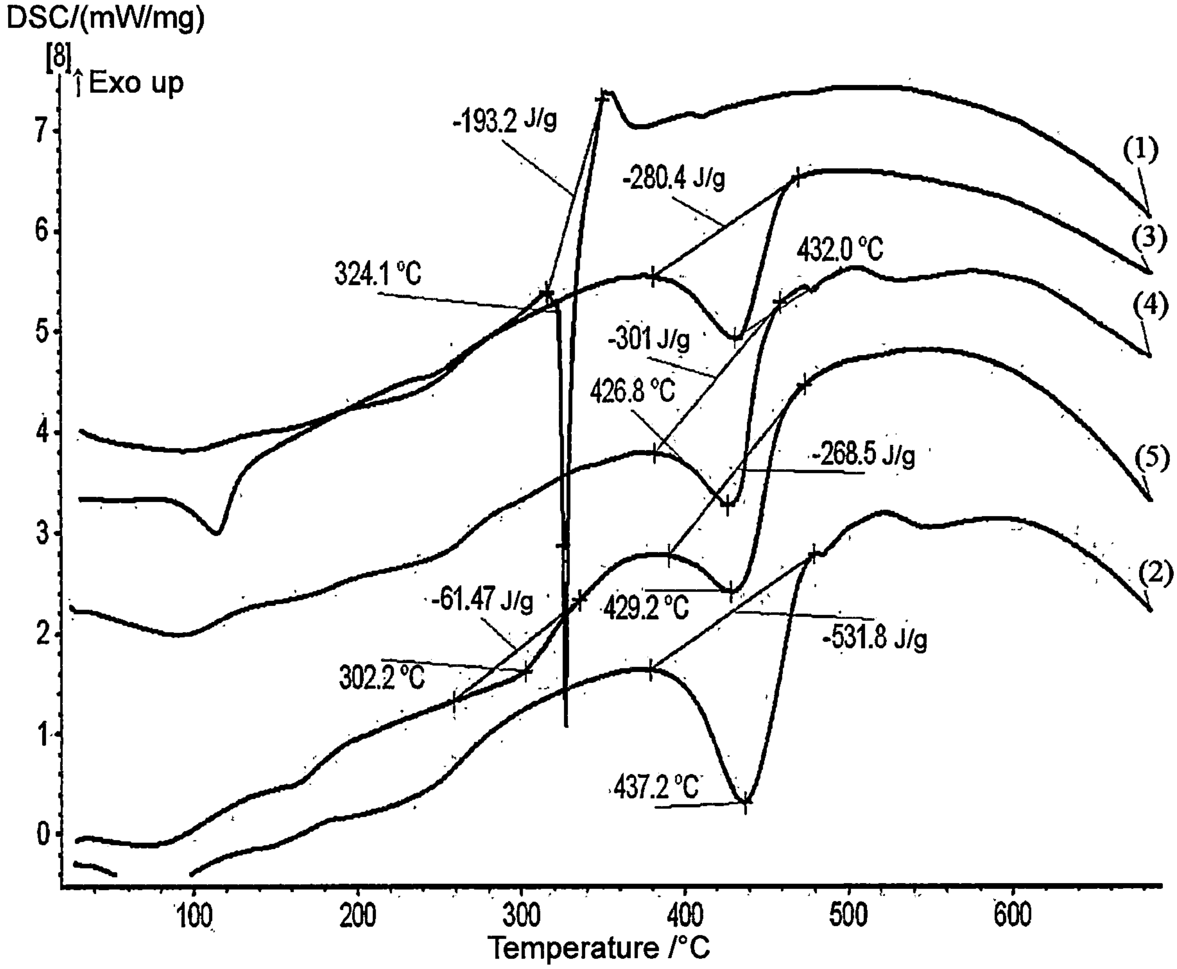


(1) quercetin;

(2) polyvinyl pyrrolidone;

(3) solid dispersion of quercetin : polyvinyl pyrrolidone 1:7;

(4) solid dispersion of quercetin : polyvinyl pyrrolidone 1:9;

(5) physical mixture quercetin + polyvinyl pyrrolidone 1:9

**FIG. 8**

**Stability of solid dispersions solutions in 0.9 % sodium chloride solution with pH 5.5.**

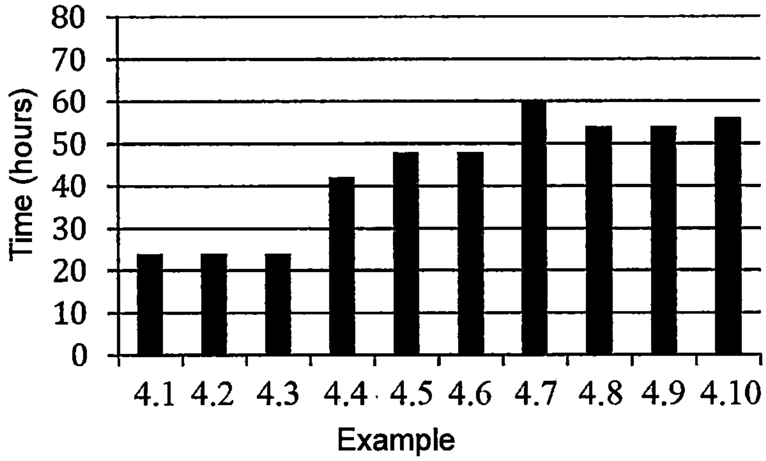


**FIG. 9**

**Stability of solid dispersions solutions in 0.9 % sodium chloride solution with pH 6.2.**

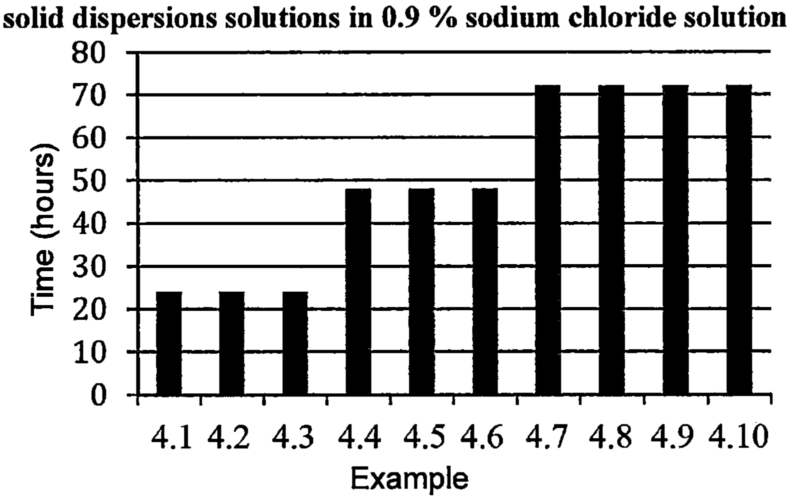


**FIG. 10**

**Stability of solid dispersions solutions in 0.9 % sodium chloride solution with pH 7.0.**

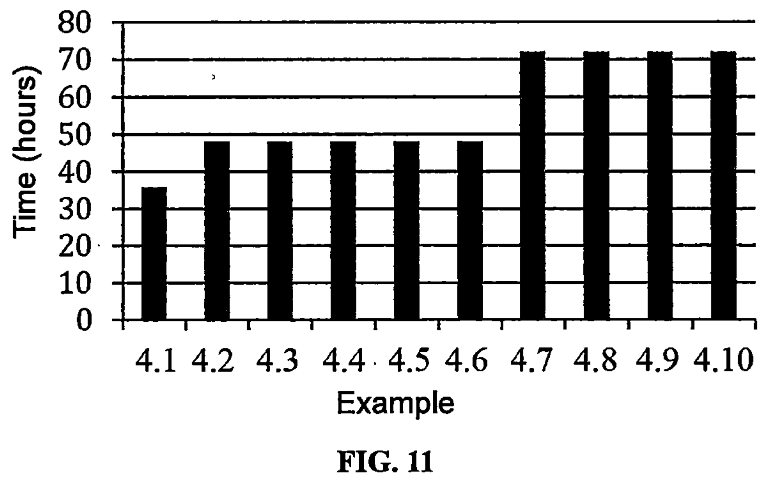


**FIG. 11**

**pH value of infusion solution as a function of concentration of quercetin within the range of 20 to 0.03125 mg/ml in 0.9 % solution of sodium chloride with pH = 5.50; 6.20; 7.00**

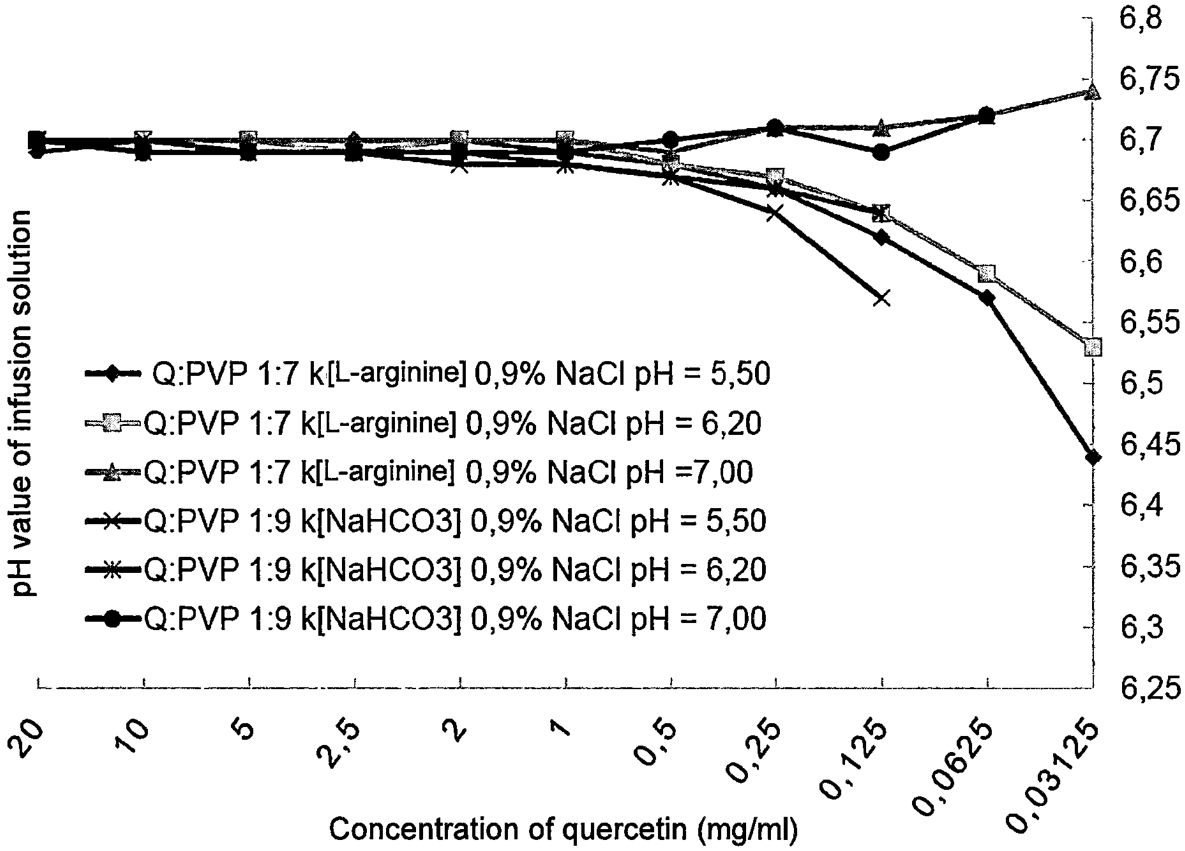


FIG. 12

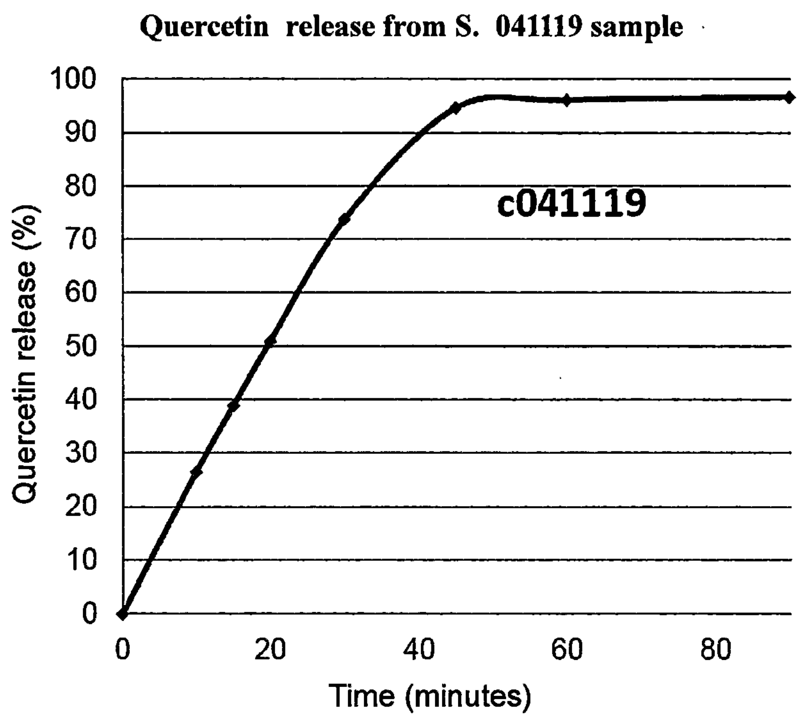
Quercetin release from S. 041119 sample
100
90
80
70
60
50
40
30
20
10
0
Quercetin release (%)
0
20
40
60
80
Time (minutes)
c041119

FIG. 13

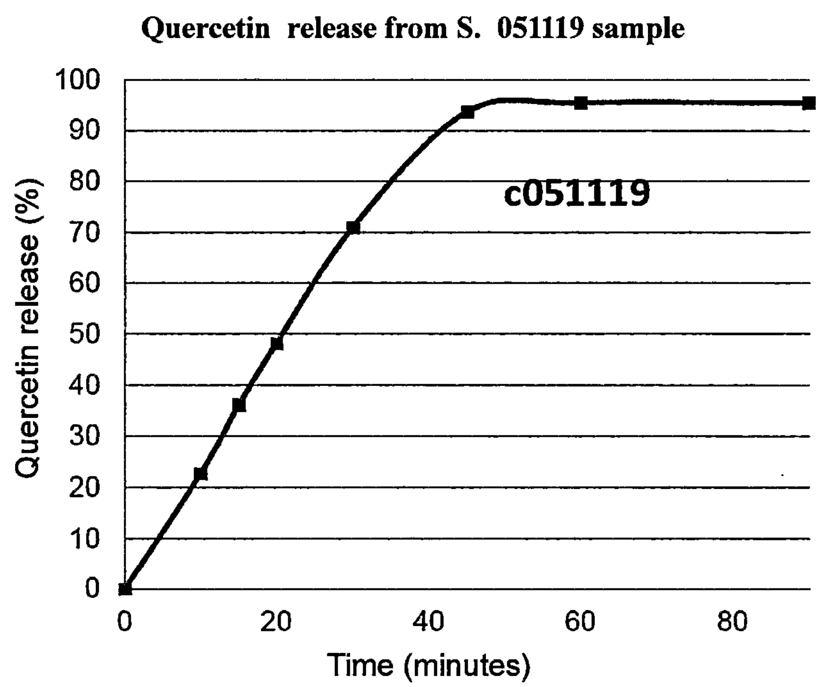
Quercetin release from S. 051119 sample
100
90
80
70
60
50
40
30
20
10
0
Quercetin release (%)
0
20
40
60
80
Time (minutes)
c051119

FIG. 14

**IR spectra of PVP K17, physical mixture Q + PVP (1:9) + L-arginine and solid dispersion of Q:PVP 1:9 k[L-arginine] obtained using the methods of coevaporation of solvents (Example 3.7i) and spraying (Example 8.3)**

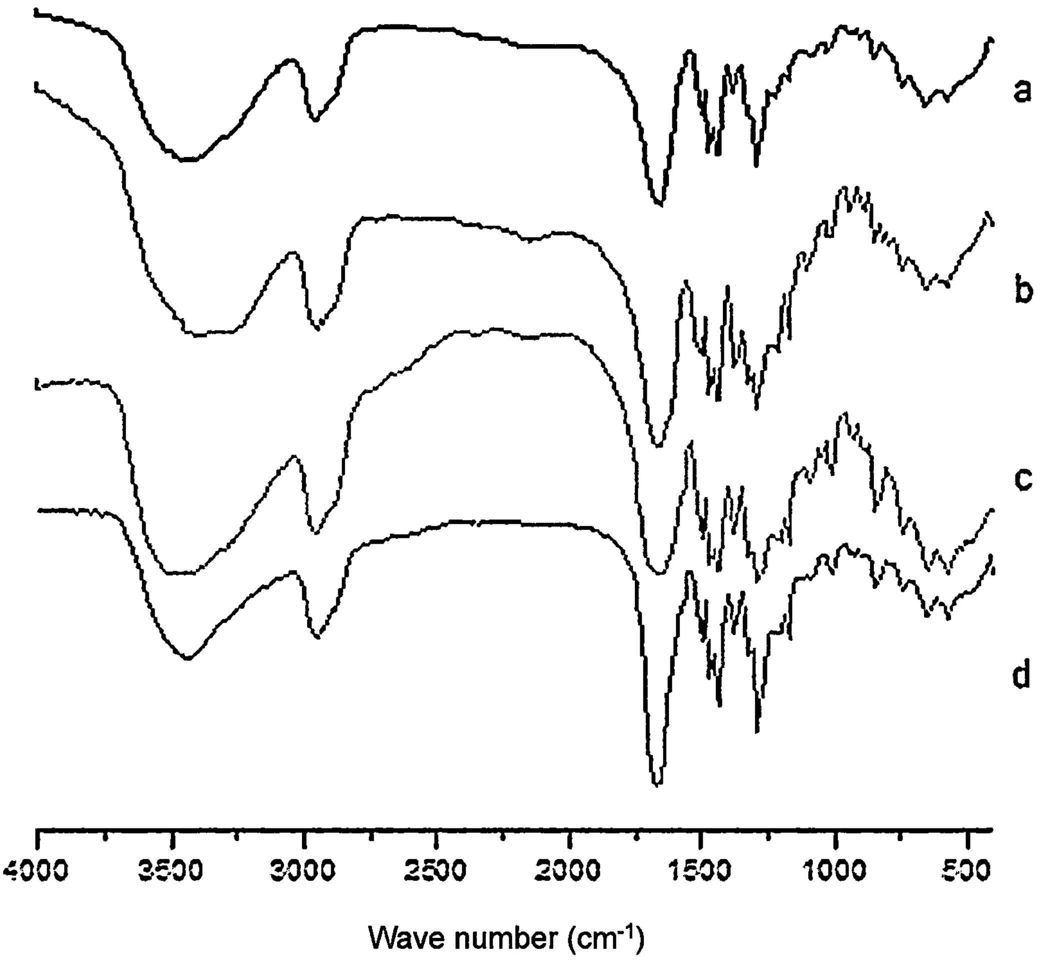


a) Polyvinyl pyrrolidone PVP K17PF

b) Physical mixture Q + PVP (1:9) + L-arginine

c) Solid dispersion of Q:PVP 1:9 k[L-arginine], Example 3.7i (coevaporation method)

d) Solid dispersion of Q:PVP 1:9 k[L-arginine], Example 8.3 (spraying method)

**FIG. 15**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7387805 B2
- RU 2181051
- EA 021352 B1
- UA 23996
- CN 109771395 A
- UA 38504


### Non-patent literature cited in the description


- *Monograph. Quercetin. Alternative Medicine Review.*, 1998, vol. 3, 140-143
- **MILES S.L., MCFARLAND M., NILES R.** Molecular and physiological actions of quercetin: need for clinical trials to assess its benefits in human disease.. *Nutr. Rev.*, 16 November 2014, vol. 72 (11), 720-734
- The Flavonoids: Advances in Research.. Chapman and Hall, 1982, 1018
- **RICE-EVANS A** ; **PACKER L.** Flavonoids in Health and Disease. M. Dekker Inc, 1998
- **NIJVELDT R.J.** ; **VAN NOOD E.** ; **VAN HOORN D.E. et al.** Flavonoids: a review of probable mechanisms of action and potential applications.. *Am. J. Clin. Nutr.*, 2001, vol. 74 (4), 418-425
- **MIDDLETON E.J.** ; **KANDASWAVI C.** ; **THEOHARIDES T.C.** The effects of plant flavonoids on mammalian cells: Implications of inflammation, heart disease, and cancer.. *Pharmacol. Res.*, 2000, vol. 52 (4), 673-751
- **ANDERSEN O.M.** ; **MARKHAM K.R.** Flavonoids: Chemistry, Biochemistry and Applications.. CRC Press Taylor & Francis Group, 2006, 1197
- **BORS W.** ; **HELLER W.** ; **MICHEL C.** ; **SARAN M.** Flavonoids as antioxidants: Determination of radical-scavenging efficiencies.. *Methods Ensymology.*, 1990, vol. 186, 343-355
- **SAIJA A.** ; **SCALESE M.** ; **LANZA M et al.** Flavonoids as antioxidant agents: importance of their interaction with biomembranes.. *Free Radic. Biol. Med*, 1995, vol. 19, 481-486
- **VAN ACKER S.A** ; **VAN DER BERG H.** ; **TROMP M.N. et al.** Structural aspects of antioxidant activity of flavonoids.. *Free Rad. Biol. Med.*, 1996, vol. 20, 331-342
- **ALVAREDA E.** ; **DENIS P.A.** ; **IRIBARNE F.** ; **PAULINO M.** Bond dissociation energies and enthalpies of formation of flavonoids: A G4 and M06-2X investigation.. *Computat. Theoret. Chem*, 2016, vol. 1091, 18-23
- **PEREZ-GONZALES A.** ; **REBOLLAR-ZEPEDA A.M.** ; **GALANO A.** Reactivity Indexes and O-H Bond Dissociation Energies of a Large Series of Polyphenols: Implications for their Free Radical Scavenging Activity.. *J. Mex. Chem. Soc.*, 2012, vol. 56 (3), 241-249
- **KENDRE PN.** ; **PANDE V.V.** ; **CHAVAN K.M.** Novel formulation strategy to enhance solubility quercetin.. *Pharmacophore.*, 2014, vol. 5 (3), 358-370
- **DE MELLO COSTA A. R.** ; **MARQUIAFÁVEL F.S.** ; **MIRELA MARA DE OLIVEIRA M.M.** Quercetin-PVP K25 solid dispersion.. *J. Term. Anal. Calormtre.*, 2011, vol. 104, 273-278
- **RUFFER W.** Clinical follow-up on the problem of Kollidon storage.. *Medizinische*, 1955, vol. 15 (9), 539-541
- Storage of Polyvinyl Pyrrolidone.. **HOSORAWA S.** ; **UCHINO F.** ; **MIYAZATO T. et al.** Bull.. Yamaguchi Med. School, 1966, vol. 13, 281-298
- **KOJIMA M.** ; **TAKAHASHI K.** ; **HONDA K.** Morphological Study on the Effect of Polyvinyl Pyrrolidone Infusion upon the Reticuloendothelial System.. *Tohoku J. exp. Med.*, 1967, vol. 92, 27-54
- Final Report on the Safety Assessment of Polyvinylpyrrolidone (PVP).. *International Journal of Toxicology.*, 1998, vol. 17 (4), 95-130
- **PORCUA E.A.** ; **COSSUA M.** ; **RASSUA G.** ; **GIUNCHEDIA H**. Al. Aqueous injection of quercetin: An approach for conformation of its direct in vivo cardiovascular effects. *Intern. J. Pharmac.*, 2018, vol. 541, 224-233
- **LI BIN et al.** Solid dispersion of quercetin in cellulose derivative matrices influences both solubility and stability. *Carbohydr Polym*, 15 February 2013, vol. 92 (2), 2033-40
- **I.G. ZENKEVICH** ; **A.Y ESHCHENKO** ; **S.V. MAKAROVA** ; **A.G. VITENBERG** ; **Y.G. DOBRYAKOV** ; **C.A. UTSAL.** Identification of the products of oxidation of quercetin by air oxygen at ambient temperature.. *Molecules*, 2007, vol. 12, 654-672

- **R. SOKOKOVA** ; **S. RAMESOVA** ; **I. DEGAANO** ; **M. HROMADOVA** ; **M. GAL** ; **J. ZABKA**. The oxidation of natural flavonoid quercetin. *Chem. Commun.*, 2012, vol. 48, 3433-3435
- **R. ALVAREZ-DIDUK** ; **M.T. RAMIREZ-SILVA** ; **A. GALANO** ; **A. MERKOCI**. Deprotonation mechanism and acidity constants in aqueous solution of flavonols: a combined experimental and theoretical study.. *J. Phys. Chem.*, 2013, vol. 117, 12347-12359
- **CATAURO, M.** ; **PAPALE, F.** ; **BOLLINO, F. et al.** Silica/quercetin sol-gel hybrids as antioxidant dental implant materials.. *Sci. Technol. Adv. Mater.*, 2015, vol. 16, 035001
- **BORGHETTI, G.S.** ; **LULA, I.S.** ; **SINISTERRA, R.D.** ; **BASSANI, VL.** Quercetin/β-Cyclodextrin solid complexes prepared in aqueous solution followed by spray-drying or by physical mixture.. *AAPS Pharm. Sci. Tech.*, 2009, vol. 10, 235-242
- **DE MELLO COSTA A. R.** ; **MARQUIAFÁVEL F.S.** ; **MIRELA MARA DE OLIVEIRA M.M.** Quercetin-PVP K25 solid dispersion.. *J. Term. Anal. Calorimetre*, 2011, vol. 104, 273-278
- **BRYASKOVA, R.** ; **PENCHEVA, D.** ; **NIKOLOV, S.** ; **KANTARDJIEV, T.** Synthesis and comparative study on the antimicrobial activity of hybrid materials based on silver nanoparticles (AgNps) stabilized by polyvinylpyrrolidone (PVP).. *J. Chem. Biol.*, 2011, vol. 4, 185-191
- **ZHU, J.** ; **YANG, Z.** ; **CHEN, X.** Preparation and physicochemical characterization of solid dispersion of quercetin and polyvinylpyrrolidone.. *J. Chin. Pharm. Sci.*, 2007, vol. 16, 51-57
- **L. DE LEMOS M** ; **MONFARED SH** ; **DENYSSEVYCH T.** Evaluation of osmolality and pH of various concentrations of methotrexate, cytarabine, and thiotepa prepared in normal saline, sterile water for injection, and lactated Ringer's solution for intrathecal administration.. *J. Oncol. Pharm. Practice.*, 2009, vol. 15, 45-52